# EUROPÄISCHE PATENTSCHRIFT

(11) **EP 1 742 945 B1**
(45) Veröffentlichungstag und Bekanntmachung des Hinweises auf die Patenterteilung: **30.12.2009**
(21) Anmeldenummer: 05739438.9
(22) Anmeldetag: 14.04.2005
(51) Int. Cl.: C07D 471/04, A61K 31/437, A61P 9/10

(54) **SUBSTITUIERTE PHENYLAMINOPYRIMIDINE**
SUBSTITUTED PHENYLAMINO-PYRIMIDINES
PHENYLAMINOPYRIMIDINES SUBSTITUEES

(30) Priorität: 27.04.2004 DE 102004020570
(43) Veröffentlichungstag der Anmeldung: 17.01.2007
(73) Patentinhaber: Bayer Schering Pharma Aktiengesellschaft, 13353 Berlin (DE)
(72) Erfinder: SCHIROK, Hartmut, 42287 Wuppertal (DE); STASCH, Johannes-Peter, 42651 Solingen (DE); KAST, Raimund, 42349 Wuppertal (DE); MÜNTER, Klaus, 42489 Wülfrath (DE); GNOTH, Mark, Jean, 40822 Mettmann (DE); FIGUEROA, PEREZ, Santiago, 51373 Leverkusen (DE); THUTEWOHL, Michael, 34305 Niedenstein (DE); BENNABI, Samir, F-69004 Lyon (FR); LANG, Dieter, 42553 Velbert (DE); MITTENDORF, Joachim, 42113 Wuppertal (DE); RADTKE, Martin, 46099 Erkrath (DE); EHMKE, Heimo c/o Universität Hamburg, 20146 Hamburg (DE)
(86) Internationale Anmeldenummer: PCT/EP2005/003925
(87) Internationale Veröffentlichungsnummer: WO 2005/108397

(56) Entgegenhaltungen:
- WO-A-03/062225
- WO-A-03/062227
- WO-A-03/106450

## Beschreibung

Die Erfindung betrifft substituierte Phenylaminopyrimidine, ein Verfahren zu ihrer Herstellung sowie ihre Verwendung zur Herstellung von Arzneimitteln zur Behandlung und/oder Prophylaxe von Krankheiten bei Menschen und Tieren, insbesondere von kardiovaskulären Erkrankungen.

Ein Anstieg der intrazellulären Calcium-Konzentration ist ein Hauptauslöser für die Kontraktion der Gefäßmuskulatur (Somlyo, A.P. und Himpens, B. FASEB J. 1989, 3, 2266-2276). Dies geschieht in erster Linie durch Agonisten wie z.B. Phenylephrin oder Thromboxan A2, die nach Stimulierung der Phosphatidylinositolkaskade die Freisetzung von Calcium aus dem sarkoplasmatischen Retikulum bewirken. Die Erhöhung des intrazellulären Calciums aktiviert die MLC-Kinase (Myosin-Leichte-Ketten-Kinase), die die MLC-Untereinheiten des Myosinmoleküls phosphoryliert (Kamm, K.H. und Stull, J.T., Annu. Rev. Pharmacol. Toxicol. 1985, 25, 593-603). MLC-Phosphorylierung induziert die Glattmuskelkontraktion, MLC-Dephosphorylierung nach einer Reduktion der intrazellulären Calciumkonzentration resultiert in der Relaxation des Gefäßes.

Neben der Calcium-abhängigen MLC-Phosphorylierung existiert noch ein weiterer zentraler aber Calcium-unabhängiger Regulationsmechanismus des Gefäßtonus. Hierbei handelt es sich um den Rho/Rho-Kinase-Signalweg (Noda, M. et al., FEBS Lett. 1995, 367, 246-250; Uehata, M. et al., Nature 1997, 389, 990-994; Fukata, Y. et al., Trends in Pharmacological Sciences 2001, 22, 32-39). Binden Agonisten wie z.B. Phenylephrin oder Thromboxan A2 an ihre Rezeptoren, so fühlt dies zur Aktivierung der kleinen G-Proteine Rho, die dann mit der Rho-Kinase interagieren und diese aktivieren. Die aktivierte Rho-Kinase inhibiert die Myosin-Phosphatase, nachdem sie eine Untereinheit des Enzyms phosphoryliert hat. Gleichzeitig phosphoryliert Rho-Kinase MLC an der Stelle, die auch von der MLC-Kinase phosphoryliert wird. Eine Hemmung der Myosin-Phosphatase sowie der Phosphorylierung von MLC induziert die Kontraktion der Gefäßmuskulatur. Im Gegensatz dazu führt eine Hemmung der Rho-Kinase zu einer Gefäßrelaxation. Inhibitoren der Rho-Kinase bewirken daher eine Senkung des Blutdruckes und eine Steigerung des koronaren Blutflusses.

Darüber hinaus führen Inhibitoren der Rho-Kinase zu einer Hemmung des Wachstums von Tumorzellen und Metastasen (Itoh et al. Nat. Med. 1999, 5, 221; Somlyo et al. Biochem. Biophys. Res. Commun. 2000, 269,_652) und inhibieren die Angiogenese (Uchida et al. Biochem. Biophys. Res. Commun. 2000, 269, 633; Gingras et al. Biochem. J. 2000, 348 Bd. 2, 273).

Den erfindungsgemäßen Verbindungen ähnliche Strukturen sind in WO 03/062225, WO 03/062227, WO 03/106450 und WO 04/039796 als Rho-Kinase-Inhibitoren zur Behandlung von Krebs und kardiovaskulären Erkrankungen offenbart. WO 03/02542 offenbart unter anderem substituierte Pyrimidine als p38 Kinase Inhibitoren zur Behandlung von inflammatorischen und kardiovaskulären Erkrankungen.

Den erfindungsgemäßen Verbindungen ähnliche Strukturen sind weiterhin aus anderen Indikationen bekannt. So offenbart beispielsweise WO 01/28561 substituierte Pyrimidine als DNA Polymerase III Inhibitoren zur Behandlung von bakteriellen Infektionen.

Gegenstand der vorliegenden Erfindung sind Verbindungen der Formel (I) 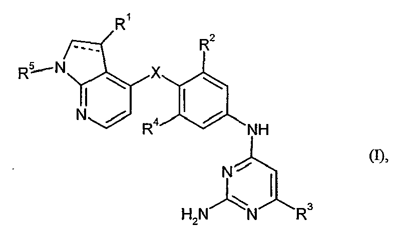 worin
- -----: eine Einfach- oder eine Doppelbindung bedeutet,
- X: -NR⁶-, -CR⁷R⁸- oder -C(=O)- bedeutet,
worin
- R⁶: für Wasserstoff oder (C₁-C₃)-Alkyl steht,
- R⁷ und R⁸: unabhängig voneinander für Wasserstoff oder Methyl stehen,
- R¹: Wasserstoff, Halogen, Cyano, (C₁-C₆)-Alkyl, (C₃-C₆)-Cycloalkyl, (C₁-C₆)-Alkoxycarbonyl oder (C₁-C₆)-Alkylaminocarbonyl bedeutet,
wobei Alkyl, Alkoxycarbonyl und Alkylaminocarbonyl ihrerseits durch Hydroxy, Halogen, Hydroxycarbonyl, (C₁-C₆)-Alkoxy, (C₁-C₆)-Alkoxycarbonyl, Amino, Aminocarbonyl, (C₁-C₆)-Alkylamino oder (C₁-C₆)-Alkylaminocarbonyl substituiert sein können,
- R²: Fluor oder Chlor bedeutet,
- R³: einen Rest bedeutet, der ausgewählt ist aus der Gruppe von:

- Wasserstoff, Hydroxy, Halogen, Trifluormethyl, Pentafluorethyl,
- (C₁-C₆)-Alkyl, (C₁-C₆)-Alkoxy, (C₃-C₈)-Cycloalkyl,
   wobei Alkyl, Alkoxy und Cycloalkyl ihrerseits durch Hydroxy, Hydroxycarbonyl, (C₁-C₆)-Alkoxy, (C₁-C₆)-Alkoxycarbonyl, (C₆-C₁₀)-Aryl, -NR⁹R¹⁰ oder -C(=O)NR⁹R¹⁰ substituiert sein können,
   worin

- R⁹ und R¹⁰: unabhängig voneinander für Wasserstoff, (C₁-C₈)-Alkyl, gegebenenfalls mit (C₁-C₆)-Alkyl substituiertes (C₃-C₆)-Cycloalkyl, gegebenenfalls mit Halogen substituiertes (C₆-C₁₀)-Aryl oder 5- bis 10-gliedriges Heteroaryl stehen
oder
- R⁹ und R¹⁰: gemeinsam mit dem Stickstoffatom, an das sie gebunden sind, einen 5-oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,

- (C₆-C₁₀)-Aryl, (C₆-C₁₀)-Aryloxy, 5- bis 10-gliedriges Heteroaryl, 5- bis 10-gliedriges Heteroaryloxy, über ein Kohlenstoffatom gebundenes 5- bis 10-gliedriges Heterocyclyl,
   wobei Aryl, Aryloxy, Heteroaryl, Heteroaryloxy und Heterocyclyl ihrerseits durch Halogen, Cyano, Nitro, Hydroxycarbonyl, Amino, Trifluormethyl, gegebenenfalls mit Hydroxy substituiertes (C₁-C₆)-Alkyl, (C₁-C₆)-Alkoxy, (C₁-C₆)-Alkylamino, (C₁-C₆)-Alkanoyl, (C₁-C₆)-Alkoxycarbonyl, (C₁-C₆)-Alkanoylamino, (C₁-C₆)-Alkoxycarbonylamino oder 5- oder 6-gliedriges Heterocyclyl substituiert sein können,
- -NR¹¹R¹²,
   worin

- R¹¹ und R¹²: unabhängig voneinander für Wasserstoff, (C₁-C₆)-Alkyl, (C₃-C₈)-Cycloalkyl, (C₆-C₁₀)-Aryl oder 5- bis 10-gliedriges Heteroaryl stehen, wobei Alkyl und Cycloalkyl ihrerseits durch Hydroxy, (C₁-C₆)-Alkoxy, (C₆-C₁₀)-Aryl, 5- bis 10-gliedriges Heteroaryl oder -NR¹³R¹⁴ substituiert sein können,
worin
- R¹³ und R¹⁴: unabhängig voneinander für Wasserstoff, (C₁-C₆)-Alkyl, (C₃- C₆)-Cycloalkyl, (C₆-C₁₀)-Aryl oder 5- oder 6-gliedriges Heteroaryl stehen
oder
- R¹³ und R¹⁴: gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,
und
wobei Aryl und Heteroaryl ihrerseits durch Halogen, Hydroxy, Amino, Cyano, Trifluormethyl, (C₁-C₆)-Alkyl, (C₁-C₆)-Alkoxy, (C₁-C₆)-Alkylamino oder (C₁-C₆)-Alkanoylamino substituiert sein können,
oder
- R¹¹ und R¹²: gemeinsam mit dem Stickstoffatom, an das sie gebunden sind, einen 4- bis 6- gliedrigen Heterocyclus bilden, der ein weiteres Heteroatom O oder N im Ring enthalten kann und der durch Fluor, Hydroxy, Hydroxycarbonyl, 5- bis 7- gliedriges Heterocyclyl, das ein oder zwei weitere Heteroatome N und/oder O im Ring enthalten kann, das seinerseits durch (C₁-C₄)-Alkyl oder (C₁-C₄)-Alkoxycarbonyl substituiert sein kann, (C₁-C₄)-Alkoxy, (C₁-C₄)-Alkyl, (C₁-C₄)-Alkanoyl, (C₁-C₄)-Alkoxycarbonyl oder -NR¹⁵R¹⁶ substituiert sein kann,
wobei Alkyl seinerseits durch Hydroxy, (C₁-C₄)-Alkoxy oder -NR¹⁷R¹⁸ substituiert sein kann,
worin
- R¹⁷ und A¹⁸: unabhängig voneinander für Wasserstoff, gegebenenfalls mit Hydroxy substituiertes (C₁-C₆)-Alkyl, (C₃-C₆)-Cycloalkyl, (C₆-C₁₀)-Aryl oder 5- oder 6-gliedriges Heteroaryl stehen
oder
- R¹⁷ und R¹⁸: gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,
worin
- R¹⁵ und R¹⁶: unabhängig voneinander Wasserstoff, (C₁-C₆)-Alkyl, (C₁-C₄)- Alkoxycarbonyl, (C₃-C₈)-Cycloalkyl oder (C₁-C₄)-Alkanoyl bedeuten
oder
- R¹⁵ und R¹⁶: gemeinsam mit dem Stickstoffatom, an das sie gebunden sind, einen 5-oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,
oder
- R¹¹ und R¹²: gemeinsam mit dem Stickstoffatom, an das sie gebunden sind, einen 7- bis 12- gliedrigen bicyclischen oder tricyclischen Heterocyclus bilden, der anneliert oder spirocyclisch ist und ein oder zwei weitere Heteroatome aus der Reihe N und/oder O im Ring aufweisen kann und der durch Fluor, (C₁-C₄)-Alkyl, (C₁-C₄)-Alkoxycarbonyl, (C₁-C₄)-Alkanoyl oder Benzyl substituiert sein kann,

- und -C(=O)R¹⁹,
   worin

- R¹⁹: für (C₁-C₆)-Alkoxy, (C₁-C₆)-Alkylamino oder einen über ein Stickstoffatom gebundenen 5- bis 10-gliedrigen mono- oder bicyclischen Heterocyclus, der anneliert oder spirocyclisch ist und ein oder zwei weitere Heteroatome aus der Reihe N und/oder O im Ring aufweisen kann, steht, wobei Alkylamino seinerseits mit einem 5- oder 6-gliedrigen Heterocyclyl substituiert sein kann,
- R⁴: Wasserstoff, Fluor oder Chlor bedeutet,
- R⁵: Wasserstoff oder (C₁-C₆)-Alkyl bedeutet
und ihre Salze, Hydrate, Hydrate der Salze und Solvate.

Erfindungsgemäße Verbindungen sind die Verbindungen der Formel (I) und deren Salze, Solvate und Solvate der Salze; die von Formel (I) umfassten Verbindungen der nachfolgend genannten Formeln und deren Salze, Solvate und Solvate der Salze sowie die von Formel (I) umfassten, nachfolgend als Ausführungsbeispiele genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung umfasst deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfmdungsgemäßen Verbindungen in tautomeren Formen vorkommen können, umfasst die vorliegenden Erfindung sämtliche tautomere Formen.

Als Salze sind im Rahmen der vorliegenden Erfindung physiologisch unbedenkliche Salze der erfmdungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, *N*-Methylmorpholin, Arginin, Lysin, Ethylendiamin und *N*-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkanoyl, Alkylamino, Alkylaminocarbonyl, Alkoxycarbonyl, Alkoxycarbonylamino und Alkanoylamino stehen für einen linearen oder verzweigten Alkylrest mit in der Regel 1 bis 6, vorzugsweise 1 bis 4, besonders bevorzugt 1 bis 3 Kohlenstoffatomen, beispielhaft und vorzugsweise für Methyl, Ethyl, n-Propyl, Isopropyl, *tert*-Buryl, n-Pentyl und n-Hexyl.

Alkoxy steht beispielhaft und vorzugsweise für Methoxy, Ethoxy, n-Propoxy, Isopropoxy, *tert-*Butoxy, n-Pentoxy und n-Hexoxy.

Alkanoyl steht beispielhaft und vorzugsweise für Acetyl und Propanoyl.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, *tert*-Burylamino, n-Pentylamino, n-Hexylamino, *N,N*-Dimethylamino, *N,N*-Diethylamino, *N,N*-Diisopropylamino, *N*-Ethyl-*N*-methylamino, *N*-Methyl-*N*-n-propylamino, *N-*Isopropyl-*N*-n-propylamino, *N*-*tert*-Butyl-*N*-methylamino, *N*-Ethyl-*N*-n-pentylamino und *N*-n-Hexyl-*N*-methylamino. C₁-C₄-Alkylamino steht beispielsweise für einen Monoalkylaminorest mit 1 bis 4 Kohlenstoffatomen oder für einen Dialkylaminorest mit jeweils 1 bis 4 Kohlenstoffatomen pro Alkylsubstituent.

Alkylaminocarbonyl steht für einen Alkylaminocarbonylrest mit einem oder zwei (unabhängig voneinander gewählten) Alkylsubstituenten. (C₁-C₃)-Alkylaminocarbonyl steht beispielsweise für einen Monoalkylaminocarbonylrest mit 1 bis 3 Kohlenstoffatomen oder für einen Dialkylaminocarbonylrest mit jeweils 1 bis 3 Kohlenstoffatomen pro Alkylsubstituent. Beispielhaft und vorzugsweise seien genannt: Methylaminocarbonyl, Ethylaminocarbonyl, n-Propylaminocarbonyl, Isopropylaminocarbonyl, n-Butylaminocarbonyl, *tert*-Butylaminocarbonyl, n-Pentylaminocarbonyl, n-Hexylaminocarbonyl, *N,N-*Dimethylaminocarbonyl, *N,N*-Diethylaminocarbonyl, *N*-Ethyl-*N-*methylaminocarbonyl, *N*-Methyl-*N*-n-propylaminocarbonyl, *N*-Isopropyl-*N*-n-propylaminocarbonyl, *N*-*tert*-Butyl-*N*-methylaminocarbonyl, *N*-Ethyl-*N*-n-pentylamino-carbonyl und *N*-n-Hexyl-*N*-methylaminocarbonyl.

Alkoxycarbonyl steht beispielhaft und vorzugsweise für Methoxycarbonyl, Ethoxycarbonyl, n-Propoxycarbonyl, Isopropoxycarbonyl, *tert*-Butoxycarbonyl, n-Pentoxycarbonyl und n-Hexoxycarbonyl.

Alkoxycarbonylamino steht beispielhaft und vorzugsweise für Methoxycarbonylamino, Ethoxycarbonylamino, n-Propoxycarbonylamino, Isopropoxycarbonylamino, *tert*-Butoxycarbonylamino, n-Pentoxycarbonylamino und n-Hexoxycarbonylamino.

Alkanoylamino steht beispielhaft und vorzugsweise für Acetylamino und Ethylcarbonylamino.

Cycloaklyl steht für eine Cycloalkylgruppe mit in der Regel 3 bis 8, bevorzugt 5 bis 7 Kohlenstoffatomen, beispielhaft und vorzugsweise für Cyclopropyl, Cyclobutyl, Cyclopentyl, Cyclohexyl und Cycloheptyl.

Aryl per se und in Aryloxy steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.

Aryloxy steht beispielhaft und vorzugsweise für Phenyloxy und Naphthyloxy.

Heteroaryl per se und in Heteroaryloxy steht für einen aromatischen, mono- oder bicyclischen Rest mit in der Regel 5 bis 10, vorzugsweise 5 bis 6 Ringatomen und bis zu 5, vorzugsweise bis zu 4 Heteroatomen aus der Reihe S, O und N, beispielhaft und vorzugsweise für Thienyl, Furyl, Pyrrolyl, Thiazolyl, Oxazolyl, Imidazolyl, Pyridyl, Pyrimidyl, Pyridazinyl, Indolyl, Indazolyl, Benzofuranyl, Benzothiophenyl, Chinolinyl, Isochinolinyl.

Heteroaryloxy steht beispielhaft und vorzugsweise für Pyridyloxy, Pyrimidyloxy, Indolyloxy, Indazolyloxy.

Heterocyclyl und Heterocyclus steht für einen mono- oder polycyclischen, vorzugsweise mono- oder bicyclischen, nicht-aromatischen heterocyclischen Rest mit 4 bis 10, in der Regel 5 bis 8, vorzugsweise 5 oder 6 Ringatomen und bis zu 3, vorzugsweise bis zu 2 Heteroatomen und/oder Heterogruppen aus der Reihe N, O, S, SO, SO₂. Die Heterocyclyl-Reste können gesättigt oder teilweise ungesättigt sein. Bevorzugt sind 5- oder 6-gliedrige, monocyclische gesättigte Heterocyclylreste mit bis zu zwei Heteroatomen aus der Reihe O, N und S, wie beispielhaft und vorzugsweise Tetrahydrofuran-2-yl, Tetrahydrothienyl, Pyrrolidin-2-yl, Pyrrolidin-3-yl, Pyrrolinyl, Pyranyl, Piperidin-1-yl, Piperidin-2-yl, Piperidin-3-yl, Piperidin-4-yl, Thiopyranyl, Morpholin-1-yl, Morpholin-2-yl, Morpholin-3-yl, Perhydroazepinyl, Piperazin-1-yl, Piperazin-2-yl.

Halogen steht für Fluor, Chlor, Brom und Jod.

Wenn Reste in den erfindungsgemäßen Verbindungen substituiert sind, können die Reste, soweit nicht anders spezifiziert, ein- oder mehrfach gleich oder verschieden substituiert sein. Eine Substitution mit bis zu drei gleichen oder verschiedenen Substituenten ist bevorzugt. Ganz besonders bevorzugt ist die Substitution mit einem Substituenten.

Bevorzugt sind Verbindungen der Formel (I), worin
- -----: eine Doppelbindung bedeutet,
- X: -NR⁶-, -CH₂- oder -C(=O)- bedeutet,
worin R⁶ für Wasserstoff oder Methyl steht,
- R¹: Wasserstoff, Fluor, Chlor, Cyano, Trifluormethyl, Difluormethyl, Methyl, Ethyl, Hydroxyethyl, Methoxyethyl, Cyclopropyl, (C₁-C₆)-Alkoxycarbonyl oder (C₁-C₆)-Alkylaminocarbonyl bedeutet,
wobei Alkoxycarbonyl und Alkylaminocarbonyl ihrerseits durch Hydroxy, Hydroxycarbonyl, (C₁-C₆)-Alkoxy, (C₁-C₆)-Alkoxycarbonyl, Amino, Aminocarbonyl, (C₁-C₆)-Alkylamino oder (C₁-C₆)-Alkylaminocarbonyl substituiert sein können,
- R²: Fluor oder Chlor bedeutet,
- R³: einen Rest bedeutet, der ausgewählt ist aus der Gruppe von:

- Wasserstoff, Hydroxy, Halogen, Trifluormethyl, Pentafluorethyl,
- (C₁-C₆)-Alkyl, (C₁-C₆)-Alkoxy, (C₃-C₈)-cycloalkyl,
   wobei Alkyl, Alkoxy und Cycloalkyl ihrerseits durch Hydroxy, Hydroxycarbonyl, (C₁-C₆)-Alkoxy, (C₁-C₆)-Alkoxycarbonyl, (C₆-C₁₀)-Aryl, -NR⁹R¹⁰ oder -C(=O)NR⁹R¹⁰ substituiert sein können,
   worin

- R⁹ und R¹⁰: unabhängig voneinander für Wasserstoff oder (C₁-C₈)-Alkyl stehen
oder
- R⁹ und R¹⁰: gemeinsam mit dem Stickstoffatom, an das sie gebunden sind, einen
5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,

- Phenyl, 5- oder 6-gliedriges Heteroaryl, über ein Kohlenstoffatom gebundenes 5- bis 10- gliedriges Heterocyclyl,
   wobei Phenyl, Heteroaryl und Heterocyclyl ihrerseits durch Halogen, Cyano, Nitro, Hydroxycarbonyl, Amino, Trifluormethyl, gegebenenfalls mit Hydroxy substituiertes (C₁-C₆)-Alkyl, (C₁-C₆)-Alkoxy, (C₁-C₆)-Alkylamino, (C₁-C₆)-Alkanoyl, (C₁-C₆)-Alkoxycarbonyl, (C₁-C₆)-Alkanoylamino oder (C₁-C₆)-Alkoxycarbonylamino substituiert sein können,
- -NR¹¹R¹²,
   worin

- R¹¹ und R¹²: unabhängig voneinander für Wasserstoff, (C₁-C₆)-Alkyl, (C₃-C₈)-Cycloalkyl, (C₆-C₁₀)-Aryl oder 5- bis 10-gliedriges Heteroaryl stehen,
wobei Alkyl und Cycloalkyl ihrerseits durch Hydroxy, (C₁-C₆)-Alkoxy, (C₆-C₁₀)-Aryl oder 5- bis 10-gliedriges Heteroaryl substituiert sein können,
und
wobei Aryl und Heteroaryl ihrerseits durch Halogen, Hydroxy, Amino, Cyano, Trifluormethyl, (C₁-C₆)-Alkyl, (C₁-C₆)-Alkoxy, (C₁-C₆)-Alkylamino oder (C₁-C₆)-Alkanoylamino substituiert sein können,
oder
- R¹¹ und R¹²: gemeinsam mit dem Stickstoffatom, an das sie gebunden sind, einen 4- bis 6- gliedrigen Heterocyclus bilden, der ein weiteres Heteroatom O oder N im Ring enthalten kann und der durch Fluor, Hydroxy, Hydroxycarbonyl, (C₁-C₄)-Alkoxy, (C₁-C₄)-Alkyl, (C₁-C₄)-Alkanoyl oder (C₁-C₄)-Alkoxycarbonyl substituiert sein kann,

- und -C(=O)R¹⁹,
   worin

- R¹⁹: für (C₁-C₆)-Alkoxy, (C₁-C₆)-Alkylamino oder einen über ein Stickstoffatom gebundenen 5- bis 10-gliedrigen mono- oder bicyclischen Heterocyclus, der anneliert oder spirocyclisch ist und ein oder zwei weitere Heteroatome aus der Reihe N und/oder O im Ring aufweisen kann, steht,
wobei Alkylamino seinerseits mit einem 5- oder 6-gliedrigen Heterocyclyl substituiert sein kann,

- R⁴: Wasserstoff, Fluor oder Chlor bedeutet,
- R⁵: Wasserstoff oder Methyl bedeutet und ihre Salze, Hydrate, Hydrate der Salze und Solvate.

Besonders bevorzugt sind Verbindungen der Formel (I), worin
- -----: eine Doppelbindung bedeutet,
- X: -NR⁶-, -CH₂- oder -C(=O)- bedeutet,
worin
R⁶ für Wasserstoff oder Methyl steht,
- R¹: Wasserstoff, Fluor, Chlor, Cyano, Trifluormethyl, Difluormethyl, Methyl, Ethyl, Hydroxyethyl, Methoxyethyl oder Cyclopropyl bedeutet,
- R²: Fluor oder Chlor bedeutet,
- R³: einen Rest bedeutet, der ausgewählt ist aus der Gruppe von:

- Wasserstoff, Hydroxy, Halogen, Trifluormethyl, Pentafluorethyl,
- (C₁-C₆)-Alkyl, (C₁-C₆)-Alkoxy, (C₃-C₈)-Cycloalkyl,
   wobei Alkyl, Alkoxy und Cycloalkyl ihrerseits durch Hydroxy, (C₁-C₆)-Alkoxy oder -NR⁹R¹⁰ substituiert sein können,
   worin

- R⁹ und R¹⁰: unabhängig voneinander für Wasserstoff oder (C₁-C₈)-Alkyl stehen
oder
- R⁹ und R¹⁰: gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₃)-Alkyl oder (C₁-C₃)-Alkanoyl substituiert sein kann,

- Phenyl und 5- oder 6-gliedriges Heteroaryl,
   wobei Phenyl und Heteroaryl ihrerseits durch Halogen, Amino oder Trifluormethyl substituiert sein können,

- R⁴: Wasserstoff, Fluor oder Chlor bedeutet,
- R⁵: Wasserstoff oder Methyl bedeutet,
und ihre Salze, Hydrate, Hydrate der Salze und Solvate.

Ganz besonders bevorzugt sind Verbindungen der Formel (I), worin
- -----: eine Doppelbindung bedeutet,
- X: -NH- oder -CH₂- bedeutet,
- R¹: Wasserstoff, Chlor oder Methyl bedeutet,
- R²: Fluor bedeutet,
- R³: einen Rest bedeutet, der ausgewählt ist aus der Gruppe von Wasserstoff, Halogen, Trifluormethyl, gegebenenfalls durch Halogen substituiertes Phenyl und Pyridin,
- R⁴: Wasserstoff oder Fluor bedeutet,
- R⁵: Wasserstoff bedeutet,
und ihre Salze, Hydrate, Hydrate der Salze und Solvate.

Bevorzugt sind auch Verbindungen der Formel (I), worin R¹ Wasserstoff, Fluor, Chlor, Cyano, Trifluormethyl, Difluormethyl oder Methyl bedeutet.

Bevorzugt sind auch Verbindungen der Formel (I), worin R² Fluor bedeutet.

Bevorzugt sind auch Verbindungen der Formel (I), worin R³ einen Rest bedeutet, der ausgewählt ist aus der Gruppe von: Wasserstoff, Chlor, Trifluormethyl, para-Fluorphenyl und Pyridin.

Bevorzugt sind auch Verbindungen der Formel (I), worin R⁴ Wasserstoff oder Fluor bedeutet.

Bevorzugt sind auch Verbindungen der Formel (I), worin R⁵ Wasserstoff bedeutet.

Weiterer Gegenstand der vorliegenden Erfindung ist ein Verfahren zur Herstellung der Verbindungen der Formel (I), das dadurch gekennzeichnet ist, dass man

Verbindungen der Formel (II) 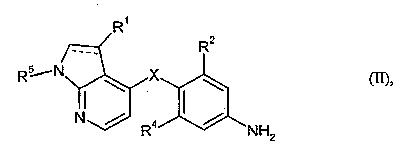 worin
-----, X, R¹, R², R⁴ und R⁵ die oben angegebene Bedeutung aufweisen, mit Verbindungen der Formel (III) 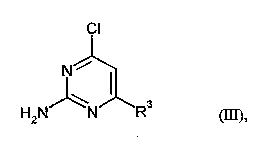 worin
- R³: die oben angegebene Bedeutung aufweist,
umsetzt,
wobei sich für den Fall, dass X für eine CH₂-Gruppe steht, eine Oxidation zu der entsprechenden Ketogruppe -C(=O)- oder eine Methylierung zu der entsprechenden Mono- bzw. Dimethylverbindung [ -CH(CH₃)- bzw. -C(CH₃)₂- ] anschließen kann.

Die Umsetzung erfolgt im Allgemeinen in wässriger salzsaurer Lösung, bevorzugt in einem Temperaturbereich von 70°C bis 110°C bei Normaldruck.

Die für den Fall, dass X für eine CH₂-Gruppe steht, gegebenenfalls stattfindene Oxidation oder Alkylierung kann nach üblichen, dem Fachmann geläufigen Methoden erfolgen.

Zur Herstellung der Verbindungen der Formel (II) werden beispielsweise Verbindungen der Formel (IV) 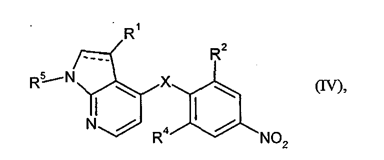 worin
- -----,: X, R¹, R² und R⁴ die oben angegebene Bedeutung aufweisen, und
- R⁵: die oben angegebene Bedeutung aufweist oder für eine [2-(Trimethylsilyl)ethoxy]methyl-Schutzgruppe steht,
mit Reduktionsmitteln umgesetzt.

Die Umsetzung erfolgt im Allgemeinen in inerten Lösungsmitteln, bevorzugt in einem Temperaturbereich von Raumtemperatur bis zum Rückfluss der Lösungsmittel bei Normaldruck bis 3 bar.

Reduktionsmittel sind beispielsweise Palladium auf Aktivkohle und Wasserstoff, Platinoxid und Wasserstoff, Zinndichlorid oder Titantrichlorid, bevorzugt ist Palladium auf Aktivkohle und Wasserstoff oder Platinoxid und Wasserstoff.

Inerte Lösungsmittel sind beispielsweise Ether wie Diethylether, Methyl-*tert*-butylether, 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol, *tert*-Butanol oder 2-Ethylhexanol, Kohlenwasserstoffe wie Benzol, Xylol, Toluol, Hexan, Cyclohexan oder Erdölfraktionen, oder andere Lösungsmittel wie Dimethylformamid, Dimethylacetamid, Acetonitril, Pyridin oder Essigsäure, als Lösungsmittel sind bevorzugt Ethanol, n-Butanol oder 2-Ethylhexanol.

Die 6-Position des Pyrrolopyridins in Verbindungen der Formal (IV) kann gegebenenfalls mit einem Chlor substituiert sein. Dieser Chlor-Substituent wird während der Reduktion abgespalten. Für den Fall, dass R⁵ in der Zielverbindung für Wasserstoff steht, kann das Stickstoffatom, an das R⁵ gebunden ist, gegebenenfalls während dieser Synthese mit einer [2-(Trimethylsilyl)-ethoxy]methyl-Schutzgruppe geschützt sein.

Die Abspaltung der Schutzgruppe erfolgt nach der Reduktion der Nitro-Gruppe mit Trifluoressigsäure in Dichlormethan in einem Temperaturbereich von Raumtemperatur bis 40°C bei Normaldruck. Gegebenenfalls wird eine Umsetzung mit einer Base wie beispielsweise Natrium-, Kalium- oder Lithiumhydroxid in THF in einem Temperaturbereich von Raumtemperatur bis 40°C bei Normaldruck zur vollständigen Abspaltung der Schutzgruppe angeschlossen.

Zur Herstellung der Verbindungen der Formel (IV) werden beispielsweise nach Verfahren [A] Verbindungen der Formel (V) 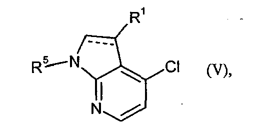 worin
- -----: und R¹ die oben angegebene Bedeutung aufweisen, und
- R⁵: die oben angegebene Bedeutung aufweist oder für eine [2-(Trimethylsilyl)ethoxy]methyl-Schutzgruppe steht,
mit Verbindungen der Formel (VI) 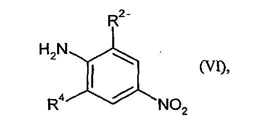 worin
R² und R⁴ die oben angegebene Bedeutung aufweisen,
zu Verbindungen der Formel (IVa) 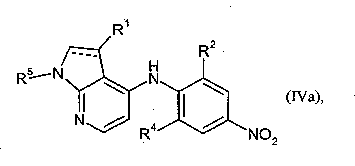 worin
-----, R¹, R² und R⁴ die oben angegebene Bedeutung aufweisen, und
R⁵ die oben angegebene Bedeutung aufweist oder für eine [2-(Trimethylsilyl)-ethoxy]methyl-Schutzgruppe steht,
umgesetzt,
oder
nach Verfahren [B] Verbindungen der Formel (VII) 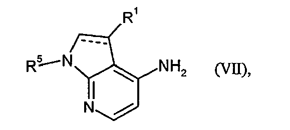 worin
----- und R¹ die oben angegebene Bedeutung aufweisen, und
R⁵ die oben angegebene Bedeutung aufweist oder für eine [2-(Trimethylsilyl)-ethoxy]methyl-Schutzgruppe steht,
mit Verbindungen der Formel (VIII) 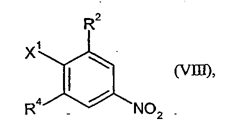
worin
R² und R⁴ die oben angegebene Bedeutung aufweisen, und
X¹ für Halogen, bevorzugt Iod, Chlor oder Brom steht, zu Verbindungen der Formel (IVa) umgesetzt,
oder
nach Verfahren [C] Verbindungen der Formel (IX) 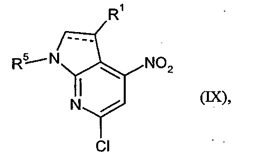 worin
----- und R¹ die oben angegebene Bedeutung aufweisen, und
R⁵ die oben angegebene Bedeutung aufweist oder für eine [2-(Trimethylsilyl)-ethoxylmethyl-Schutzgruppe steht, mit Verbindungen der Formel (X) 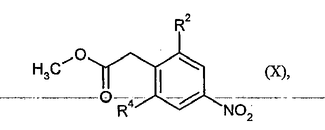 worin
R² und R⁴ die oben angegebene Bedeutung aufweisen,
und einer anschließenden Decarboxylierung des Esters
zu Verbindungen der Formel (IVb) 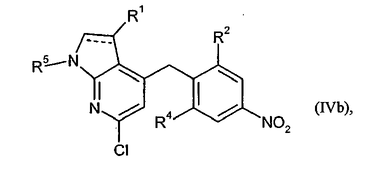 worin
-----, R¹, R² und R⁴ die oben angegebene Bedeutung aufweisen, und
R⁵ die oben angegebene Bedeutung aufweist oder für eine [2-(Trimethylsilyl)-ethoxy]methyl-Schutzgruppe steht,
umgesetzt.

Bei der gemäß Verfahrensvariante [A] oder [B] erhaltenen Verbindung (IVa) kann sich gegebenenfalls noch eine Alkylierung am verbrückenden Stickstoffatom nach üblichen, dem Fachmann geläufigen Methoden anschließen. Hierbei werden dann Verbindungen mit X = N-Alkyl erhalten.

Bei der gemäß Verfahrensvariante [C] erhaltenen Verbindung (IVb) kann sich gegebenenfalls noch an der verbrückenden Methylengruppe eine Oxidation zur entsprechenden Ketogruppe oder eine Alkylierung zu der entsprechenden Mono- bzw. Dimethylverbindung jeweils nach üblichen, dem Fachmann geläufigen Methoden anschließen. Hierbei werden dann Verbindungen mit X = -C(=O)-, -CH(CH₃)- bzw. -C(CH₃)₂- erhalten.

Die Verbindungen der Formeln (IVa) und (IVb) repräsentieren einen Teil der Verbindungen der Formel (IV).

Die Umsetzung nach Verfahren [A] und [B] erfolgt vorzugsweise nach dem Buchwald-Verfahren mit einer Palladiumverbindung wie beispielsweise Tris(dibenzylidenaceton)dipalladium; einem Phosphin wie beispielsweise Dieyclohexyl(2',4',6'-triisopropylbiphenyl-2-yl)phosphin oder 1,1'-Bis(diphenylphosphino)ferrocen und einer Base wie beispielsweise Natrium- oder Kalium-*tert-*butylat oder Natrium- oder Kaliumcarbonat in einem inerten Lösungsmittel wie beispielsweise *tert*-Butanol oder Toluol, in einem Temperaturbereich von 90°C bis 140°C bei Normaldruck oder leicht erhöhtem Druck.

Die Umsetzung nach Verfahren [C] erfolgt vorzugsweise in einem inerten Lösungsmittel wie beispielsweise *N,N-*Dimethylformamid, *N*-Methylpyrrolidon, Dimethylsulfoxid oder Nitrobenzol in Gegenwart einer Base wie beispielsweise Kaliumcarbonat, Kaliumhydroxid, Kalium-*tert*-butylat oder Natriumhydrid bei einer Temperatur von 120°C bis 280°C bei Normaldruck.

Die Decarboxylierung im Verfahren [C] erfolgt durch Umsetzung mit einer Base wie beispielsweise Kaliumhydroxid, Natriumhydroxid oder Lithiumhydroxid in einem inerten Lösungsmittel wie beispielsweise Methanol oder Ethanol bei einer Temperatur von Raumtemperatur bis 40°C bei Normaldruck.

Die Verbindungen der Formeln (III), (V), (VI), (VII), (VIII), (IX) und (X) sind dem Fachmann an sich bekannt oder lassen sich nach üblichen literaturbekannten Verfahren herstellen.

Die Herstellung der erfindungsgemäßen Verbindungen kann durch folgende Syntheseschemata verdeutlicht werden.

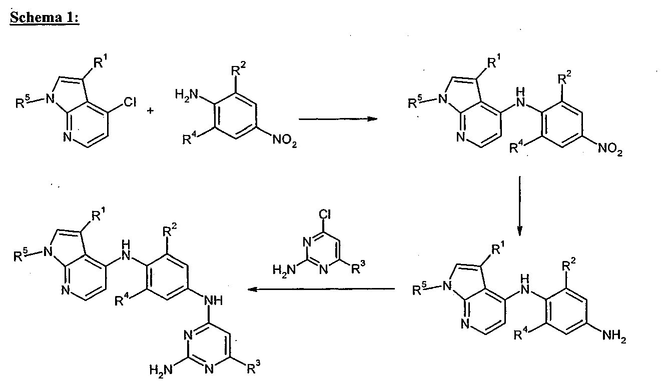

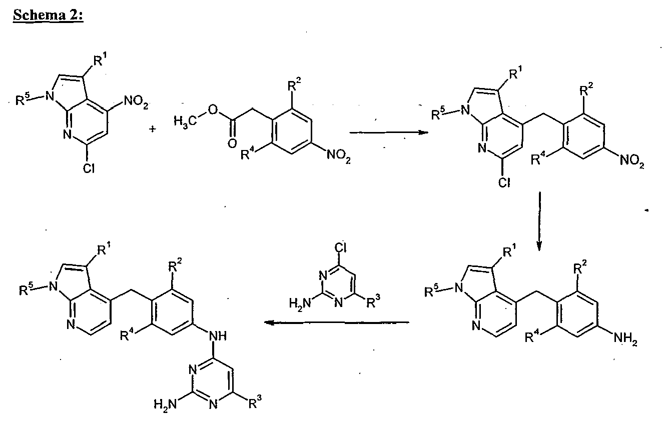

Die erfindungsgemäßen Verbindungen zeigen ein nicht vorhersehbares, wertvolles pharmakologisches und pharmakokinetisches Wirkspektrum.

Sie eignen sich daher zur Verwendung als Arzneimittel zur Behandlung und/oder Prophylaxe von Krankheiten bei Menschen und Tieren.

Die pharmazeutische Wirksamkeit der erfindungsgemäßen Verbindungen lässt sich durch ihre Wirkung als Rho-Kinase-Inhibitoren erklären.

Weiterer Gegenstand der vorliegenden Erfindung ist der Einsatz der erfindungsgemäßen Verbindungen zur Behandlung und/oder Prophylaxe von Erkrankungen, vorzugsweise von kardiovaskulären Erkrankungen.

Die erfindungsgemäßen Verbindungen sind geeignet für die Prophylaxe und/oder Behandlung von kardiovaskulären Erkrankungen wie beispielsweise Bluthochdruck und Herzinsuffizienz, stabiler und instabiler Angina pectoris, peripheren und kardialen Gefäßerkrankungen, von Arrhythmien, von thromboembolischen Erkrankungen und Ischämien wie Myokardinfarkt, Hirnschlag, transitorischen und ischämischen Attacken, peripheren Durchblutungsstörungen, Subarachnoidalblutungen, Verhinderung von Restenosen wie beispielsweise nach Thrombolysetherapien, percutanen transluminalen Angioplastien (PTA), percutanen transluminalen Koronarangioplastien (PTCA), Bypass sowie zur Prophylaxe und/oder Behandlung von Arteriosklerose, Alzheimer, Niereninsuffizienz, Glaukom, asthmatischen Erkrankungen, COPD und Krankheiten des Urogenitalsystems wie beispielsweise Prostatahypertrophie, erektiler Dysfunktion, weiblicher sexueller Dysfunktion, Osteoporose, Gastroparese und Inkontinenz.

Weiterhin können die erfindungsgemäßen Verbindungen zur Prophylaxe und/oder Behandlung von Krebserkrankungen, insbesondere von Tumoren eingesetzt werden.

Im Rahmen der vorliegenden Erfindung umfasst die Definition von Tumoren sowohl benigne, wie auch maligne Tumore und damit beispielsweise auch benigne Neoplasien, Dysplasien, Hyperplasien, wie auch Neoplasien mit Metastasenbildung. Weitere Beispiele für Tumore sind Karzinome, Sarkome, Karzinosarkoine, Tumore der blutbildenden Organe,Tumore des Nervengewebes z.B. des Gehirns oder Tumore von Hautzellen. Bei der Tumorbildung kommt es zur unkontrollierten oder unzureichend kontrollierten Zellteilung. Der Tumor kann örtlich begrenzt sein, er kann aber auch das umliegende Gewebe infiltrieren und sich dann durch das lymphatische System oder durch den Blutstrom an einem neuen Ort festsetzen. Somit gibt es primäre und sekundäre Tumore. Primäre Tumore sind ursprünglich in dem Organ entstanden, in dem sie gefunden werden. Sekundäre Tumore haben sich durch Metastäsenbildung in einem anderen Organ festgesetzt und sich dann an ihrem neuen Ort ausgebreitet.

Weiterer Gegenstand der vorliegenden Erfindung ist die Verwendung der erfindungsgemäßen Verbindungen zur Prophylaxe und/oder Behandlung von Erkrankungen, insbesondere der zuvor genannten Krankheitsbilder.

Weiterer Gegenstand der vorliegenden Erfindung ist die Verwendung der erfindungsgemäßen Verbindungen zur.Herstellung eines Arzneimittels zur Prophylaxe und/oder Behandlung von Erkrankungen, insbesondere der zuvor genannten Krankheitsbilder.

Weiterer Gegenstand der vorliegenden Erfindung sind Arzneimittel, enthaltend eine erfindungsgemäße Verbindung in Kombination mit einem oder mehreren weiteren Wirkstoffen, insbesondere zur Prophylaxe und/oder Behandlung der zuvor genannten Erkrankungen.

Die erfindungsgemäße Verbindung kann systemisch und/oder lokal wirken. Zu diesem Zweck kann sie auf geeignete Weise appliziert werden, wie z.B. oral, parenteral, pulmonal, nasal, sublingual, lingual, buccal, rectal, transdermal, conjunctival, otisch, als Stents oder als Implantat.

Für diese Applikationswege kann die erfindungsgemäße Verbindung in geeigneten Applikationsformen verabreicht werden.

Für diese Applikationswege kann die erfindungsgemäße Verbindung in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/oder amorphisierter und/oder gelöster Form enthalten, wie z.B. Tabletten (nicht überzogene oder überzogene Tabletten, beispielsweise mit magensaftresistenten, sich verzögert auflösenden oder unlöslichen Überzügen, die die Freisetzung der erfindungsgemäßen Verbindungen kontrollieren), in der Mundhöhle schnell zerfallende Tabletten oder Filme/Oblaten, Kapseln, Dragees, Granulate, Pellets, Pulver, Emulsionen, Suspensionen, Aerosole oder Lösungen.

Die parenterale Applikation kann unter Umgehung eines Resorptionsschrittes geschehen (intravenös, intraarteriell, intrakardial, intraspinal oder intralumbal) oder unter Einschaltung einer Resorption (intramuskulär, subcutan, intracutan, percutan, oder intraperitoneal). Für die parenterale Applikation eignen sich als Applikationsformen u.a. Injektions- und Infusionszubereitungen in Form von Lösungen, Suspensionen, Emulsionen, Lyophilisaten und sterilen Pulvern.

Für die sonstigen Applikationswege eignen sich z.B. Inhalationsarzneiformen (u.a. Pulverinhalatoren, Nebulizer), Nasentropfen/-lösungen, Sprays; lingual, sublingual oder buccal zu applizierende Tabletten oder Kapseln, Suppositorien, Ohren- und Augenpräparationen, Vaginalkapseln, wässrige Suspensionen (Lotionen, Schüttelmixturen), lipophile Suspensionen, Salben, Cremes, Milch, Pasten, Streupuder, Stents oder Implantate.

Die erfindungsgemäßen Verbindungen können in an sich bekannter Weise in die angeführten Applikationsformen überführt werden. Dies geschieht unter Verwendung inerter nichttoxischer, pharmazeutisch geeigneter Hilfsstoffe. Hierzu zählen u.a. Trägerstoffe (z.B. mikrokristalline Cellulose), Lösungsmittel (z.B. flüssige Polyethylenglycole), Emulgatoren (z.B. Natriumdodecylsulfat), Dispergiermittel (z.B. Polyvinylpyrrolidon), synthetische und natürliche Biopolymere (z.B. Albumin), Stabilisatoren (z.B. Antioxidantien wie Ascorbinsäure), Farbstoffe (z.B. anorganische Pigmente wie Eisenoxide) oder Geschmacks- und/oder Geruchskorrigentien.

Weiterer Gegenstand der vorliegenden Erfindung sind Arzneimittel, die mindestens eine erfindungsgemäße Verbindung, vorzugsweise zusammen mit einem oder mehreren inerten nichttoxischen, pharmazeutisch geeigneten Hilfsstoff enthalten, sowie deren Verwendung zu den zuvor genannten Zwecken.

Im Allgemeinen hat es sich sowohl in der Human- als auch in der Veterinärmedizin als vorteilhaft erwiesen, die erfindungsgemäße Verbindung in Gesamtmengen von etwa 0.01 bis etwa 700, vorzugsweise 0.01 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält die erfindungsgemäße Verbindung vorzugsweise in Mengen von etwa 0.1 bis etwa 80, insbesondere 0.1 bis 30 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Abkürzungen:

- DC: Dünnschichtchromatographie
- DCI: direkte chemische Ionisation (bei MS)
- DCM: Dichlormethan
- DIEA: *N,N*-Diisopropylethylamin
- DMA: *N,N*-Dimethylacetamid
- DMF: *N,N*-Dimethylformamid
- DMSO: Dimethylsulfoxid
- d. Th.: der Theorie
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- h: Stunde
- HATU: *O*-(7-Azabenzotriazol-1-yl)-*N,N,N',N'*-tetramethyluroniumhexafluorphosphat
- HOAT: 3H-[1,2,3]-Triazol[4,5-b]pyridin-3-ol
- HOBt: 1-Hydroxy-1H-benzotriazol x H₂O
- HPLC: Hochdruck-, Hochleistungsflüssigchromatographie
- konz.: konzentriert
- LC-MS: Flüssigchromatographie-gekoppelte Massenspektroskopie
- LDA: Lithiumdiisopropylamid
- min: Minuten
- MPLC: Mitteldruck-, Mittelleistungsflüssigchromatographie
- MS: Massenspektroskopie
- NMR: Kernresonanzspektroskopie
- org.: organisch
- proz.: prozentig
- quant.: quantitativ
- RF: Rückfluss
- R_{f}: Retentionsfaktor (bei DC)
- RP-HPLC: Reverse Phase HPLC
- RT: Raumtemperatur
- Rₜ: Retentionszeit (bei HPLC)
- TFA: Trifluoressigsäure
- THF: Tetrahydrofuran

### HPLC-, LCMS- und GCMS-Methoden:

**Methode 1 (LC/MS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.
**Methode 2 (LC/MS):** Instrument: Micromass Quattro LCZ mit HPLC Agilent Serie 1100; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 208- 400 nm.
**Methode 3 (LC-MS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min. 2 ml/min; Ofen: 50°C; UV-Detektion: 210 nm.
**Methode 4 (HPLC):** Instrument: HP 1100 mit DAD-Detektion; Säule: Kromasil 100 RP-18, 60 mm x 2.1 mm, 3.5 µm; Eluent A: 5 ml Perchlorsäure (70%ig)/l Wasser, Eluent B: Acetonitril; Gradient: 0 min 2%B, 0.5 min 2%B, 4.5 min 90%B, 6.5 min 90%B, 6.7 min 2%B, 7.5 min 2%B; Fluss: 0.75 ml/min; Ofen: 30°C; UV-Detektion: 210 nm.
**Methode 5 (LC-MS):** Instrument: Micromass Platform LCZ mit HPLC Agilent Serie 1100; Säule: Thermo HyPURITY Aquastar 3µ 50 mm x 2.1 mm; Eluent A: 1 l Wasser + 0.5 ml 50%ige Ameisensäure, Eluent B: 1 l Acetonitril + 0.5 ml 50%ige Ameisensäure; Gradient: 0.0 min 100%A → 0.2 min 100%A → 2.9 min 30%A → 3.1 min 10%A → 5.5 min 10%A; Ofen: 50°C; Fluss: 0.8 ml/min; UV-Detektion: 210 nm.
**Methode 6 (LC/MS):** Instrument: Micromass Platform LCZ mit HPLC Agilent Serie 1100; Säule: Phenomenex Synergi 2µ Hydro-RP Mercury 20 mm x 4 mm; Eluent A: 1 l Wasser + 0.5 ml 50%ige Ameisensäure, Eluent B: 1 l Acetonitril + 0.5 ml 50%ige Ameisensäure; Gradient: 0.0 min 90%A → 2.5 min 30%A → 3.0 min 5%A → 4.5 min 5%A; Fluss: 0.0 min 1 ml/min, 2.5 min/3.0 min/4.5 min 2 ml/min; Ofen: 50°C; UV-Detektion: 210 nm.
**Methode 7 (LC/MS):** Gerätetyp MS: Micromass ZQ; Gerätetyp HPLC: Waters Alliance 2790; Säule: Grom-Sil 120 ODS-4 HE 50 mm x 2 min, 3.0 µm; Eluent B: Acetonitril + 0.05% Ameisensäure, Eluent A: Wasser + 0.05% Ameisensäure; Gradient: 0.0 min 5%B → 2.0 min 40%B → 4.5 min 90%B → 5.5 min 90%B; Ofen: 45°C; Fluss: 0.0 min 0.75 ml/min → 4.5 min 0.75 ml/min→ 5.5 min 1.25 ml/min; UV-Detektion: 210 nm.
**Methode 8 (LCMS):** Instrument: Micromass Quattro LCZ, mit HPLC Agilent Serie 1100; Säule: Grom-SIL120 ODS-4 HE, 50 mm x 2.0 mm, 3 µm; Eluent A: 1 l Wasser + 1 ml 50%ige Ameisensäure, Eluent B: 1 l Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.
**Methode 9 (LCMS):** Gerätetyp MS: Micromass ZQ; Gerätetyp HPLC: Waters Alliance 2795; Säule: Merck Chromolith SpeedROD RP-18e 50 mm x 4.6 mm; Eluent A: Wasser + 500 µl 50%ige Ameisensäure/l; Eluent B: Acetonitril + 500 µl 50%ige Ameisensäure/l; Gradient: 0.0 min 10%B → 3.0 min 95%B → 4.0 min 95%B; Ofen: 35°C; Fluss: 0.0 min 1.0 ml/min → 3.0 min 3.0 ml/min → 4.0 min 3.0 ml/min; UV-Detektion: 210 nm.
**Methode 10 (LCMS):** Instrument: Micromass Platform LCZ mit HPLC Agilent Serie 1100; Säule: Grom-SIL120 ODS-4 HE, 50 mm x 2.0 mm, 3 µm; Eluent A: 1 l Wasser + 1 ml 50%ige Ameisensäure, Eluent B: 1 l Acetonitril + 1 ml 50%ige Ameisensäure; Gradient: 0.0 min 100%A → 0.2 min 100%A → 2.9 min 30%A → 3.1 min 10%A → 4.5 min 10%A; Ofen: 55°C; Fluss: 0.8 ml/min; UV-Detektion: 208-400 nm.
**Präparative HPLC:** Säule: YMC Gel ODS-AQ S-5 / 15 µ→M, 250 mm x 30 mm, Eluent A: Wasser, Eluent B: Acetonitril; Gradient: 0.00 min 30%B → 3.00 min 30%B → 34.0 min 95%B → 38.0 min 30%B; Temp.: Raumtemperatur; Fluss: 50 ml/min; UV-Detektion.

### Ausgangsverbindungen

### Beispiel 1A

### 1H-Pyrrolo[2,3-b]pyridin-7-oxid

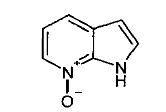

(Antonini, Ippolito; Claudi, Francesco; Cristalli, Gloria; Franchetti, Palmarisa; Grifantini, Mario; Martelli, Sante; J. Med. Chem. 1982, 25, 1258-1261.) 540 g (2.35 mol) 3-Chlorperbenzoesäure werden in 6.11 1 Dichlormethan gelöst, und abgeschiedenes Wasser wird abgetrennt. Die organische Phase wird über Natriumsulfat getrocknet und auf 0°C gekühlt. Dann gibt man eine Lösung aus 163 g (1.38 mol) 1H-Pyrrolo[2,3-b]pyridin (Hands, D.; Bishop, B.; Cameron, M.; Edwards, T.S.; Cottrell, I.F.; Wright, S.H.B.; Synthesis 1996, 877-882.) in 1.001 Dichlormethan zu und lässt die Temperatur auf Raumtemperatur ansteigen. Nach 2 Stunden gibt man soviel Methanol zu, dass sich der gebildete Niederschlag wieder auflöst. Die Mischung wird über Kieselgel filtriert (Eluens: Dichlormethan/Methanol 95:5) und die Produktfraktionen nach Einengen im Hochvakuum getrocknet.
Ausbeute: 145 g (75% d. Th.)
HPLC (Methode 4): Rₜ = 2.02 min.
MS (ESI pos.): m/z = 135 [M+H]⁺, 152 [M+NH₄]⁺, 269 [2M+H]⁺.
¹H-NMR (DMSO-d₆, 200 MHz): δ = 6.58 (d, 1H), 7.07 (dd, 1H), 7.48 (d, 1H), 7.65 (d, 1H), 8.17 (d, 1H), 12.42-12.64 (br. s, 1H).

### Beispiel 2A

### 4-Nitro-1H-pyrrolo[2,3-b]pyridin-7-oxid

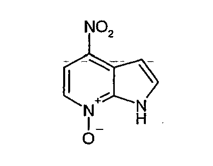

Eine Durchführung größerer Ansätze als die fünffache Menge der beschriebenen Größe ist aufgrund der Ergebnisse der Differentialthermoanalyse nicht empfehlenswert.

Eine Lösung von 20.0 g (149 mmol) 1H-Pyrrolo[2,3-b]pyridin-7-oxid in 160 ml Trifluoressigsäure wird auf Raumtemperatur gekühlt. Anschließend tropft man langsam 69.3 ml 65%ige Salpetersäure zu und lässt 2 h bei Raumtemperatur rühren. Man gießt auf Eis und stellt mit 45%iger Natriumhydroxid-Lösung einen pH-Wert von 8-9 ein. Der Niederschlag wird abgesaugt und mit Wasser gewaschen. Es werden die Rohprodukte aus 4 Ansätzen der beschriebenen Größe und einem analog durchgeführten 13 g-Ansatz vereinigt und gemeinsam gereinigt. Die Rohprodukte werden in Wasser aufgeschlämmt und mit 2N Natriumhydroxid-Lösung auf pH 8-9 eingestellt. Nach 10 min Rühren wird der Niederschlag abgesaugt und im Hochvakuum getrocknet. (Schneller, Stewart W.; Luo, Jiann-Kuan; J. Org. Chem. 1980, 45, 4045-4048.)
Ausbeute: 29.7 g (24% d. Th.)
HPLC (Methode 4): Rₜ = 3.02 min.
MS (ESI pos.): m/z = 180 (M+H)⁺, 197 (M+NH4)⁺, 359 (2M+H)⁺.
¹H-NMR (DMSO-d₆, 200 MHz): δ = 7.03 (d, 1H), 7.80 (d, 1H), 8.03 (d, 1H), 8.31 (d, 1H), 13.12-13.41 (br. s, 1H).

### Beispiel 3A

### 6-Chlor-4-nitro-1H-pyrrolo[2,3-b]pyridin

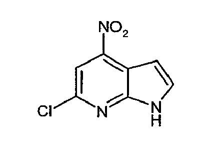

5.00 g (27.9 mmol) 4-Nitro-1H-pyrrolo[2,3-b]pyridin-7-oxid und 11.8 ml (55.8 mmol) Hexamethyldisilazan werden in 290 ml THF unter einer Argonatmosphäre vorgelegt. 10.8 ml (140 mmol) Chlorameisensäuremethylester werden bei RT zugegeben. Die Lösung wird über Nacht bei RT gerührt. Die Reaktionslösung wird über eine Kieselgelkartusche filtriert und mit Dichlormethan/Methanol 10:1 nachgewaschen.
Ausbeute: 2.8 g (70% d. Th.)
LC-MS (Methode 3): Rₜ = 2.15 min.
MS (ESI pos.): m/z = 198 (M+H)⁺.
1H-NMR (DMSO-d₆, 200 MHz): δ = 7.00 (m, 1H), 7.97 (s, 1H), 8.00 (t, 1H), 12.79 (s, 1H).

### Beispiel 4A

### 6-Chlor-4-nitro-1-{[2-(trimethylsilyl)ethoxy]methyl}-1H-pyrrolo[2,3-b]pyridin

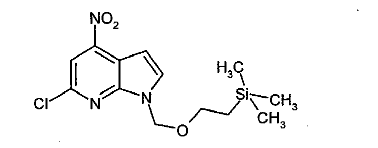

Zu einer Lösung von 5.6 g (28.3 mmol) 6-Chlor-4-nitro-1H-pyrrolo[2,3-b]pyridin in Dimethylformamid (50 ml) werden bei 0°C in einer Argonatmosphäre 1.34 g (28.3 mmol) Natriumhydrid (60% in Mineralöl) in kleinen Portionen zugegeben, und die Suspension wird 30 Minuten bei RT gerührt. Man kühlt wieder auf 0°C, tropft 5.3 ml (29.8 mmol) 2-(Trimethylsilyl)ethoxymethylchlorid hinzu und lässt 2 h bei RT nachrühren. Man gießt die Suspension auf Eiswasser (250 ml) und lässt rühren bis das Gemisch RT erreicht hat. Dann extrahiert man mit Essigsäureethylester (dreimal 50 ml) und wäscht die organische Phase mit gesättigter Natriumchlorid-Lösung. Die Lösung wird über Magnesiumsulfat getrocknet und eingeengt. Man reinigt den Rückstand durch Säulenchromatographie an Kiesegel (Eluens: Cyclohexan/Essigsäureethylester 6:1).
Ausbeute: 6.88 g (74% d. Th.)
1H-NMR (DMSO-d₆, 300 MHz): δ = -0.09 (s, 9H), 0.83 (t, 2H), 3.54 (t, 2H), 5.67 (s, 2H), 7.06 (d,
1H), 8.02 (s, 1H), 8.12 (d, 1H).

### Beispiel 5A

### 4-Nitro-1-{[2-(trimethylsilyl)ethoxy]methyl}-1H-pyrrolo[2,3-b]pyridin

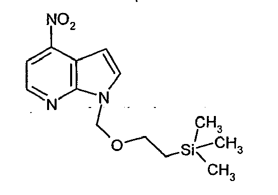

Zu einer Lösung von 980 mg (6.01 mmol) 4-Nitro-1H-pyrrolo[2,3-b]pyridin (Antonini, Ippolito; Claudi, Francesco; Cristalli, Gloria; Franchetti, Palmarisa; Grifantini, Mario; Martelli, Sante; J. Med. Chem. 1982, 25, 1258-1261.) in DMF (10 ml) werden 240 mg (6.01 mmol) Natriumhydrid gegeben. Nach Rühren bei RT für 30 min werden 1.05 g (6.31 mmol) (Trimethylsilyl)-ethoxymethylchlorid zugegeben und die Reaktionsmischung für 1 h bei RT gerührt. Nach Einengen im Vakuum wird der Rückstand durch Säulenchromatographie an Kieselgel (Eluens: Cyclohexan/Essigsäureethylester 10:1) gereinigt. Einengen liefert das Zielprodukt.
Ausbeute: 1.22 g (69% d. Th.)
LC-MS (Methode 7): Rₜ = 4.15 min.
MS (ESI pos.): m/z = 294 (M+H)⁺.
¹H-NMR (DMSO-d₆, 300 MHz): δ = -0.09 (s, 9H), 0.93 (t, 2H), 3.63 (t, 2H), 5.84 (s, 2H), 7.18 (d, 1H), 8.08 (d, 1H), 8.22 (d, 1H), 8.68 (d, 1H).

### Beispiel 6A

### 1-{[2-(Trimethylsilyl)ethoxy]methyl}-1H-pyrrolo[2,3-b]pyridin-4-amin

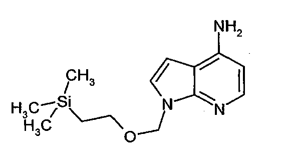

1.30 g (3.97 mmol) 6-Chlor-4-nitro-1-{[trimethylsilyl)ethoxy]methy}-1H-pyrrolo[2,3-b]pyridin werden in 10 ml Ethanol/THF (1:1) gelöst. Man gibt 90 mg (0.4 mmol) Platin(IV)oxid hinzu und hydriert über Nacht in einer Wasserstoffatmosphäre unter normalem Druck. Dann wird die gleiche Menge Katalysator nochmals hinzugesetzt und eine weitere Nacht hydriert. Man filtriert durch Celite und wäscht mit Ethanol nach. Das Lösungsmittel wird entfernt, und der Rückstand wird in Ethanol (10 ml) aufgenommen und mit 0.55 ml (3.97 mmol) Triethylamin versetzt. Man setzt 422 mg (0.40 mmol) 10%iges Palladium auf Aktivkohle hinzu und reduziert über Nacht bei einem Wasserstoffdruck von 3.5 bar. Man filtriert durch Celite, wäscht mit Ethanol nach und entfernt das Lösungsmittel im Vakuum. Man reinigt durch Säulenchromatographie an Kieselgel (Eluens: Dichlormethan/Aceton 20:1 bis 10:1). Das erhaltene Produkt wird mit Pentan verrührt und abgesaugt.
Ausbeute: 548 mg (46% d. Th.)

### Alternatives Herstellungsverfahren:

Eine Mischung von 900 mg (3.07 mmol) 4-Nitro-1-{[2-(trimethylsilyl)ethoxy]methyl}-1H-pyrrolo[2,3-b]pyridin und Platin(IV)oxid in 30 ml Ethanol/THF (1:1) wird über Nacht in einer Wasserstoffatmosphäre bei Normaldruck gerührt. Anschließend wird die Suspension über Celite filtriert und mit Ethanol gewaschen. Einengen des Filtrats liefert das Zielprodukt.
Ausbeute: 760 mg (94% d. Th.)
LC-MS (Methode 3): Rₜ = = 1.49 min.
MS (ESI pos.): m/z = 264 [M+H]⁺.
¹H-NMR (DMSO-d₆, 300 MHz): δ =-0.10 (s, 9H), 0.80 (t, 2H), 3.47 (t, 2H), 5.47 (s, 2H), 6.15 (br. s, 2H), 6.18 (d, 1H), 6.55 (d, 1H), 7.17 (d, 1H), 7.75 (d, 1H).

### Beispiel 7A

### N-(2-Fluor-4-nitrophenyl)-1-{[2-(trimethylsilyl)ethoxy]methyl}-1H-pyrrolo[2,3-b]pyridin-4-amin

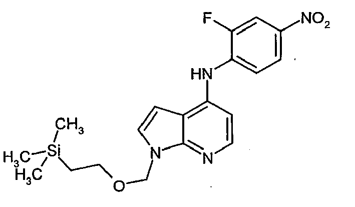

50 mg (0.19 mmol) 1-{[2-(Trimethylsilyl)ethoxy]methyl}-1H-pyrrolo[2,3-b]pyridin-4-amin und 61 mg (0.23 mmol) 2-Fluor-1-iod-4-nitrobenzol werden mit 26 mg (0.27 mmol) Natrium-*tert*-butylat in 1 ml Toluol vorgelegt. Das Gemisch wird entgast. Dann werden 8.7 mg (0.01 mmol) Tris(dibenzylidenaceton)dipalladium und 9.1 mg (0.02 mmol) Dicyclohexyl(2',4',6'-triisopropylbiphenyl-2-yl)phosphin zugesetzt. Man erhitzt über Nacht im geschlossenen Gefäß auf 120°C. Dann wird über eine Extrelutkartusche filtriert (Eluens: Dichlormethan/Methanol 10:1) und durch präparative HPLC aufgereinigt.
Ausbeute: 38 mg (50% d. Th.)
LC-MS (Methode 3): Rₜ = 2.73 min.
MS (ESI pos.): m/z = 403 [M+H]⁺.
¹H-NMR (DMSO-d₆, 300 MHz): δ = -0.09 (s, 9H), 0.82 (t, 2H), 3.52 (t, 2H), 5.60 (s, 2H), 6.50 (d, 1H), 6.84 (d, 1H), 7.37 (t, 1H), 7.50 (d, 1H), 8.04 (dd, 1H), 8.13 (d, 1H), 8.17 (dd, 1H), 9.33 (s, 1H).

### Beispiel 8A

### N-(2-Fluor-4-nitrophenyl)-1H-pyrrolo[2,3-b]pyridin-4-amin

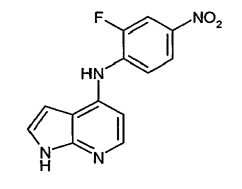

Eine Lösung von 500 mg (3.28 mmol) 4-Chlor-1H-pyrrolo[2,3-b]pyridin (Schneller, Stewart, W.; Luo, Jiann-Kuan; J. Org. Chem. 1980, 45, 4045-4048.), 614 mg (3.93 mmol) 2-Fluor-4-nitroanilin, 150 mg (0.16 mmol) Tris(dibenzylidenaceton)dipalladium und 156 mg (0.33 mmol) Dicyclohexyl(2',4',6'-triisopropylbiphenyl-2-yl)phosphin und 996 mg (7.21 mmol) Kaliumcarbonat in 5.00 ml entgastem *tert*-Butanol wird für 3 h im geschlossenen Druckgefäß bei 100°C gerührt.

Nach Abkühlen auf RT wird die Mischung über Celite filtriert, mit Essigsäureethylester nachgewaschen und die Filtrate im Vakuum eingeengt. Der Rückstand wird durch Säulenchromatographie an Kieselgel gereinigt (Eluens: Cyclohexan/Essigsäureethylester 1:1).
Ausbeute: 694 mg (78% d. Th.)

### Alternative Herstellungsmethode:

250 mg (0.62 mmol) *N*-(2-Fluor-4-nitrophenyl)-1-{[2-(trimethylsilyl)ethoxy]methyl}-1H-pyrrolo-[2,3-b]pyridin-4-amin werden in 2.5 ml Dichlormethan gelöst. Man versetzt mit 2.5 ml Trifluoressigsäure und lässt 3 Stunden bei RT rühren. Die Reaktionslösung wird vollständig eingeengt. Den Kolbenrückstand nimmt man in THF auf und gibt 6.2 ml (6.2 mmol) 1M Lithiumhydroxid-Lösung hinzu. Man lässt über Nacht bei RT rühren, setzt nochmals 3.1 ml (3.1 mmol) 1M Lithiumhydroxid-Lösung zu und lässt 4 Stunden rühren. Die Reaktionslösung wird mit 1M Zitronensäure versetzt und zweimal mit Essigsäureethylester extrahiert. Die wässrige Phase wird mit Natronlauge alkalisch gestellt und zweimal mit Essigsäureethylester extrahiert. Dann werden die vereinigten organischen Phasen über Natriumsulfat getrocknet. Man entfernt das Lösungsmittel im Vakuum. Der Rückstand wird durch Säulenchromatographie an Kieselgel gereinigt (Eluens: Dichlormethan/Methanol 50:1 bis 5:2).
Ausbeute: 165 mg (98% d. Th.)
LC-MS (Methode 1): Rₜ = 1.11 min.
MS (ESI pos.): m/z = 273 (M+H)⁺.
¹H-NMR (DMSO-d₆, 300 MHz): δ = 6.39 (d, 1 H), 6.81 (m, 1 H), 7.28-7.37 (m, 2 H), 8.04 (dd, 1 H), 8.10 (d, 1 H), 8.18 (dd, 1 H), 9.32 (s, 1 H), 11.68 (s, 1 H).

### Beispiel 9A

### 2-Fluor-N¹-1H-pyrrolo[2,3-b]pyridin-4-yl-1,4-diaminobenzol

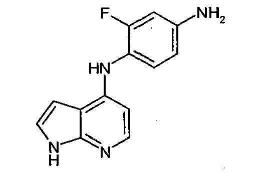

165 mg (0.61 mmol) *N*-(2-Fluor-4-nitrophenyl)-1H-pyrrolo[2,3-b]pyridin-4-amin werden in 10 ml Ethanol gelöst. Man setzt 65 mg (0.06 mmol) 10%iges Palladium auf Aktivkohle hinzu und hydriert über Nacht in einer Wasserstoffatmosphäre bei normalem Druck. Man filtriert über Celite und entfernt das Lösungsmittel im Vakuum.
Ausbeute: 139 mg (95% d. Th.)
LC-MS (Methode 2): Rₜ = 1.00 min.
MS (ESI pos.): m/z = 243 [M+H]⁺.
¹H-NMR (DMSO-d₆, 300 MHz): δ = 5.38 (s, 1H), 5.93 (d, 1H), 6.34-6.49 (m, 4H), 6.92-7.02 (m, 1H), 7.08 (d, 1H), 7.73 (d, 1 H), 8.0 (s, 1 H), 11.15 (s, 1H).

### Beispiel 10A

### 3-Chlor-N-(2-fluor-4-nitrophenyl)-1H-pyrrolo[2,3-b]pyridin-4-amin

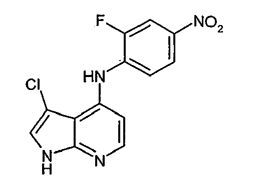

Zu einer Lösung von 200 mg (730 µmol) *N*-(2-Fluor-4-nitrophenyl)-1H-pyrrolo[2,3-b]pyridin-4-amin in 5 ml THF werden 108 mg (810 µmol) *N*-Chlorsuccinimid gegeben, und es wird über Nacht bei RT gerührt. Zur Vervollständigung der Reaktion werden weitere 49 mg (365 µmol) *N-*Chlorsuccinimid zugegeben und eine weitere Nacht gerührt. Anschließend wird die Reaktionsmischung mit gesättigter Natriumhydrogencarbonat-Lösung versetzt und mit Essigsäureethylester extrahiert. Nach Waschen der organischen Phasen mit gesättigter Natriumchlorid-Lösung wird mit Magnesiumsulfat getrocknet und das Lösungsmittel im Vakuum entfernt. Reinigung mittels Säulenchromatographie an Kieselgel (Eluens: Gradient Cyclohexan/Essigsäureethylester 1:1 bis Essigsäureethylester 100%) und präparativer RP-HPLC liefert die Zielverbindung.
Ausbeute: 125 mg (48% d. Th.)
LC-MS (Methode 1): Rₜ = 2.23 min.
MS (ESI pos.): m/z = 307 (M+H)⁺.
¹H-NMR (DMSO-d₆, 300 MHz): δ = 6.95 (d, 1H), 7.12 (dd, 1H), 7.58 (s, 1H), 7.98 (d, 1H), 8.16 (d, 1H), 8.22 (d, 1H), 8.97 (s, 1H), 12.08 (s, 1H).

### Beispiel 11A

### N¹-(3-Chlor-1H-pyrrolo[2,3-b]pyridin-4-yl)-2-fluorbenzol-1,4-diamin

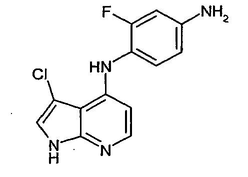

Zu einer Lösung von 40 mg (130 µmol) 3-Chlor-*N*-(2-fluor-4-nitrophenyl)-1H-pyrrolo[2,3-b]pyridin-4-amin in 2.50 ml DMF werden 147 mg (650 µmol) Zinn(II)-chlorid-Dihydrat gegeben und die Lösung bei RT über Nacht gerührt. Anschließend wird mit 5 ml Wasser versetzt und mit gesättigter Natriumhydrogencarbonat-Lösung ein pH-Wert von 10 eingestellt. Nach Filtration über Celite wird mit 40 ml Essigsäureethylester nachgewaschen. Vom Filtrat wird die wässrige Phase zweimal mit 10 ml Essigsäureethylester extrahiert. Die vereinigten organischen Phasen werden je zweimal mit 5 ml Wasser und 5 ml gesättigter Natriumchlorid-Lösung gewaschen und mit Natriumsulfat getrocknet. Einengen im Vakuum liefert die Zielverbindung, die ohne Reinigung weiter umgesetzt wird.
Ausbeute: 29 mg (8 1 % d. Th.)
LC-MS (Methode 1): Rₜ = 1.32 min.
MS (ESI pos.): m/z = 276 (M+H)⁺.

### Beispiel 12A

### 2-Fluor-N¹-(1-{[2-(trimethylsilyl)ethoxy]methyl}-1H-pyrrolo[2,3-b]pyridin-4-yl)benzol-1,4-diamin

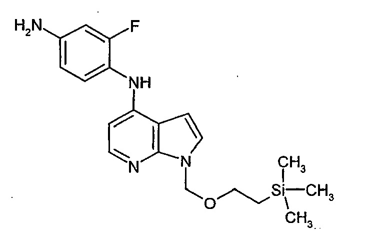

115 mg (0.29 mmol) *N*-(2-Fluor-4-nitrophenyl)-1-{[2-(trimethylsilyl)ethoxy]methyl}-1H-pyrrolo-[2,3-b]pyridin-4-amin werden in 10 ml einer Mischung von Ethanol/THF (1:1) gelöst. Man setzt 13 mg (0.06 mmol) Platin(IV)oxid hinzu und rührt über Nacht unter einer Wasserstoffatmosphäre bei normalem Druck. Anschließend wird die Suspension über Celite filtriert und das Lösungsmittel im Vakuum entfernt.
Ausbeute: 79 mg (74% d. Th.)
LC-MS (Methode 8): Rₜ = 2.26 min.
MS (ESI pos.): m/z = 373 (M+H)⁺.
¹H-NMR (DMSO-d₆, 300 MHz): δ = -0.09 (s, 9H), 0.88 (t, 2H), 3.57 (t, 2H), 5.43 (s, 2H), 5.59 (s, 2H), 6.08 (d, 2H), 6.46-6.64 (m, 3H), 7.07 (dd, 1H), 7.31 (d, 1H), 7.88 (d, 1H), 8.12 (s, 1H).

### Beispiel 13

### (2-Fluor-4-nitrophenyl)malonsäuredimethylester

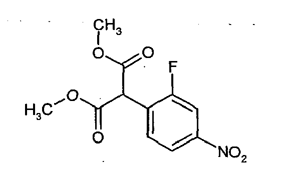

Zu einer Suspension aus 1.66 g (69.1 mmol) gewaschenem Natriumhydrid in 70 ml DMSO werden vorsichtig 9.13 g (69.1 mmol) Malonsäuredimethylester zugetropft. Man erhitzt 40 min auf 100°C, kühlt dann auf RT und setzt 5.00 g (31.4 mmol) 3,4-Difluornitrobenzol hinzu. Man lässt die Mischung 30 min bei RT rühren und erhitzt dann 1 Stunde auf 100°C. Die abgekühlte Reaktionsmischung wird auf eine Mischung aus Essigsäureethylester (50 ml), Cyclohexan (50 ml) und gesättigte Ammoniumchlorid-Lösung (250 ml) gegossen. Die wässrige Phase wird zweimal mit je 100 ml Essigsäureethylester/Cyclohexan (1:1) extrahiert. Die vereinigten organischen Phasen werden mit Wasser und gesättigter Natriumchlorid-Lösung gewaschen und über Natriumsulfat getrocknet. Man entfernt das Lösungsmittel im Vakuum und erhält ein Öl, das nach Zugabe von Cyclohexan durchkristallisiert. Man saugt ab und wäscht mit Cyclohexan gründlich nach und erhält einen Feststoff.
Ausbeute: 7.68 g (90% d. Th.)
LC-MS (Methode 2): Rₜ = 2.16 min.
MS (ESI neg.): m/z = 270 [M-H]⁻.
¹H-NMR (DMSO-d₆, 400 MHz): δ = 3.72 (s, 6H), 5.44 (s, 1H), 7.75 (dd, 1H), 8.13 (dd, 1H), 8.18 (dd, 1H).

### Beispiel 14A

### (2-Fluor-4-nitrophenyl)essigsäureethylester

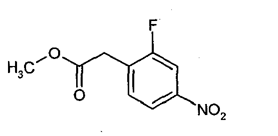

500 mg (1.84 mmol) (2-Fluor-4-nitrophenyl)malonsäuredimethylester werden in 10 ml DMSO gelöst. Man gibt 33.2 mg (1.84 mmol) Wasser und 313 mg (7.37 mmol) Lithiumchlorid hinzu und erhitzt 3 Stunden auf 100°C. Die Reaktionslösung wird dann auf 30 ml Essigsäureethylester gegossen und mit gesättigter Natriumchlorid-Lösung und mit verdünnter Salzsäure geschüttelt. Man trocknet über Natriumsulfat und entfernt das Lösungsmittel im Vakuum. Das erhaltene Öl wird durch präparative HPLC gereinigt. Die Produktfraktionen werden mit Essigsäureethylester extrahiert. Man trocknet die vereinigten organischen Phasen über Natriumsulfat und entfernt das Lösungsmittel im Vakuum. Es wird ein Öl erhalten, das nach Animpfen mit Produktkristallen rasch durchkristallisiert.
Ausbeute: 254 mg (63% d. Th.)
LC-MS (Methode 1): Rₜ = 1.92 min.
MS (ESI pos.): m/z = 214 [M+H]⁺.
¹H-NMR (DMSO-d₆, 300 MHz): δ = 3.65 (s, 3H), 3.92 (d, 2H), 7.69 (dd, 1H), 8.06-8.13 (m, 2H).

### Beispiel 15A

### (6-Chlor-1-{[2-(trimethylsilyl)ethoxy]methyl}-1H-pyrrolo[2,3-b]pyridin-4-yl)(2-fluor-4-nitrophenyl)essigsäuremethylester

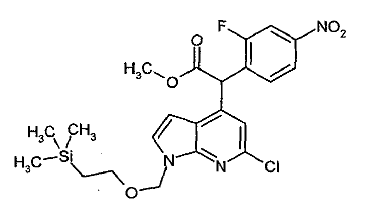

908 mg (4.26 mmol) (2-Fluor-4-nitrophenyl)essigsäureethylester werden in 10 ml DMF gelöst und mit 170 mg (4.26 mmol) Natriumhydrid (60%ig in Mineralöl) versetzt. Man lässt 30 min bei RT rühren und setzt dann 698 mg (2.13 mmol) 6-Chlor-4-nitro-1-{[2-(trimethylsilyl)ethoxy]methyl}-1H-pyrrolo[2,3-b]pyridin hinzu. 2 Stunden lang wird auf 70°C erhitzt, dann in Eiswasser eingerührt und mit Essigsäureethylester extrahiert. Die organische Phase wird mit Wasser und gesättigter Natriumchlorid-Lösung gewaschen, über Natriumsulfat getrocknet und im Vakuum eingeengt. Man reinigt durch Chromatographie an Kieselgel (Eluens: Dichlormethan/Pentan 2:1).
Ausbeute: 1.00 g (95% d. Th.)
LC-MS (Methode 2): Rₜ = 3.24 min.
MS (ESI pos.): m/z = 494, 496 [M+H]⁺.
¹H-NMR (DMSO-d₆, 400 MHz): δ = -0.12 (s, 9H), 0.80 (t, 2H), 3.52 (t, 2H), 3.74 (s, 3H), 5.57 (s, 2H), 6.02 (s, 1H), 6.60 (d, 1H), 7.15 (s, 1H), 7.57 (dd, 1H), 7.72 (d, 1H), 8.05 (dd, 1H), 8.17 (dd, 1H).

### Beispiel 16A

### 6-Chlor-4-(2-fluor-4-nitrobenzyl)-1-{[2-(trimethylsilyl)ethoxy]methyl}-1H-pyrrolo[2,3-b]pyridin

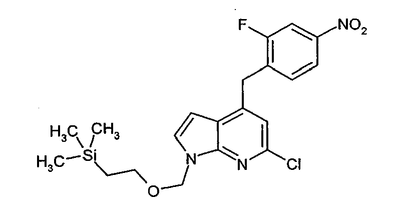

358 mg (0.72 mmol) (6-Chlor-1-{[2-(trimethylsilyl)ethoxy]methyl}-1H-pyrrolo[2,3-b]pyridin-4-yl)(2-fluor-4-nitrophenyl)essigsäuremethylester werden in 6 ml Methanol gelöst. Man versetzt mit 21 mg (0.87 mmol) Lithiumhydroxid und 2 Tropfen Wasser und lässt 20 Stunden bei RT rühren.

Man engt im Vakuum ein, setzt Dichlormethan und Wasser hinzu, trennt die organische Phase ab und trocknet sie über Natriumsulfat. Man entfernt das Lösungsmittel im Vakuum und reinigt durch Chromatographie an Kieselgel (Eluens: Dichlormethan). Man erhält ein Öl.
Ausbeute: 260 mg (82% d. Th.)
LC-MS (Methode 1): Rₜ = 3.14 min.
MS (ESI pos.): m/z = 436, 438 [M+H]⁺.
¹H-NMR (DMSO-d₆, 400 MHz): δ = -0.12 (s, 9H), 0.81 (t, 2H), 3.50 (t, 2H), 4.42 (s, 2H), 5.55 (s, 2H), 6.64 (d, 1H), 7.05 (s, 1H), 7.65-7.70 (m, 2H), 8.06 (dd, 1H), 8.12 (dd, 1H).

### Beispiel 17A

### 3-Fluor-4-[(1-{[2-(trimethylsilyl)ethoxy]methyl}-1H-pyrrolo[2,3-b]pyridin-4-yl)methyl]anilin

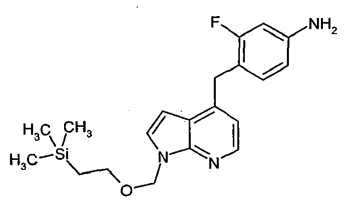

270 mg (0.62 mmol) 6-Chlor-4-(2-fluor-4-nitrobenzyl)-1-{[2-(trimethylsilyl)ethoxy]-methyl}-1H-pyrrolo[2,3-b]pyridin werden in 2 ml Ethanol gelöst. Man setzt 170 µl (1.24 mmol) Triethylamin und 50 mg 10%iges Palladium auf Kohle hinzu. Es wird 20 h bei RT und 1.5 bar Wasserstoffatmosphäre hydriert. Man filtriert, engt ein, versetzt mit Essigsäureethylester und wäscht mit 1N Natronlauge. Die organische Phase wird über Natriumsulfat getrocknet und eingeengt. Das Produkt wird ohne Aufreinigung weiter umgesetzt.
Ausbeute: 199 mg (87% d. Th.)
LC-MS (Methode 2): Rₜ = 2.81 min.
MS (ESI pos.): m/z = 372 [M+H]⁺.
¹H-NMR (DMSO-d₆, 400 MHz): δ = -0.11 (s, 9H), 0.80 (t, 2H), 3.45 (t, 2H), 4.03 (s, 2H), 5.25 (s, 2H), 5.59 (s, 2H), 6.28-6.35 (m, 2H), 6.57 (d, 1H), 6.85 (d, 1H), 6.93 (t, 1H), 7.57 (d, 1H), 8.14 (d, 1H).

### Beispiel 18A

### 3-Fluor-4-(1H-pyrrolo[2,3-b]pyridin-4-ylmethyl)anilin

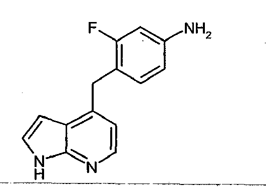

477 mg (1.00 mmol) 3-Fluor-4-[(1-{[2-(trimethylsilyl)ethoxy]methyl}-1H-pyrrolo[2,3-b]pyridin-4-yl)methyl]anilin werden in 4 ml Dichlormethan gelöst. Man setzt 4.0 ml Trifluoressigsäure hinzu und rührt 2 Stunden bei RT. Die Lösung wird zur Trockene eingeengt. Man nimmt den Rückstand in 15 ml THF auf und setzt 15 ml einer 1M wässrigen Lithiumhydroxid-Lösung hinzu. Man rührt die Lösung 20 Stunden bei RT, engt dann ein und versetzt mit Wasser. Es wird mit Essigsäureethylester extrahiert. Man trocknet über Natriumsulfat und entfernt das Lösungsmittel im Vakuum. Der Rückstand wird mittels präparativer HPLC aufgereinigt.
Ausbeute: 42 mg (17% d. Th.)
LC-MS (Methode 1): Rₜ = 1.18 min.
MS (ESI pos.): m/z = 242 [M+H]⁺.
¹H-NMR (DMSO-d₆, 400 MHz): δ = 4.01 (s, 2H), 5.24 (s, 2H), 6.28-6.33 (m, 2H), 6.47 (dd, 1H), 6.76 (d, 1H), 6.94 (t, 1H), 7.38 (dd, 1H), 8.07 (d, 1H), 11.55 (br. s, 1H).

### Beispiel 19A

### 3-Methyl-1H-pyrrolo[2,3-b]pyridin-7-oxid

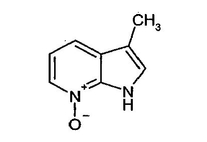

Zu einer Lösung von 10.45 g (45.40 mmol) *meta*-Chlorperoxybenzoesäure in 250 ml Dichlormethan werden portionsweise 3.00 g (22.70 mmol) 3-Methyl-1H-pyrrolo[2,3-b]pyridin (Hands, David; Bishop, Brian; Cameron, Mark; Edwards, John S.; Cottrell, Ian F.; Wright, Stanley H. B.; Synthesis 1996, 877-882.) gegeben und die Mischung für 2 h bei 10°C gerührt. Durch Zugabe von Methanol wird eine klare Lösung erhalten, die direkt einer Säulenchromatographie an Kieselgel unterzogen wird (Eluens: Dichlormethan/Methanol 100:4 bis 3:1). Ein weiterer Reinigungsschritt mittels präparativer HPLC liefert die Zielverbindung.
Ausbeute: 1.4 g (42% d. Th.)
LC-MS (Methode 3): Rₜ = 1.22 min.
MS (ESI pos.): m/z = 149 [M+H]⁺.
¹H-NMR (DMSO-d₆, 400 MHz): δ = 2.25 (s, 3H), 7.05 (dd, 1H), 7.22 (s, 1H), 7.60 (d, 1H), 8.10 (d, 1H), 11.97 (br. s, 1H).

### Beispiel 20A

### 4-Chlor-3-methyl-1H-pyrrolo[2,3-b]pyridin

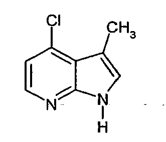

Zu einer Lösung von 1.30 g (8.77 mmol) 3-Methyl-1H-pyrrolo[2,3-b]pyridin-7-oxid in 7.5 ml DMF werden bei 50°C tropfenweise 1.77 ml (22.81 mmol) Methansulfonsäurechlorid hinzugegeben, so dass die Temperatur auf 59°C steigt. Anschließend wird die Mischung für 4 h bei 70°C gerührt. Nach Abkühlen auf Raumtemperatur werden langsam 30 ml Wasser hinzugegeben, durch Zugabe von 20%iger Natriumhydroxid-Lösung bei 5°C wird ein pH-Wert von 10 eingestellt, und die Mischung wird für eine weitere Stunde gerührt. Der ausgefallene Feststoff wird abfiltriert, mit Wasser gewaschen und anschließend im Vakuum getrocknet.
Ausbeute: 395 mg (27% d. Th.)
LC-MS (Methode 2): Rₜ = 1.99 min.
MS (ESI pos.): m/z = 167 (M+H)⁺.
¹H-NMR (DMSO-d₆, 300 MHz): δ = 2.43 (s, 3H), 7.08 (d, 1H), 7.32 (s, 1H), 8.08 (d, 1H), 11.65 (s, 1H).

### Beispiel 21A

### N-(2-Fluor-4-nitrophenyl)-3-methyl-1H-pyrrolo[2,3-b]pyridin-4-amin

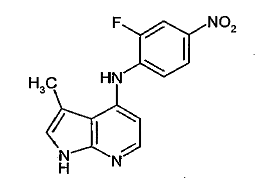

Eine Lösung von 8 mg (50 µmol) 4-Chlor-3-methyl-1H-pyrrolo[2,3-b]pyridin, 9 mg (60 µmol) 2-Fluor-4-nitroanilin, 4 mg (5 µmol) Tris(dibenzylidenaceton)dipalladium und 5 mg (10 µmol) Dicyclohexyl(2',4',6'-triisopropylbiphenyl-2-yl)phosphin und 10 mg (70 µmol) Kaliumcarbonat in 1.00 ml entgastem *tert*-Butanol wird für 3 h im geschlossenen Druckgefäß bei 100°C gerührt. Nach Abkühlen auf RT wird die Mischung über Celite filtriert, mit Methanol nachgewaschen und die Filtrate im Vakuum eingeengt. Der Rückstand wird durch Säulenchromatographie an Kieselgel gereinigt (Eluens: Cyclohexan/Essigsäureethylester 1:1).
Ausbeute: 12 mg (87% d. Th.)
LC-MS (Methode 3): Rₜ = 1.63 min.
MS (ESI pos.): m/z = 287 (M+H)⁺.
¹H-NMR (DMSO-d₆, 300 MHz): δ = 2.13 (s, 3H), 6.82-6.96 (m, 2H), 7.18 (s, 1H), 7.94 (d, 1H), 8.08-8.18 (m, 2H).

### Beispiel 22A

### 2-Fluor-N¹-(3-methyl-1H-pyrrolo[2,3-b]pyridin-4-yl)benzol-1,4-diamin

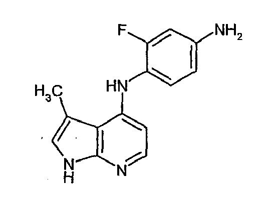

23 mg (80 µmol) *N*-(2-Fluor-4-nitrophenyl)-3-methyl-1H-pyrrolo[2,3-b]pyridin-4-amin werden in 12 ml Ethanol gelöst. Man setzt 19 mg (19 µmol) 10%iges Palladium auf Aktivkohle hinzu und hydriert über Nacht in einer Wasserstoffatmosphäre bei normalem Druck. Man filtriert über Celite und entfernt das Lösungsmittel im Vakuum.
Ausbeute: 17 mg (82% d. Th.)
LC-MS (Methode 3): Rₜ = 1.22 min.
MS (ESI pos.): m/z = 257 (M+H)⁺.
¹H-NMR (DMSO-d₆, 300 MHz): δ = 2.45 (s, 3H), 5.36 (s, 2H), 5.82 (d, 1H), 6.37-6.50 (m, 2H), 6.86 (s, 1H), 6.93-7.09 (m, 2H), 7.68 (d, 1H), 10.87 (s, 1H).

### Beispiel 23A

### 3-Brom-4-nitro-1H-pyrrolo[2,3-b]pyridin

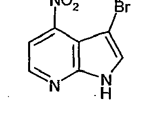

Zu einer Lösung von 1.00 g (6.13 mmol) 4-Nitro-1H-pyrrolo[2,3-b]pyridin (Antonini, Ippolito; Claudi, Francesco; Cristalli, Gloria; Franchetti, Palmarisa; Grifantini, Mario; Martelli, Sante; J. Med. Chem. 1982, 25, 1258-1261.) in 50 ml Dichlormethan werden bei 0°C 316 µl (6.13 mmol) Brom zugetropft. Nach Rühren für 1 h bei 0°C und 1 h bei RT wird die Lösung auf ein Gemisch von Eis und gesättigter Natriumhydrogencarbonat-Lösung geschüttet. Nun wird zweimal mit Dichlormethan extrahiert. Die vereinigten organischen Phasen werden mit Natriumsulfat getrocknet, und das Lösungsmittel wird im Vakuum entfernt. Der Rückstand wird in einem Gemisch von Cyclohexan/Essigsäureethylester aufgenommen und der gebildete Niederschlag abfiltriert.
Ausbeute: 534 mg (31 % d. Th.)
LC-MS (Methode 7): Rₜ = 2.40 min.
MS (ESI neg.): m/z = 240 (M-H)⁻.
¹H-NMR (DMSO-d₆, 300 MHz): δ = 7.73 (s, 1H), 8.12 (d, 1H), 8.54 (d, 1H), 12.99 (d, 1H).

### Beispiel 24A

### 3-Brom-4-nitro-1-{[2-(trimethylsilyl)ethoxy]methyl}-1H-pyrrolo[2,3-b]pyridin

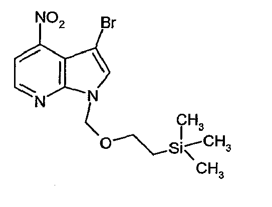

Zu einer Lösung von 100 mg (340 µmol) 4-Nitro-1-{[2-(trimethylsilyl)ethoxy]methyl}-1H-pyrrolo[2,3-b]pyridin in 10 ml Dichlormethan werden bei 0°C 18 µl (6.13 mmol) Brom zugetropft. Nach Rühren für 1 h bei 0°C und 1 h bei RT wird die Reaktionslösung über eine Kieselgel-Extrelutkartusche filtriert, mit etwas Dichlormethan/Methanol nachgewaschen und das Filtrat im Vakuum eingeengt. Der Rückstand wird mittels präparativer RP-HPLC gereinigt.
Ausbeute: 79 mg (62% d. Th.)
LC-MS (Methode 9): Rₜ = 2.87 min.
MS (ESI pos.): m/z = 372 (M+H)⁺.
¹H-NMR (DMSO-d₆, 300 MHz): δ = -0.03 (s, 9H), 0.92 (t, 2H), 3.63 (t, 2H), 5.81 (s, 2H), 7.92 (d, 1H), 8.42 (s, 1H), 8.71 (d, 1H).

### Beispiel 25A

### 3-Methyl-4-nitro-1-{[2-(trimethylsilyl)ethoxy]methyl}-1H-pyrrolo[2,3-b]pyridin

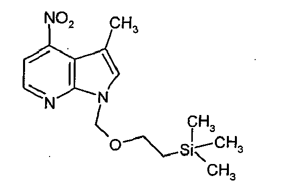

Eine Lösung von 80 mg (210 µmol) 3-Brom-4-nitro-1-{[2-(trimethylsilyl)ethoxy]methyl}-1H-pyrrolo[2,3-b]pyridin, 27 mg (210 µmol) Trimethylboroxin, 25 mg (20 µmol) Tetrakis(triphenylphosphin)palladium und 103 mg (640 µmol) Kaliumcarbonat in 2.00 ml entgastem DMF wird für 6 h im geschlossenen Druckgefäß bei 110°C und über Nacht bei RT gerührt. Die Suspension wird anschließend über eine Kieselgel-Extrelutkartusche filtriert, mit einer Mischung von Dichlormethan/Methanol 1:1 nachgewaschen und das Filtrat im Vakuum eingeengt. Der Rückstand wird durch präparative RP-HPLC gereinigt.
Ausbeute: 28 mg (42% d. Th.)
LC-MS (Methode 7): Rₜ = 3.80 min.
MS (ESI pos.): m/z = 309 (M+H)⁺.
¹H-NMR (DMSO-d₆, 300 MHz): δ = -0.10 (s, 9H), 0.81 (t, 2H), 2.32 (s, 3H), 3.50 (t, 2H), 5.65 (s, 2H), 7.76 (d, 1H), 7.83 (s, 1H), 8.49 (d, 1H).

### Beispiel 26A

### 3-Methyl-1-{[2-(trimethylsilyl)ethoxy]methyl}-1H-pyrrolo[2,3-b]pyridin-4-amin

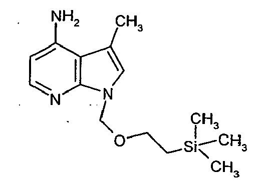

Eine Mischung von 27 mg (90 µmol) 3-Methyl-4-nitro-1-{[2-(trimethylsilyl)ethoxy]methyl}-1H-pyrrolo[2,3-b]pyridin und 10 mg (40 µmol) Platin(IV)oxid in 8 ml Ethanol/THF (1:1) wird über Nacht in einer Wasserstoffatmosphäre unter Normaldruck gerührt. Anschließend wird die Suspension über Celite filtriert und mit Ethanol gewaschen. Einengen des Filtrats liefert das Zielprodukt.
Ausbeute: 21 mg (83% d. Th.)
LC-MS (Methode 9): Rₜ = 1.70 min.
MS (ESI pos.): m/z = 278 (M+H)⁺.

### Beispiel 27A

### N-(2-Fluor-4-nitrophenyl)-3-methyl-1-{[2-(trimethylsilyl)ethoxy]methyl}-1H-pyrrolo[2,3-b]pyridin-4-amin

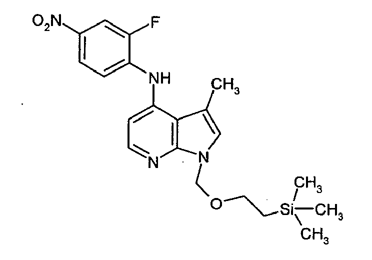

Eine Lösung von 29 mg (100 µmol) 3-Methyl-1-{[2-(trimethylsilyl)ethoxy]methyl}-1H-pyrrolo-[2,3-b]pyridin-4-amin, 33 mg (130 µmol) 2-Fluor-1-iod-4-nitrobenzol, 5 mg (10 µmol) Tris-(dibenzylidenaceton)dipalladium, 6 mg (10 µmol) 1,1'-Bis(diphenylphosphino)ferrocen und 14 mg (150 µmol) Natrium-*tert*-butylat in 3 ml entgastem Toluol wird über Nacht bei 100°C gerührt.
Nach Abkühlen auf RT wird die Reaktionsmischung direkt auf eine Kieselgelsäule gegeben und aufgetrennt (Eluens: Cyclohexan/Essigsäureethylester 10:1 bis 8:2).
Ausbeute: 20 mg (46% d. Th.)
LC-MS (Methode 9): Rₜ = 2.78 min.
MS (ESI pos.): m/z = 417 (M+H)⁺.

### Beispiel 28A

### 2-Fluor-N¹-(3-methyl-1-{[2-(trimethylsilyl)ethoxylmethyl}-1H-pyrrolo[2,3-b]pyridin-4-yl)benzol-1,4-diamin

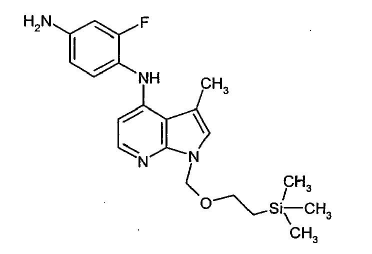

20 mg (50 µmol) *N*-(2-Fluor-4-nitrophenyl)-3-methyl-1-{[2-(trimethylsilyl)etboxy]methyl}-1H-pyrrolo[2,3-b]pyridin-4-amin werden in 8 ml einer Mischung von Ethanol/THF (1:1) gelöst. Man setzt 11 mg (50 µmol) Platin(IV)oxid hinzu und rührt über Nacht unter einer Wasserstoffatmosphäre bei normalem Druck. Anschließend wird die Suspension über Celite filtriert, mit etwas Ethanol gewaschen und das Lösungsmittel im Vakuum entfernt.
Ausbeute: 17 mg (95% d. Th.)
LC-MS (Methode 8): Rₜ = 2.39 min.
MS (ESI pos.): m/z = 387 (M+H)⁺.

### Beispiel 29A

### 4-(Dibenzylamino)-2,6-difluorbenzoesäure

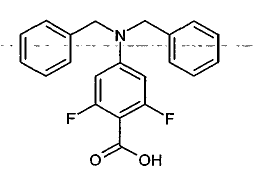

10.0 g (32.3 mmol) *N,N*-Dibenzyl-3,5-difluoranilin (Florvall, Lennart; Fagervall, Ingrid; Larsson, Lars-Gunnar; Ross, Svante B.; Eur. J. Med. Chem. Chim. Ther. 1999, 34, 137-152.) werden in 90 ml THF bei -78°C vorgelegt. Man tropft 12.9 ml (32.3 mmol) einer 2.5 M Lösung von n-Butyllithium in Hexan zu und lässt 45 min bei dieser Temperatur rühren. Dann wird Trockeneis im Überschuss zugesetzt. Man lässt eine weitere Stunde bei dieser Temperatur rühren und danach langsam auf RT kommen. Der Reaktionslösung wird Wasser zugesetzt und zweimal mit *tert-*Butylmethylether extrahiert. Die wässrige Phase wird mit 10 ml 4N Salzsäure angesäuert, wobei ein Niederschlag ausfällt. Man extrahiert zweimal mit Dichlormethan, trocknet die vereinigten Phasen über Natriumsulfat und entfernt das Lösungsmittel im Vakuum.
Ausbeute: 8.1 g (71% d. Th.)
LC-MS (Methode 2): Rₜ = 2.59 min.
MS (ESI pos.): m/z = 354 (M+H)⁺.
¹H-NMR (DMSO-d₆, 400 MHz): δ = 4.78 (s, 4H), 6.33-6.42 (m, 2H), 7.22-7.39 (m, 10H), 12.80 (br. s, 1H).

### Beispiel 30A

### Ethyl-[4-(dibenzylamino)-2,6-difluorphenyl]carbamat

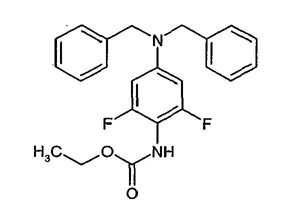

2.60 g (7.36 mmol) 4-(Dibenzylamino)-2,6-difluorbenzoesäure werden in 20 ml Dioxan gelöst. Man versetzt tropfenweise mit 2.42 g (8.83 mmol) Phosphorsäurediphenylesterazid und anschließend mit 1.08 g (10.7 mmol) Triethylamin und lässt eine Stunde bei RT rühren. Man setzt dann 6.0 ml Ethanol hinzu und erhitzt 2 Stunden auf Rückfluss. Die Reaktionslösung wird dann eingeengt und der Rückstand in Dichlormethan gelöst. Man wäscht mit halbkonzentrierter Natriumhydrogencarbonat-Lösung und Wasser, trocknet über Natriumsulfat und entfernt das Lösungsmittel im Vakuum. Der Rückstand wird durch Chromatographie an Kieselgel gereinigt (Eluens: Dichlormethan). Das erhaltene Öl wird in wenig Dichlormethan gelöst. Nach Zugabe von etwas Pentan und Anreiben erhält man Kristalle, die abgesaugt werden.
Ausbeute: 2.2 g (75% d. Th.)
LC-MS (Methode 2): Rₜ = 2.86 min.
MS (ESI pos.): m/z = 397 (M+H)⁺.
¹H-NMR (DMSO-d₆, 400 MHz): δ = 1.18 (t, 3H), 4.03 (q, 2H), 4.73 (s, 4H), 6.30-6.37 (m, 2H), 7.22-7.28 (m, 6H), 7.32-7.37 (m, 4H), 8.55 (br. s, 1H).

### Beispiel 31A

### N⁴,N⁴-Dibenzyl-2,6-difluorbenzol-1,4-diamin

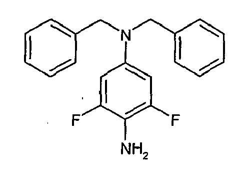

2.10 g (5.30 mmol) Ethyl-[4-(dibenzylamino)-2,6-difluorphenyl]carbamat werden in 40 ml Ethanol gelöst. Man setzt 3.0 g (53 mmol) gepulvertes Kaliumhydroxid hinzu und erhitzt 20 Stunden auf Rückfluss. Das Lösungsmittel wird dann entfernt und der Rückstand mit Wasser und Dichlormethan versetzt. Die organische Phase wird abgetrennt, dreimal mit Wasser gewaschen, über Natriumsulfat getrocknet und eingeengt. Der Rückstand wird in Essigsäureethylester aufgenommen und über Aktivkohle geklärt. Mit Pentan erhält man Kristalle, die abgesaugt werden.
Ausbeute: 1.2 g (70% d. Th.)
LC-MS (Methode 3): Rₜ = 2.91 min.
MS (ESI pos.): m/z = 325 (M+H)⁺.
¹H-NMR (DMSO-d₆, 300 MHz): δ = 4.56 (s, 4H), 6.21-6.36 (m, 2H), 7.19-7.36 (m, 10H).

### Beispiel 32A

### N⁴,N⁴-Dibenzyl-2,6-difluor-N¹-1H-pyrrolo[2,3-b]pyridin-4-ylbenzol-1,4-diamin

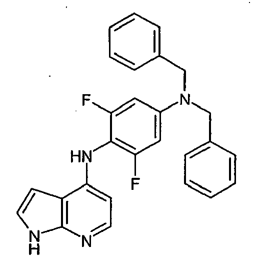

Eine Lösung von 100 mg (660 µmol) 4-Chlor-1H-pyrrolo[2,3-b]pyridin, 255 mg (790 µmol) 2-*N*⁴,*N*⁴-Dibenzyl-2,6-difluorbenzol-1,4-diamin, 30 mg (30 µmol) Tris(dibenzylidenaceton)dipalladium und 31 mg (70 µmol) Dicyclohexyl(2',4',6'-triisopropylbiphenyl-2-yl)phosphin und 199 mg (1.44 mmol) Kaliumcarbonat in 1.50 ml entgastem *tert*-Butanol wird für 3 h im geschlossenen Druckgefäß bei 100°C gerührt. Nach Abkühlen aufRT wird die Mischung über Celite filtriert, mit Essigsäureethylester/Methanol (1:1) nachgewaschen und die Filtrate werden im Vakuum eingeengt. Säulenchromatographie des Rückstands an Kieselgel (Eluens: Cyclohexan/Essigsäureethylester 1:1, Essigsäureethylester/Methanol 100:1 bis 95:5) liefert das Zielprodukt. Die Verbindung wird ohne weitere Reinigung umgesetzt.
Ausbeute: 122 mg (42% d. Th.)
LC-MS (Methode 3): Rₜ = 2.20 min.
MS (ESI pos.): m/z = 441 (M+H)⁺.
¹H-NMR (DMSO-d₆, 400 MHz): δ = 4.76 (s, 4H), 5.82 (d, 1 H), 6.48-6.58 (m, 3H), 7.12 (s, 1H), 7.21-7.43 (m, 10H), 7.76 (d, 1H), 7.91 (s, 1H), 11.25 (s, 1H).

### Beispiel 33A

### 2,6-Difluor-N¹-1H-pyrrolo[2,3-b]pyridin-4-ylbenzol-1,4-diamin

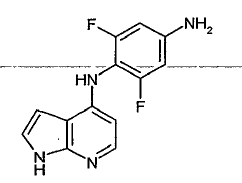

Eine Lösung von 140 mg (235 µmol) *N*⁴,*N*⁴-Dibenzyl-2,6-difluor-*N*¹-1H-pyrrolo[2,3-b]pyridin-4 ylbenzol-1,4-diamin in 40 ml Ethanol wird mit 706 µl (706 µmol) 1M wässriger Salzsäure-Lösung und 25 mg (24 µmol) 10%igem Palladium auf Aktivkohle versetzt und über Nacht in einer Wasserstoffatmosphäre bei normalem Druck hydriert. Man filtriert über Celite, entfernt das Lösungsmittel im Vakuum und reinigt den Rückstand über präparative RP-HPLC.
Ausbeute: 52 mg (86% d. Th.)
LC-MS (Methode 3): Rₜ = 0.74 min.
MS (ESI pos.): m/z = 261 (M+H)⁺.
¹H-NMR (DMSO-d₆, 300 MHz): δ = 6.02 (s, 2H), 6.16-6.35 (m, 1H), 6.39 (s, 1H), 6.40 (s, 1H), 7.29-7.43 (m, 1H), 7.98 (d, 1H), 9.66 (s, 1H), 12.38 (s, 1H).

### Beispiel 34A

### 1-Methyl-4-nitro-1H-pyrrolo[2,3-b]pyridin

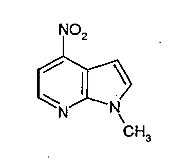

Zu einer Lösung von 500 mg (3.06 mmol) 4-Nitro-1H-pyrrolo[2,3-b]pyridin (Antonini, Ippolito; Claudi, Francesco; Cristalli, Gloria; Franchetti, Palmarisa; Grifantini, Mario; Martelli, Sante; J. Med. Chem. 1982, 25, 1258-1261.) in 10 ml absolutem DMA werden 170 mg (4.29 mmol) Natriumhydrid gegeben und die Suspension für 30 min gerührt. Dann wird eine Lösung von 230 µl (3.68 mmol) Methyliodid in 2 ml absolutem DMA langsam zugetropft und die Mischung erneut für 60 min gerührt. Nach Einengen im Vakuum wird der Rückstand mittels Säulenchromatographie an Kieselgel gereinigt (Eluens: Cyclohexan/Essigsäureethylester 10:1).
Ausbeute: 318 mg (59% d. Th.)
LC-MS (Methode 8): Rₜ = 3.10 min.
MS (ESI pos.): m/z = 178 (M+H)⁺.
¹H-NMR (DMSO-d₆, 300 MHz): δ = 3.93 (s, 3H), 6.69 (d, 1H), 7.94 (d, 1H), 7.99 (d, 1H), 8.56 (d, 1H).

### Beispiel 35A

### 1-Methyl-1H-pyrrolo[2,3-b]pyridin-4-amin

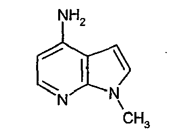

300 mg (1.69 mmol) 1-Methyl-4-nitro-1H-pyrrolo[2,3-b]pyridin werden in einer Mischung von 20 ml Ethanol/THF (1:1) gelöst. Man setzt 38 mg (0.17 mmol) Platin(IV)oxid hinzu und hydriert über Nacht in einer Wasserstoffatmosphäre bei normalem Druck. Man filtriert über Celite und entfernt das Lösungsmittel im Vakuum.
Ausbeute: 265 mg (quant.)
LC-MS (Methode 10): Rₜ = 1.70 min.
MS (ESI pos.): m/z = 148 (M+H)⁺.
¹H-NMR (DMSO-d₆, 400 MHz): δ = 3.68 (s, 3H), 6.02-6.21 (m, 3H), 6.47 (d, 1H), 7.07 (d, 1H),
7.74 (d, 1H).

### Beispiel 36A

### N-(2-Fluor-4-nitrophenyl)-1-methyl-1H-pyrrolo[2,3-b]pyridin-4-amin

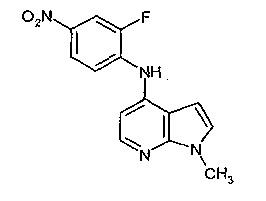

Eine Lösung von 260 mg (1.77 mmol) 1-Methyl-1H-pyrrolo[2,3-b]pyridin-4-amin, 566 mg (2.12 mmol) 2-Fluor-1-iod-4-nitrobenzol, 81 mg (0.09 mmol) Tris(dibenzylidenaceton)dipalladium, 98 mg (0.18 mmol) 1,1'-Bis(diphenylphosphino)ferrocen und 238 mg (2.47 mmol) Natrium-tert-butylat in 8 ml entgastem Toluol wird über Nacht bei 100°C gerührt. Nach Abkühlen auf RT wird die Reaktionsmischung direkt auf eine Kieselgelsäule gegeben und aufgetrennt (Eluens: Cyclohexan/Essigsäureethylester 10:1 bis 1:1).
Ausbeute: 210 mg (42% d. Th.)
LC-MS (Methode 8): Rₜ = 1.89 min.
MS (ESI pos.): m/z = 287 (M+H)⁺.
¹H-NMR (DMSO-d₆, 300 MHz): δ = 3.80 (s, 3H), 6.48 (d, 1H), 6.83 (d, 1H), 7.35 (dd, 1H), 7.40 (d, 1H), 8.03 (d, 1H), 8.13 (d, 1H), 8.18 (d, 1H), 9.32 (s, 1H).

### Beispiel 37A

### 2-Fluor-N¹-(1-methyl-1H-pyrrolo[2,3-b]pyridin-4-yl)benzol-1,4-diamin

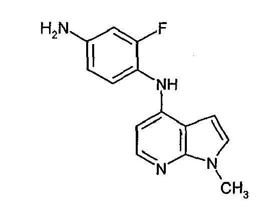

195 mg (0.68 mmol) *N*-(2-Fluor-4-nitrophenyl)-1-methyl-1H-pyrrolo[2,3-b]pyridin-4-amin werden in 10 ml einer Mischung von Ethanol/THF (1:1) gelöst. Man setzt 15 mg (0.07 mmol) Platin(IV)oxid hinzu und rührt über Nacht unter einer Wasserstoffatmosphäre bei normalem Druck. Anschließend wird die Suspension über Celite filtriert, mit etwas Ethanol gewaschen und das Lösungsmittel im Vakuum entfernt.
Ausbeute: 204 mg (quant.)
LC-MS (Methode 10): Rₜ = 2.10 min.
MS (ESI pos.): m/z = 257 (M+H)⁺.
¹H-NMR (DMSO-d₆, 300 MHz): δ = 3.73 (s, 3H), 5.41 (s, 2H), 5.98 (d, 1H), 6.34-6.58 (m, 3H), 6.99 (dd, 1H), 7.17 (d, 1H), 7.81 (d, 1H), 8.13 (s, 1H).

### Beispiel 38A

### (4-Nitrophenyl)-1H-pyrrolo[2,3-b]pyridin-4-amin

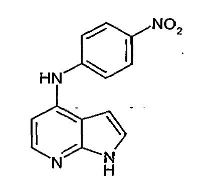

Eine Lösung von 300 mg (1.97 mmol) 4-Chlor-1H-pyrrolo[2,3-b]pyridin, 326 mg (2.36 mmol) 4-Nitroanilin, 90 mg (0.10 mmol) Tris(dibenzylidenaceton)dipalladium, 51 mg (0.1 mmol) Dicyclohexyl(2',4',6'-triisopropylbiphenyl-2-yl)phosphin und 598 mg (4.33 mmol) Kaliumcarbonat in 3.00 ml entgastem *tert*-Butanol wird für 3 h im geschlossenen Druckgefäß bei 100°C gerührt. Nach Abkühlen auf RT wird die Reaktionsmischung über Kieselgur filtriert, mit Essigsäureethylester/Methanol 100:5 nachgewaschen und vom Filtrat das Lösungsmittel entfernt. Der Rückstand wird in Dichlormethan aufgenommen. Der auskristallisierte Feststoff wird abfiltriert, mit Dichlormethan gewaschen und getrocknet. Die Mutterlauge und das Filtrat werden vereinigt und im Vakuum eingeengt. Reinigung des Rückstands durch präparative HPLC liefert das Zielprodukt.
Ausbeute: 340 mg (78% d. Th.)
LC-MS (Methode 3): Rₜ = 1.38 min.
MS (ESI pos.): m/z = 255 (M+H)⁺.
¹H-NMR (DMSO-d₆, 300 MHz): δ = 6.48-6.58 (m, 1H), 6.97 (d, 1H), 7.28-7.42 (m, 3H), 8.08 (d, 1H), 8.19 (d, 2H), 9.55 (s, 1H), 11.62 (s, 1H).

### Beispiel 39A

### N-1H-Pyrrolo[2,3-b]pyridin-4-ylbenzol-1,4-diamin

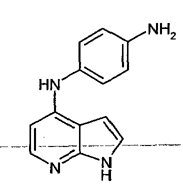

Eine Lösung von 170 mg (0.67 mmol) N-(4-Nitrophenyl)-1H-pyrrolo[2,3-b]pyridin-4-amin in 6 ml Ethanol wird mit 36 mg 10%igem Palladium auf Aktivkohle versetzt, und die Suspension wird über Nacht in einer Wasserstoffatmosphäre bei Normaldruck gerührt. Die Suspension wird über Celite filtriert und mit Ethanol wird nachgewaschen. Einengen im Vakuum liefert das Zielprodukt.
Ausbeute: 151 mg (100% d. Th.)
LC-MS (Methode 1): Rₜ = 2.10 min.
MS (ESI pos.): m/z = 225 (M+H)⁺.
¹H-NMR (DMSO-d₆, 300 MHz): δ = 4.98 (s, 2H), 6.24 (d, 1H), 6.51 (d, 1H), 6.59 (d, 2H), 6.95 (d, 2H), 7.08 (d, 1H), 7.50 (d, 1H), 8.12 (s, 1H), 11.15 (s, 1H).

### Beispiel 40A

### N-(2-Fluor-4-nitrophenyl)-1-{[2-(trimethylsilyl)ethoxy]methyl}-1H-pyrrolo[2,3-b]pyridin-4-amin

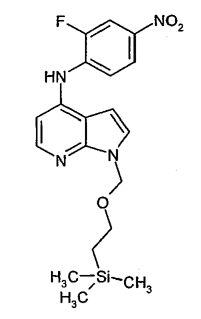

Eine Lösung von 300 mg (1.06 mmol) 4-Chlor-1-{[2-(trimethylsilyl)ethoxy]methyl}-1H-pyrrolo[2,3-b]pyridin, 199 mg (1.27 mmol) 2-Chlor-4-nitroanilin, 49 mg (0.05 mmol) Tris(dibenzylidenaceton)dipalladium und 51 mg (0.11 mmol) Dicyclohexyl(2',4',6'-triisopropylbiphenyl-2-yl)phosphin und 322 mg (2.33 mmol) Kaliumcarbonat in 3.00 ml entgastem *tert-*Butanol wird für 3 h im geschlossenen Druckgefäß bei 100°C gerührt. Nach Abkühlen auf RT wird die Reaktionsmischung über Kieselgur filtriert, mit Essigsäureethylester wird nachgewaschen, und vom Filtrat wird das Lösungsmittel entfernt. Der Rückstand wird durch Säulenchromatographie an Kieselgel (Laufmittel: Gradient Cyclohexan/ Essigsäureethylester 10:1 bis 8:2) gereinigt.
Ausbeute: 403 mg (78% d. Th.)
LC-MS (Methode 1): Rₜ = 2.71 min.
MS (ESI pos.): m/z = 403 (M+H)⁺.
¹H-NMR (DMSO-d₆, 400 MHz): δ = 0.00 (s, 9H), 0.91 (t, 2H), 3.61 (t, 2H), 5.69 (s, 2H), 6.59 (d, 1H), 6.94 (d, 1H), 7.47 (t, 1H), 7.60 (d, 1H), 8.14 (dd, 1H), 8.23 (d, 1H), 8.28 (dd, 1H), 9.47 (d, 1H).

### Beispiel 41A

### N-(2-Fluor-4-nitrophenyl)-N-methyl-1-{[2-(trimethylsilyl)ethoxy]methyl}-1H-pyrrolo[2,3-b]pyridin-4-amin

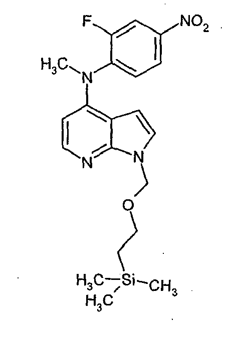

Zu einer Suspension von 30 mg (0.75 mmol) Natriumhydrid in 0.75 ml DMA wird eine Lösung von 100 mg (0.25 mmol) *N*-(2-Fluor-4-nitrophenyl)-1-{[2-(trimethylsilyl)ethoxy]methyl}-1H-pyrrolo[2,3-b]pyridin-4-amin in 0.75 ml DMA zugetropft und die Mischung wird für 30 min bei RT gerührt. Anschließend werden langsam 77 µl (1.24 mmol) Methyliodid zugetropft und die Mischung wird für weitere 30 min gerührt. Dann wird Wasser zugegeben und zweimal mit Dichlormethan extrahiert. Die vereinigten organischen Phasen werden über Magnesiumsulfat getrocknet, das Lösungsmittel im Vakuum entfernt und der Rückstand mittels präparativer HPLC gereinigt.
Ausbeute: 71 mg (69% d. Th.)
LC-MS (Methode 2): Rₜ = 2.68 min.
MS (ESI pos.): m/z = 417 (M+H)⁺.
¹H-NMR (DMSO-d₆, 300 MHz): δ = -0.11 (s, 9H), 0.78 (t, 2H), 3.48 (t, 2H), 3.52 (s, 3H), 5.49 (d, 1M, 5.55 (s, 2H), 6.82 (d, 1H), 7.34 (d, 1H), 7.53 (dd, 1H), 8.07-8.18 (m, 3H).

### Beispiel 42A

### N-(2-Fluor-4-nitrophenyl)-N-methyl-1H-pyrrolo[2,3-b]pyridin-4-amin

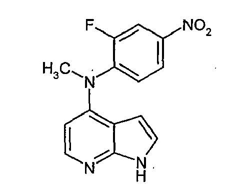

Eine Lösung von 71 mg (0.17 mmol) *N*-(2-Fluor-4-nitrophenyl)-*N*-methyl-1-{[2-(trimethylsilyl)-ethoxy]methyl}-1H-pyrrolo[2,3-b]pyridin-4-amin in 2 ml Dichlormethan und 1.0 ml (13.0 mmol) Trifluoressigsäure wird für 3 h bei RT gerührt. Die Reaktionsmischung wird im Vakuum eingeengt, der Rückstand in 2 ml THF aufgenommen, mit 1.02 ml (1.02 mmol) 1M wässriger Lithiumhydroxid-Lösung versetzt, und die Mischung wird über Nacht bei RT gerührt. Zur Vervollständigung der Reaktion werden weitere 0.5 ml (0.5 mmol) Lithiumhydroxid-Lösung zugegeben und für eine weitere Nacht gerührt. Die Mischung wird mit gesättigter Natriumhydrogencarbonat-Lösung versetzt und zweimal mit Essigsäureethylester extrahiert. Die vereinigten organischen Phasen werden über Magnesiumsulfat getrocknet, das Lösungsmittel im Vakuum entfernt und der Rückstand mittels präparativer HPLC gereinigt.
Ausbeute: 26 mg (54% d. Th.)
LC-MS (Methode 2): Rₜ = 1.46 min.
MS (ESI pos.): m/z = 287 (M+H)⁺.
¹H-NMR (DMSO-d₆, 400 MHz): δ = 3.53 (s, 3H), 5.42-5.50 (m, 1H), 6.75 (d, 1H), 7.13-7.23 (m, 1H), 7.43-7.53 (m, 1H), 8.11 (d, 1H), 8.07-8.20 (m, 2H), 11.59 (s, 1H).

### Beispiel 43A

### 2-Fluor-N¹-methyl-N¹-1H-pyrrolo[2,3-b]pyridin-4-ylbenzol-1,4-diamin

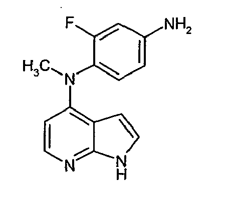

Eine Lösung von 28 mg (0.08 mmol) *N*-(2-Fluor-4-nitrophenyl)-*N*-methyl-1H-pyrrolo[2,3-b]pyridin-4-amin in 10 ml Ethanol wird mit 9 mg 10%igem Palladium auf Aktivkohle versetzt und die Suspension wird über Nacht in einer Wasserstoffatmosphäre bei Normaldruck gerührt. Die Suspension wird über Kieselgur filtriert und mit Ethanol wird nachgewaschen. Einengen im Vakuum liefert das Zielprodukt.
Ausbeute: 22 mg (98% d. Th.)
LC-MS (Methode 2): Rₜ = 0.97 min.
MS (ESI pos.): m/z = 257 (M+H)⁺.
¹H-NMR (DMSO-d₆, 300 MHz): δ = 3.26 (s, 3H), 5.03-5.12 (m, 1H), 5.52 (s, 2H), 6.28 (d, 1H), 6.34-6.46 (m, 2H), 6.83-6.92 (m, 1H), 6.93-7.04 (m, 1H), 7.90 (d, 1H), 11.16 (s, 1H).

### Beispiel 44A

### N-(2-Chlor-4-nitrophenyl)-1H-pyrrolo[2,3-b]pyridin-4-amin

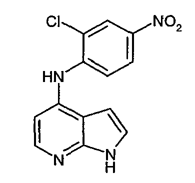

Variante A: Eine Lösung von 200 mg (1.31 mmol) 4-Chlor-1H-pyrrolo[2,3-b]pyridin, 271 mg (1.57 mmol) 2-Chlor-4-nitroanilin, 60 mg (0.07 mmol) Tris(dibenzylidenaceton)dipalladium, 62 mg (0.13 mmol) Dicyclohexyl(2',4',6'-triisopropylbiphenyl-2-yl)phosphin und 399 mg (2.88 mmol) Kaliumcarbonat in 2.00 ml entgastem *tert*-Butanol wird für 3 h im geschlossenen Druckgefäß bei 100°C gerührt. Anschließend wird aufRT abgekühlt und es werden weitere 136 mg (0.79 mmol) 2-Chlor-4-nitroanilin, 31 mg (0.07 mmol) Dicyclohexyl(2',4',6'-triisopropyl-biphenyl-2-yl)phosphin und 30 mg (0.04 mmol) Tris(dibenzylidenaceton)dipalladium zugegeben und für weitere 3 h bei 100°C gerührt.

Variante B: Eine Mischung von 200 mg (1.31 mmol) 4-Chlor-1H-pyrrolo[2,3-b]pyridin, 678 mg (3.93 mmol) 2-Chlor-4-nitroanilin, 60 mg (0.07 mmol) Tris(dibenzylidenaceton)dipalladium, 62 mg (0.13 mmol) Dicyclohexyl(2',4',6'-triisopropylbiphenyl-2-yl)phosphin und 399 mg (2.88 mmol) Kaliumcarbonat in 2.50 ml entgastem *tert*-Butanol wird für 3 h im geschlossenen Druckgefäß bei 100°C gerührt.

Die Reaktionsmischungen aus beiden Synthesevarianten werden nach Abkühlen auf RT über Celite filtriert, mit Essigsäureethylester nachgewaschen und die Filtrate im Vakuum eingeengt. Der Rückstand wird einer Säulenchromatographie an Kieselgel unterzogen (Eluens Cyclohexan/Essigsäureethylester 10 : 1 bis 100% Essigsäureethylester).

Man erhält 145 mg des partiell gereinigten Produkts (Reinheit 67%), wovon 30 mg mittels präparativer HPLC gereinigt werden.
LC-MS (Methode 1): Rₜ = 1.28 min.
MS (ESI pos.): m/z = 289 (M+H)⁺.
¹H-NMR (DMSO-d₆, 300 MHz): δ = 6.14-6.23 (m, 1H), 6.90 (d, 1H), 7.15 (d, 1H), 7.31-7.40 (m, 1H), 8.08 (dd, 1H), 8.12 (d, 1H), 8.34 (d, 1H), 9.01 (s, 1H), 11.68 (s, 1H).

### Beispiel 45A

### 2-Chlor-N¹-1H-pyrrolo[2,3-b]pyridin-4-ylbenzol-1,4-diamin

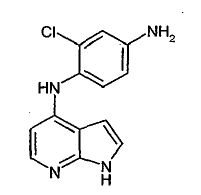

Variante A: Eine Lösung von 55 mg (130 µmol) *N*-(2-Chlor-4-nitrophenyl)-1H-pyrrolo[2,3-b]pyridin-4-amin und 144 mg (640 µmol) Zinn(II)chlorid-Dihydrat in 2.5 ml DMF wird für 4 h bei RT gerührt. Dann werden weitere 144 mg Zinn(II)chlorid-Dihydrat zugegeben und die Mischung für weitere 4 h gerührt. Dann wird die Lösung mit Essigsäureethylester verdünnt, mit gesättigter Natriumhydrogencarbonat-Lösung auf pH 9-10 gestellt und über Kieselgur abfiltriert. Die zwei Phasen des Filtrats werden getrennt, die wässrige mit Essigsäureethylester versetzt und dieses Gemisch erneut über das vorher verwendete Celite filtriert. Nach Wiederholung dieses Vorgangs werden die organischen Phasen vereinigt, über Magnesiumsulfat getrocknet und das Lösungsmittel im Vakuum entfernt. Der Rückstand wird mittels präparativer HPLC gereinigt.
Ausbeute: 5 mg (15% d. Th.)
LC-MS (Methode 1): Rₜ = 0.92 min.
MS (ESI pos.): m/z = 259 (M+H)⁺.

Variante B: Zu einer Lösung von 16 mg (60 µmol) *N*-(2-Chlor-4-nitrophenyl)-1H-pyrrolo[2,3-b]pyridin-4-amin in 3.60 ml Ethanol werden eine Lösung von 10 mg (60 µmol) Eisen(III)chlorid in 2.40 ml Wasser und anschließend 38 mg (170 µmol) Eisenpulver zugegeben. Nach Rühren bei 80°C für 3 h wird die Suspension über Kieselgur filtriert und mit Ethanol nachgewaschen. Das Lösungsmittel wird im Vakuum entfernt, der Rückstand in einem Gemisch von Dichlormethan/Methanol 10:1 gelöst und über eine Säulenfiltration an Kieselgel grob gereinigt. Man erhält 22 mg als Rohprodukt, welches ohne weitere Reinigung direkt weiter umgesetzt wird.

### Beispiel 46A

### N⁴,N⁴-Dibenzyl-2,6-difluor-N¹-{3-methyl-1-[(4-methylphenyl)sulfonyl]-1H-pyrrolo[2,3-b]pyridin-4-yl}benzol-1,4-diamin

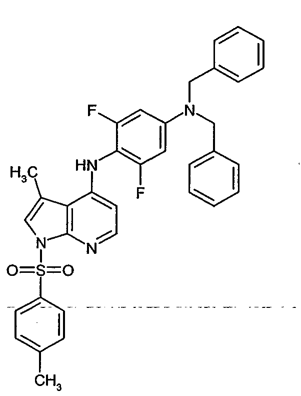

Eine Lösung von 250 mg (0.78 mmol 4-Chlor-3-methyl-1-[(4-methylphenyl)sulfonyl]-1H-pyrrolo[2,3-b]pyridin, 303 mg (0.94 mmol) *N*⁴,*N*⁴-Dibenzyl-2,6-difluorbenzol-1,4-diamin, 36 mg (0.05 mmol) Tris(dibenzylidenaceton)dipalladium, 156 mg (0.33 mmol) Dicyclohexyl(2',4',6'-triisopropylbiphenyl-2-yl)phosphin und 237 mg (1.71 mmol) Kaliumcarbonat in 5.00 ml entgastem *tert*-Butanol wird für 3 h im geschlossenen Druckgefäß bei 100°C gerührt. Nach Abkühlen auf RT wird die Mischung über Celite filtriert, mit Essigsäureethylester nachgewaschen und die Filtrate im Vakuum eingeengt. Der Rückstand wird durch Säulenchromatographie an Kieselgel gereinigt (Eluens: Cyclohexan/Essigsäureethylester 5 : 1).
Ausbeute: 395 mg (78% d. Th.)
LC-MS (Methode 1): Rₜ = 3.08 min.
MS (ESI pos.): m/z = 609 (M+H)⁺.
¹H-NMR (DMSO-d₆, 300 MHz): δ = 2.32 (s, 3H), 2.41 (s, 3H), 4.77 (s, 4H), 5.89 (d, 1H), 6.41-6.53 (m, 2H), 7.17-7.31 (m, 7H), 7.32-7.42 (m, 6H), 7.82 (d, 1H), 7.91 (d, 2H), 8.33 (s, 1H).

### Beispiel 47A

### N⁴,N⁴-Dibenzyl-2,6-difluor-N¹-(3-methyl-1H-pyrrolo[2,3-b]pyridin-4-yl)benzol-1,4-diamin

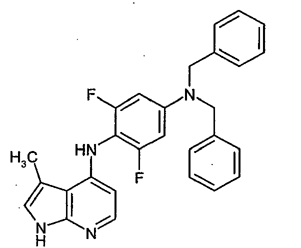

Zu einer Lösung von 390 mg (0.66 mmol) *N*⁴,*N*⁴-Dibenzyl-2,6-difluor-*N*¹-{3-methyl-1-[(4-methylphenyl)sulfonyl]-1H-pyrrolo[2,3-b]pyridin-4-yl}benzol-1,4-diamin in 15 ml Ethanol werden 5 ml einer 20%igen wässrigen Natriumhydroxid-Lösung gegeben und die Mischung über Nacht gerührt. Dann wird die Reaktionslösung auf ein Gemisch aus Wasser und Essigsäureethylester gegossen und die wässrige Phase noch zweimal mit Essigsäureethylester extrahiert. Reinigung mittels präparativer HPLC liefert die Zielverbindung.
Ausbeute: 316 mg (100% d. Th.)
LC-MS (Methode 1): Rₜ = 2.02 min.
MS (ESI pos.): m/z = 455 (M+H)⁺.
¹H-NMR (DMSO-d₆, 300 MHz): δ = 2.42 (s, 3H), 4.77 (s, 4H), 5.67 (d, 1H), 6.41-6.52 (m, 2H), 6.88 (d, 2H), 7.23-7.33 (m, 6H), 7.34-7.42 (m, 4H), 7.69 (d, 1H), 10.90 (s, 1H).

### Beispiel 48A

### 2,6-Difluor-N¹-(3-methyl-1H-pyrrolo[2,3-b]pyridin-4-yl)benzol-1,4-diamin

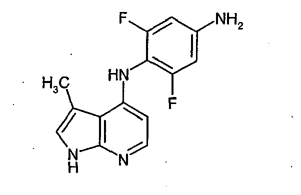

Eine Lösung von 280 mg (0.62 mmol) *N*⁴,*N*⁴-Dibenzyl-2,6-difluor-*N*¹-(3-methyl-1H-pyrrolo[2,3-b]pyridin-4-yl)benzol-1,4-diamin in 20 ml Ethanol und 1.85 ml (1.85 mmol) 1 M Salzsäure wird mit 66 mg 10%igem Palladium auf Aktivkohle versetzt und die Suspension wird über Nacht in einer Wasserstoffatmosphäre bei Normaldruck gerührt. Die Suspension wird über Celite filtriert, mit Methanol wird nachgewaschen, gefolgt vom Einengen im Vakuum. Der Rückstand wird mittels präparativer HPLC gereinigt.
Ausbeute: 29 mg (17% d. Th.)
LC-MS (Methode 1): Rₜ = 1.32 min.
MS (ESI pos.): m/z = 475 (M+H)⁺.
¹H-NMR (DMSO-d₆, 300 MHz): δ = 2.47 (s, 3H), 5.17-5.82 (m, 3H), 6.26-6.38 (m, 2H), 6.90 (s, 1H), 6.98 (s, 1H), 7.71 (d, 1H), 10.97 (s, 1H).

Als weitere Fraktion wird 2,6-Difluor-*N*¹-(3-methyl-1H-pyrrolo[2,3-b]pyridin-4-yl)benzol-1,4-diamin-Hydrochlorid isoliert. 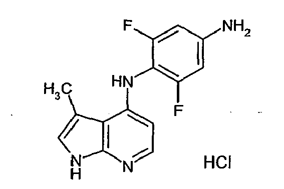 Ausbeute: 23 mg (12% d. Th.)
LC-MS (Methode 1): Rₜ = 1.33 min.
MS (ESI pos.): m/z = 475 (M+H)⁺.
¹H-NMR (DMSO-d₆, 300 MHz): δ = 2.49 (s, 3H), 6.02 (s, 2H), 6.12 (d, 1H), 6.33-6.47 (m, 2H), 6.90 (s, 1H), 6.98 (s, 1H), 7.18 (s, 1H), 7.17 (d, 1H), 7.92 (d, 1H), 8.42 (s, 1H), 12.13 (s, 1H), 14.15 (s, 1H).

### Beispiel 49A

### 2,2,2-Trifluor-N-{3-fluor-4-[(1-{[2-(trimethylsilyl)ethoxy]methyl}-1H-pyrrolo[2,3-b]pyridin-4-yl)methyl]phenyl}acetamid

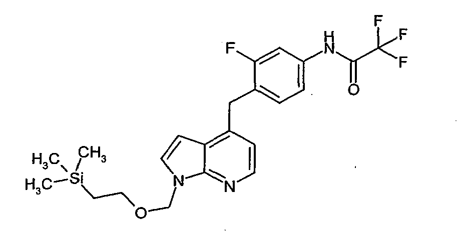

3.30 g (8.88 mmol) 3-Fluor-4-[(1-{[2-(trimethylsilyl)ethoxy]methyl}-1H-pyrrolo[2,3-b]pyridin-4-yl)methyl]anilin werden in 90 ml Dichlormethan vorgelegt, mit 1.80 g (17.8 mmol) Triethylamin versetzt und auf 0°C gekühlt. Man tropft 2.80 g (13.3 mmol) Trifluoressigsäureanhydrid hinzu und läßt 1 h bei 0°C rühren. Dann wird mit gesättigter Natriumhydrogencarbonatösung versetzt und extrahiert. Die organische Phase wird abgetrennt, über Natriumsulfat getrocknet und eingeengt.

Der Rückstand wird durch Chromatographie an Kieselgel (Eluens: Dichlormethan:Methanol = 50:1) gereinigt. Man erhält ein Öl, das langsam kristallisiert.
Ausbeute: 2.65 g (64% d. Th.)
HPLC (Methode 2): Rₜ = 3.10 min.
MS (ESI pos.): m/z = 468 [M+H]⁺.
¹H-NMR (DMSO-d₆, 400 MHz): δ = -0.12 (s, 9H), 0.79 (t, 2H), 3.50 (t, 2H), 4.24 (s, 2H), 5.60 (s 2H), 6.59 (d, 1H), 6.90 (d, 1H), 7.35-7.44 (m, 2H), 7.57 (dd, 1H), 7.60 (d, 1H), 8.18 (d, 1H), 11.39 (s, 1H).

### Beispiel 50A

### N-{4-[(3-Chlor-1-{[2-(trimethylsilyl)ethoxy]methyl}-1H-pyrrolo[2,3-b]pyridin-4-yl)methyl]-3-fluorphenyl}-2,2,2-trifluoracetamid

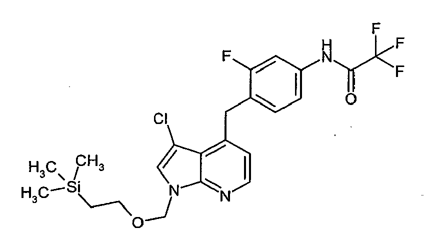

550 mg (1.07 mmol) 2,2,2-Trifluor-N-{3-fluor-4-[(1-{[2-(trimethylsilyl)ethoxy]methyl}-1H-pyrrolo[2,3-b]pyridin-4-yl)methyl]phenyl}acetamid und 143 mg (1.07 mmol) *N*-Chlorsuccinimid werden in 23 ml Tetrachlorkohlenstoff 20 min auf Rückfluss erhitzt. Nach Abkühlen wird mit Wasser und Dichlormethan verdünnt und extrahiert. Die organische Phase wird abgetrennt, über Natriumsulfat getrocknet und im Vakuum eingeengt. Der Rückstand wird durch Säulenchromatographie an Kieselgel (Eluens: Dichlormethan mit steigendem Methanol-Anteil) gereinigt.
Ausbeute: 513 mg (96% d. Th.)
HPLC (Methode 2): Rₜ = 3.23 min.
MS (ESI pos.): m/z = 502 [M+H]⁺.
¹H-NMR (DMSO-d₆, 400 MHz): δ = -0.11 (s, 9H), 0.81 (t, 2H), 3.52 (t, 2H), 4.52 (s, 2H), 5.61 (s, 2H), 6.86 (d, 1H), 7.11 (t, 1H), 7.40 (dd, 1H), 7.61 (dd, 1H), 7.86 (s, 1H), 8.26 (d, 1H), 11.41 (s, 1H).

### Beispiel 51A

### 4-[(3-Chlor-1H-pyrrolo[2,3-b]pyridin-4-yl)methyl]-3-fluoranilin

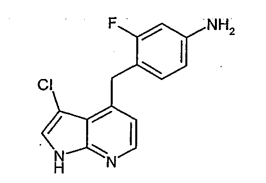

567 mg (1.13 mmol) N-{4-[(3-Chlor-1-{[2-(trimethylsilyl)ethoxy]methyl}-1H-pyrrolo[2,3-b]pyridin-4-yl)methyl]-3-fluorphenyl}-2,2,2-trifluoracetamid werden in 5 ml Dichlormethan gelöst. Man versetzt mit 5 ml (65 mmol) Trifluoressigsäure und läßt 3 h bei RT rühren. Dann wird eingeengt, mit EE versetzt und mit ges. Natriumcarbonatlösung gewaschen. Die organische Phase wird über Natriumsulfat getrocknet und eingeengt. Der Rückstand wird in 35 ml THF aufgenommen und mit 15 ml Wasser und 542 mg (22.7 mmol) Lithiumhydroxid versetzt. Die Mischung wird 20 h bei RT gerührt. Dann wird im Vakuum eingeengt und der Rückstand mit Wasser verdünnt. Man extrahiert mit Essigsäureethylester, trocknet über Natriumsulfat und engt ein. Der Rückstand wird durch Chromatographie an Kieselgel (Eluens Dichlormethan:Methanol 25:1) gereinigt. Man erhält Kristalle.
Ausbeute: 194 mg (62% d. Th.)
HPLC (Methode 1): Rₜ = 1.86 min.
MS (ESI pos.): m/z = 276 [M+H]⁺.

### Beispiel 52A

### N-{4-[(3-Brom-1-{[2-(trimethylsilyl)ethoxy]methyl}-1H-pyrrolo[2,3-b]pyridin-4-yl)methyl]-3-fluorphenyl}-2,2,2-trifluoracetamid

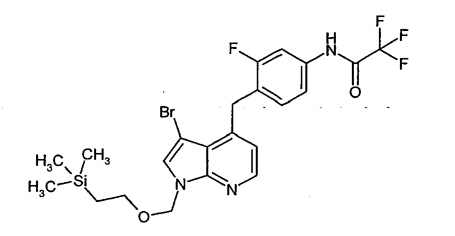

200 mg (0.38 mmol) 2,2,2-Trifluor-N-{3-fluor-4-[(1-{[2-(trimethylsilyl)ethoxy]methyl}-1H-pyrrolo[2,3-b]pyridin-4-yl)methyl]phenyl}acetamid und 68.5 mg (0.38 mmol) *N*-Bromsuccinimid werden in 8 ml Tetrachlorkohlenstoff 20 min auf Rückfluss erhitzt. Nach Abkühlen wird mit Wasser und Dichlormethan verdünnt und extrahiert. Die organische Phase wird abgetrennt, über Natriumsulfat getrocknet und im Vakuum eingeengt.
Ausbeute: 204 mg (97% d. Th.)
HPLC (Methode 1): Rₜ = 3.10 min.
MS (ESI pos.): m/z = 546, 548 [M+H]⁺.
¹H-NMR (DMSO-d₆, 400 MHz): δ = -0.11 (s, 9H), 0.81 (t, 2H), 3.52 (t, 2H), 4.56 (s, 2H), 5.62 (s, 2H), 6.85 (d, 1H), 7.07 (t, 1H), 7.39 (dd, 1H), 7.62 (dd, 1H), 7.90 (s, 1H), 8.26 (d, 1H), 11.45 (br. s, 1H).

### Beispiel 53A

### N-{4-[(3-Brom-1H-pyrrolo[2,3-b]pyridin-4-yl)methyl]-3-fluorphenyl}-2,2,2-trifluoracetamid

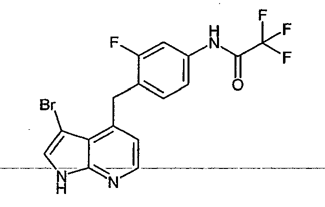

2.90 g (5.31 mmol) N-{4-[(3-Brom-1-{[2-(trimethylsilyl)ethoxy]methyl}-1H-pyrrolo[2,3-b]-pyridin-4-yl)methyl]-3-fluorphenyl}-2,2,2-trifluoracetamid werden in 40 ml Dichlormethan gelöst. Man gibt 20 ml Trifluoressigsäure hinzu und läßt 3 h bei RT rühren. Dann wird eingeengt, mit Essigsäureethylester versetzt und mit gesättigter Natriumcarbonatlösung geschüttelt. Die organische Phase wird über Natriumsulfat getrocknet und eingeengt. Das erhaltene Rohprodukt wird in 100 ml THF gelöst und mit einer Lösung von 550 mg (22.9 mmol) Lithiumhydroxid in 10 ml Wasser versetzt. Man lässt 30 min bei RT rühren, verdünnt dann mit Wasser und extrahiert mit Essigsäureethylester. Die organische Phase wird mit gesättigter Natriumchloridlösung geschüttelt, über Natriumsulfat getrocknet und eingeengt. Das Produkt wird ohne weitere Aufreinigung umgesetzt.
Ausbeute: 1.80 g (69% d. Th.)
HPLC (Methode 2): Rₜ = 2.55 min.
MS (ESI pos.): m/z = 414, 416 [M+H]⁺.
¹H-NMR (DMSO-d₆, 400 MHz): δ = 4.55 (s, 2H), 6.74 (d, 1H), 7.09 (t, 1H), 7.40 (dd, 1H), 7.62 (dd, 1H), 7.68 (d, 1H), 8.18 (d, 1H), 11.42 (br. s, 1H), 12.13 (br. s, 1H).

### Beispiel 54A

### N-[4-({3-Brom-1-[(4-methylphenyl)sulfonyl]-1H-pyrrolo[2,3-b]pyridin-4-yl}methyl)-3-fluorphenyl]-2,2,2-trifluoracetamid

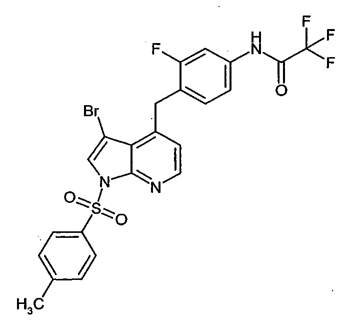

1.70 g (4.08 mmol) N-{4-[(3-Brom-1H-pyrrolo[2,3-b]pyridin-4-yl)methyl]-3-fluorphenyl}-2,2,2-trifluoracetamid werden in 50 ml THF gelöst und auf -78°C gekühlt. Eine Lösung von n-Butyllithium (2.81 ml, 1.6M in Hexan, 4.49 mmol) wird hinzugetropft. Nach 15 min wird eine Lösung von 860 mg (4.49 mmol) *p*-Toluolsulfonylchlorid in 5 ml THF hinzugetropft. Man lässt auf RT kommen und 1 h rühren. Dann wird Wasser und gesättigte Natriumhydrogencarbonat-lösung zugesetzt und mit Essigsäureethylester extrahiert. Die organische Phase wird über Natriumsulfat getrocknet. Das Lösungsmittel wird im Vakuum entfernt und der Rückstand durch Säulenchromatographie an Kieselgel (Eluens Dichlormethan:Methanol = 50 : 1) gereinigt.
Ausbeute: 1.10 g (47% d. Th.)
HPLC (Methode 2): Rₜ = 3.08 min.
MS (ESI pos.): m/z = 570, 572 [M+H]⁺.
¹H-NMR (DMSO-d₆, 400 MHz): δ = 2.36 (s, 3H), 4.53 (s, 2H), 6.96 (d, 1H), 7.08 (t, 1H), 7.40 (dd, 1H), 7.44 (d, 2H), 7.61 (dd, 1H), 8.03 (d, 2H), 8.18 (s, 1H), 8.32 (d, 1H), 11.43 (s, 1H).

### Beispiel 55A

### 4-(4-Amino-2-fluorbenzyl)-1H-pyrrolo[2,3-b]pyridin-3-carbonitril

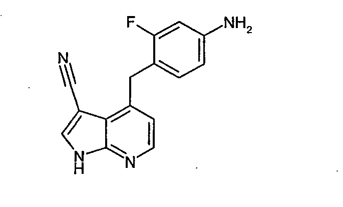

1.70 g (2.98 mmol) N-[4-({3-Brom-1-[(4-methylphenyl)sulfonyl]-1H-pyrrolo[2,3-b]pyridin-4-yl}-methyl)-3-fluorphenyl]-2,2,2-trifluoracetamid, 219 mg (1.19 mmol) Zinkacetat, 78 mg (1.19 mmol) Zinkstaub, 189 mg (1.61 mmol) Zinkcyanid, 50.0 mg (0.09 mmol) Bis(diphenylphosphino)ferrocen und 27.3 mg (0.03 mmol) Tris(dibenzylidenaceton)dipalladium werden vorgelegt. Man gibt unter Argon eine entgaste Mischung aus 17 ml DMF und 0.17 ml Wasser hinzu und erhitzt 20 Stunden auf 100°C. Die Rohmischung wird in 10 ml Wasser, 8 ml THF und 1.21 g (50.3 mmol) Lithiumhydroxid versetzt und 20 h bei RT gerührt. Man engt dann etwas ein und extrahiert mit Essigsäureethylester. Die organische Phase wird über Natriumsulfat getrocknet und im Vakuum eingeengt. Man reinigt durch Chromatographie an Kieselgel (Eluens: Dichormethan:Methanol 25:1).
Ausbeute: 477 mg (35% d. Th.)
LC-MS (Methode 3): Rₜ = 1.84 min.
MS (ESI pos.): m/z = 267 [M+H]⁺.
¹H-NMR (DMSO-d₆, 300 MHz): δ = 4.25 (s, 2H), 5.31 (s, 2H), 6.30-6.37 (m, 2H), 6.83 (d, 1H), 6.89 (t, 1H), 8.25 (d, 1H), 8.42 (s, 1H), 12.2 (br. s, 1H).

### Beispiel 56A

### 4-Chlor-1-[(4-methylphenyl)sulfonyl]-3-(trimethylstannyl)-1H-pyrrolo[2,3-b]pyridin

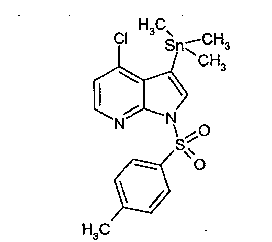

In einen Schlenkkolben (im Hochvakuum ausgeheizt und mit Argon belüftet) wird 1.0 g 4-Chlor-3-iod-1-[(4-methylphenyl)sulfonyl]-1H-pyrrolo[2,3-b]pyridin (2.31 mmol) in wasserfreiem THF (30 ml) vorgelegt und auf -78°C gekühlt. Dazu tropft man langsam 2.85 ml tert.-Butyllithium (1.7 M in Pentan) (4.85 mmol) und läßt 30 min bei -78°C rühren. Dann tropft man 2.42 ml einer 1M Lösung aus Trimethylzinnchlorid in THF zu und läßt über Nacht auf RT kommen. Nun gibt man 10 ml einer gesättigten Kaliumfluoridlösung zu, trennt die organische Phase ab und extrahiert einmal mit Essigsäureethylester. Man trocknet die vereinigten organischen Phasen über Magnesiumsulfat und engt die Lösung ein. Das Produkt wird durch Säulenchromatographie an Kieselgel (Eluens: Toluol/Essigsäureethylester 20:1) gereinigt.
LC-MS (Methode 2): Rₜ = 3.31 min.
MS (ESI pos.): m/z = 471 (M+H)+.
¹H-NMR (DMSO-d₆, 300 MHz): δ = 0.40 (s, 9H), 2.35 (s, 3H), 7.40-7.45 (m, 3H), 7.67 (s, 1H),
8.05 (d, 2H), 8.30 (d, 1H).

### Beispiel 57A

### 4-Chlor-3-fluor-1-[(4-methylphenyl)sulfonyl]-1H-pyrrolo[2,3-b]pyridin

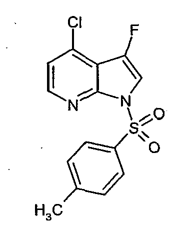

488 mg (1.03 mmol) 4-Chlor-1-[(4-methylphenyl)sulfonyl]-3-(trimethylstannyl)-1H-pyrrolo[2,3-b]pyridin werden in 20 ml wasserfreiem Acetonitril gelöst. Dann gibt man in einer Portion 368 mg (1.03 mmol) Selectfluor^{®} hinzu und lässt über Nacht bei RT rühren. Zur Aufarbeitung saugt man vom ausgefallenen Niederschlag ab und engt das Filtrat ein. Der Rückstand wird über präparative HPLC gereinigt. Das Produkt ist ein Feststoff.
LC-MS (Methode 3): Rₜ = 2.80 min.
MS (ESI pos.): m/z = 325 (M+H)⁺.
¹H-NMR (DMSO-d₆, 300 MHz): δ = 2.34 (s, 3H), 7.43 (d, 2H), 7.52 (d, 1H), 7.97 (d, 2H), 8.15 (d, 1H), 8.40 (d, 1H).

### Beispiel 58A

### 3-Fluor-N-(2-fluor-4-nitrophenyl)-1-[(4-methylphenyl)sulfonyl]-1H-pyrrolo[2,3-b]pyridin-4-amin

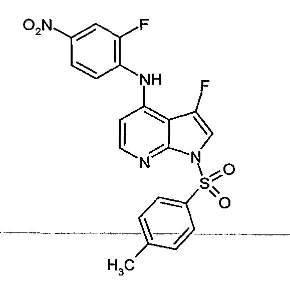

Eine Lösung von 20 mg (0.06 mmol) 4-Chlor-3-fluor-1-[(4-methylphenyl)sulfonyl]-1H-pyrrolo[2,3-b]pyridin, 11.5 mg (0.074 mmol) 2-Fluor-4-nitroanilin, 5.6 mg (0.006 mmol) Tris(dibenzylidenaceton)dipalladium, 5.8 mg (0.012 mmol) Dicyclohexyl(2',4',6'-triisopropylbiphenyl-2-yl)phosphin und 12.7 mg (0.09 mmol) Kaliumcarbonat in 1.00 ml entgastem *tert-*Butanol wird für 3 h im geschlossenen Druckgefäß bei 100°C gerührt. Nach Abkühlen auf RT wird die Reaktionsmischung über Kieselgur filtriert, mit Essigsäureethylester nachgewaschen, und vom Filtrat wird das Lösungsmittel entfernt. Der Rückstand wird durch präparative HPLC gereinigt.
Ausbeute: 21.5 mg (67% d. Th.)
LC-MS (Methode 1): Rₜ = 2.56 min.
MS (ESI pos.): m/z = 445 (M+H)⁺.

### Beispiel 59A

### 3-Fluor-N-(2-fluor-4-nitrophenyl)-1H-pyrrolo[2,3-b]pyridin-4-amin

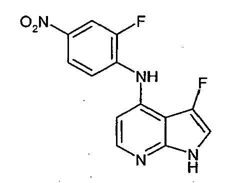

Zu einer Lösung von 80 mg (0.18 mmol) 3-Fluor-N-(2-fluor-4-nitrophenyl)-1-[(4-methylphenyl)-sulfonyl]-1H-pyrrolo[2,3-b]pyridin-4-amin in 5 ml Ethanol werden 0.4 ml einer 20%igen wässrigen Natriumhydroxid-Lösung gegeben, und die Mischung wird über Nacht gerührt. Dann wird die Reaktionslösung auf ein Gemisch aus Wasser und Essigsäureethylester gegossen und die wässrige Phase noch zweimal mit Essigsäureethylester extrahiert. Reinigung mittels präparativer HPLC liefert die Zielverbindung.
Ausbeute: 47.5 mg (91% d. Th.)
LC-MS (Methode 1): Rₜ = 1.89 min.
MS (ESI pos.): m/z = 291 (M+H)⁺.

### Beispiel 60A

### 2-Fluor-N¹-(3-fluor-1H-pyrrolo[2,3-b]pyridin-4-yl)benzen-1,4-diamin

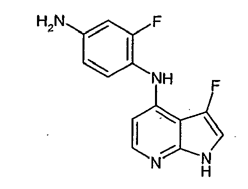

Eine Lösung von 47 mg (0.17 mmol) 3-Fluor-N-(2-fluor-4-nitrophenyl)-1H-pyrrolo[2,3-b]pyridin-4-amin in 5 ml Ethanol wird mit 4 mg Platin(IV)oxid versetzt, und die Suspension wird über Nacht in einer Wasserstoffatmosphäre bei Normaldruck gerührt. Die Suspension wird über Kieselgur filtriert und mit Ethanol wird nachgewaschen, und das Filtrat wird eingeengt. Das Produkt wird über präparative HPLC gereinigt.
Ausbeute: 7.8 mg (16% d. Th.)
LC-MS (Methode 2): Rₜ = 1.14 min.
MS (ESI pos.): m/z = 261 (M+H)⁺.

### Beispiel 61A

### 4-Chlor-3-iod-1H-pyrrolo[2,3-b]pyridin

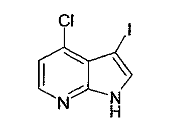

Es werden 250 g (1.64 mol) 4-Chlor-1*H*-pyrrolo[2,3-b]pyridin zusammen mit 328 g (5.85 mol) pulverförmigen Kaliumhydroxid in 3000 ml DMF vorgelegt und auf 0°C abgekühlt. Zu dieser Suspension wird unter Kühlung eine Lösung aus 416 g (1.64 mol) Iod in 3000 ml DMF getropft und nach vollständiger Zugabe wird 4 h bei 0°C gerührt. Nach Vervollständigung der Reaktion wird der Ansatz unter Rühren auf Eiswasser gegeben, der gebildete Feststoff abgesaugt und anschließend im Hochvakuum getrocknet. Es werden 403 g (88% d. Th.) der Titelverbindung als Feststoff isoliert.
¹H-NMR (DMSO-d₆, 300 MHz): δ = 7.21 (d, 1H, H-arom), 7.81 (d, 1H, H-arom), 8.19 (d, 1H, H-arom), 12.45 (br s, 1H, N-H).

### Beispiel 62A

### 4-Chlor-1-(toluol-4-sulfonyl)-3-iod-1H-pyrrolo[2,3-b]pyridin

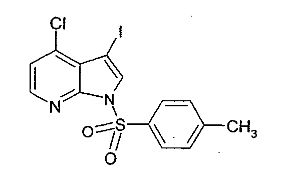

Unter Schutzgasatmosphäre (Argon) werden 63.5 g (1.59 mol) Natriumhydrid in 1000 ml abs. THF suspendiert. Hierzu tropft man unter Kühlung bei einer Temperatur von 0-5°C eine Lösung aus 402 g (1.44 mol) 4-Chlor-3-iod-1*H*-pyrrolo[2,3-b]pyridin in 3000 ml abs. THF und läßt nach vollständiger Zugabe das Reaktionsgemisch 15 Minuten nachrühren. Anschließend werden 358 g (1.88 mol) p-Toluolsulfonsäurechlorid portionsweise zum Reaktionsgemisch zugegeben, so dass die Temperatur zwischen 5-10°C gehalten wird. Nach Beendigung der Zugabe läßt man den Ansatz auf 20°C kommen und rührt eine weitere Stunde bei dieser Temperatur. Nach vollständigem Umsatz (DC-Umsatzkontrolle) wird das Reaktionsgemisch mit 20 1 gesättigter Natriumhydrogencarbonat-Lösung sowie 10 l Essigsäureethylester versetzt, extrahiert und die organische Phase abgetrennt. Die wässrige Phase wird noch zweimal mit je 10 l Essigsäureethylester nachextrahiert, die organischen Phasen vereinigt, getrocknet und im Wasserstrahlvakuum von flüchtigen Bestandteilen befreit. Zur Aufreinigung des kristallinen Rohproduktes wird dieses in Dichlormethan aufgenommen und über Kieselgel chromatographiert (Dichlormethan:Petrolether = 3:7). Nach Abziehen der Lösungsmittel wird der Kristallbrei in Diethylether ausgerührt, abgesaugt und im Hochvakuum bei 20°C getrocknet. Es werden 372 g (60% d. Th.) der Titelverbindung als Feststoff isoliert.
¹H-NMR (DMSO-d₆, 300 MHz): δ = 2.35 (s, 3H, -CH₃), 7.44 (d, 2H, H-arom), 7.46 (d, 1H, H-arom), 8.02 (d, 2H, H-arom), 8.23 (s, 1H, H-arom), 8.34 (d, 1H, H-arom).

### Beispiel 63A

### 4-Chlor-1-(toluol-4-sulfonyl)-3-trifluormethyl-1H-pyrrolo[2,3-b]pyridin

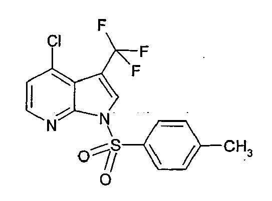

Unter Schutzgasatmosphäre (Argon) werden 23.1 g (0.398 mol) trockenes Kaliumfluorid und 75.7 g (0.398 mol) Kupfer(I)iodid miteinander verrieben und im Hochvakuum unter Schütteln 10 Minuten auf 220°C erhitzt, bis eine leicht grünliche Verfärbung entsteht. In einem Reaktionskolben werden unter Schutzgasatmosphäre (Argon) zunächst das geglühte Gemisch aus Kaliumfluorid und Kupfer(I)iodid zusammen mit 170 ml absolutiertem N-Methylpyrolidin und 170 ml absolutiertem DMF vorgelegt und anschließend bei 20°C unter Rühren 86 g (0.199 mol) 4-Chlor-1-(toluol-4-sulfonyl)-3-iod-1*H*-pyrrolo[2,3-b]pyridin portionsweise eingetragen, wobei sich eine hellgraue Suspension bildet. Man tropft innerhalb von 20 Minuten 62.1 g (0.437 mol) Trimethyltrifluormethylsilan zum Reaktionsgemisch hinzu und läßt 18 h bei 20°C rühren. Nach vollständigem Umsatz (GC-Umsatzkontrolle) wird das Reaktionsgemisch auf 2000 ml Methyl-tert.-butylether gegeben, die organische Phase dekantiert und der Rückstand ein weiteres Mal mit 1000 ml Methyl-tert.-butylether dispergiert. Die vereinten organischen Phasen werden dreimal mit je 2000 ml Wasser gewaschen und anschließend über Magnesiumsulfat getrocknet. Nach Filtration und Entfernen der flüchtigen Bestandteile im Wasserstrahlvakuum resultieren 50.1 g (67% d. Th.) der Zeilverbindung als Feststoff.
¹H-NMR (DMSO-d₆, 300 MHz): δ = 2.37 (s, 3H, -CH₃), 7.48 (d, 2H, H-arom), 7.62 (d, 1H, H-arom), 8.12 (d, 2H, H-arom), 8.47 (d, 1H, H-arom), 8.67 (s, 1H, H-arom).

### Beispiel 64A

### 4-Chlor-3-trifluormethyl-1H-pyrrolo[2,3-b]pyridin

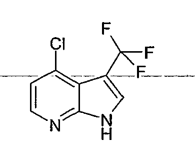

Zu einer Lösung von 30 g (80.0 mmol) 4-Chlor-1-(toluol-4-sulfonyl)-3-trifluormethyl-1*H-*pyrrolo[2,3-b]pyridin in 250 ml absolutiertem THF gibt man 52.3 g (200 mmol) Tetra-*n-*butylammoniumfluorid und rührt bei 20°C für 20 Minuten. Nach vollständigem Umsatz (DC-Kontrolle) versetzt man mit 300 ml gesättigter Natriumhydrogencarbonat-Lösung und extrahiert anschliessend dreimal mit je 500 ml Essigsäureethylester. Die vereinten organischen Phasen werden mit gesättigter Natriumchlorid-Lösung gewaschen, über Magnesiumsulfat getrocknet und im Wasserstrahlvakuum von flüchtigen Bestandteilen befreit. Nach Aufreinigung des kristallinen

Rohproduktes durch Chromatographie über Kieselgel (Dichlormethan:Methanol = 30:1) resultieren 13.1 g (74% d. Th.) der Titelverbindung als Feststoff.
¹H-NMR (DMSO-d₆, 300 MHz): δ = 7.40 (d, 1H, H-arom), 8.28 (s, 1H, H-arom), 8.34 (d, 1H, H-arom), 12.88 (br s 1H, N-H).

### Beispiel 65A

### 4-Chlor-3-(trifluormethyl)1-{[2-(trimethylsilyl)ethoxy]methyl}-1H-pyrrolo[2,3-b]pyridin

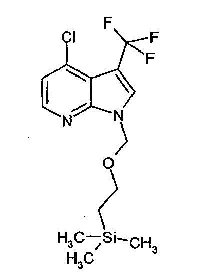

Zu einer Lösung von 200 mg (0.90 mmol) 4-Chlor-3-(trifluormethyl)-1H-pyrrolo[2,3-b]pyridin und 177 µl (0.99 mmol) Trimethylsilylethoxymethylchlorid in DMF (10 ml) werden bei 0°C 37 mg (0.90 mmol) Natriumhydrid gegeben. Die Reaktionsmischung wird für 1 h bei RT gerührt. Die Suspension wird auf Eis-Wasser gegossen und gerührt bis die Mischung aufRT kommt. Dann wird sie dreimal mit Essigsäureethylester extrahiert. Die vereinigten organische Phasen werden nacheinander mit gesättigten Lösungen von Natriumhydrogencarbonat und Natriumchlorid gewaschen. Die organische Lösung wird über Magnesiumsulfat getrocknet und eingeengt. Der Rückstand wird durch Säulenchromatographie an Kieselgel (Eluens: Cyclohexan/Essigsäureethylester 10:1) gereinigt.
Ausbeute: 286 mg (90% d. Th.)
LC-MS (Methode 2): Rₜ = 3.26 min.
MS (ESI pos.): m/z = 351 (M+H)⁺.
1H-NMR (DMSO-d₆, 300 MHz): δ = -0.09 (s, 9H), 0.93 (t, 2H), 3.63 (t, 2H), 5.84 (s, 2H), 7.48 (d, 1H), 8.40 (d, 1H), 8.52 (s, 1H).

### Beispiel 66A

### N-(2-Fluor-4-nitrophenyl)-3-(trifluormethyl)-1-{[2-(trimethylsilyl)ethoxy]methyl}-1H-pyrrolo[2,3-b]pyridin-4-amin

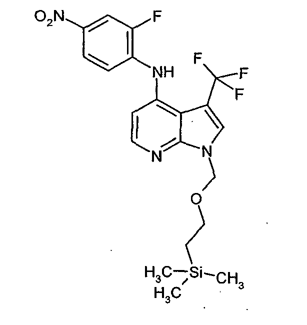

Eine Lösung von 170 mg (0.485 mmol) 4-Chlor-3-(trifluormethyl)1-{[2-(trimethylsilyl)ethoxy]-methyl}-1H-pyrrolo[2,3-b]pyridin, 91 mg (0.58 mmol) 2-Fluor-4-nitroanilin, 31 mg (0.035 mmol) Tris(dibenzylidenaceton)dipalladium, 33 mg (0.07 mmol) Dicyclohexyl(2',4',6'-triisopropylbiphenyl-2-yl)phosphin und 100 mg (0.72 mmol) Kaliumcarbonat in 3.00 ml entgastem *tert-*Butanol wird für 3 h im geschlossenen Druckgefäß bei 100°C gerührt. Nach Abkühlen auf RT wird die Reaktionsmischung über Kieselgur filtriert, mit Essigsäureethylester nachgewaschen, und vom Filtrat wird das Lösungsmittel entfernt. Der Rückstand wird durch präparative HPLC gereinigt.
Ausbeute: 187 mg (82% d. Th.)
LC-MS (Methode 3): Rₜ = 3.32 min.
MS (ESI pos.): m/z = 471 (M+H)⁺.
¹H-NMR (DMSO-d₆, 400 MHz): δ = -0.08 (s, 9H), 0.84 (t, 2H), 3.58 (t, 2H), 5.70 (s, 2H), 7.04 (t, 1H), 7.24 (d, 1H), 7.96 (dd, 1H), 8.15 (dd, 1H), 8.40 (s, 1H), 8.43 (d, 1H), 8.47 (s, 1H).

### Beispiel 67A

### N-(2-Fluor-4-nitrophenyl)-3-(trifluormethyl)-1H-pyrrolo[2,3-b]pyridin-4-amin

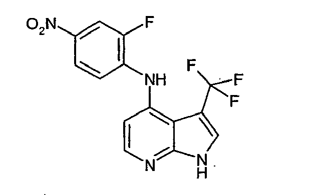

Zu einer Lösung von 182 mg (0.387 mmol) N-(2-Fluor-4-nitrophenyl)-3-(trifluormethyl)-1-{[2-(trimethylsilyl)ethoxy]methyl}-1H-pyrrolo[2,3-b]pyridin-4-amin in 3.00 ml Dichlormethan werden 3 ml Trifluoressigsäure zugegeben, und es wird 30 min bei RT gerührt. Die Lösungsmittel werden im Vakuum entfernt. Der Rückstand wird in Essigsäureethylester gelöst und nacheinander mit gesättigten Lösungen von Natriumhydrogencarbonat und Natriumchlorid gewaschen, die organische Phase wird mit Magnesiumsulfat getrocknet, und die Lösung wird eingeengt. Das Produkt wird ohne weitere Reinigung verwendet.
Ausbeute: 129 mg (98% d. Th.)
LC-MS (Methode 3): Rₜ = 2.71 min.
MS (ESI pos.): m/z = 341 (M+H)⁺.

### Beispiel 68A

### 2-Fluor-N¹-{3-(trifluormethyl)-1H-pyrrolo[2,3-b]pyridin-4-yl}benzen-1,4-diamin

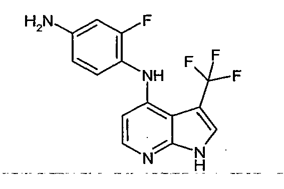

Eine Lösung von 129 mg (0.38 mmol) N-(2-Fluor-4-nitrophenyl)-3-(trifluormethyl)-1H-pyrrolo[2,3-b]pyridin-4-amin in 5 ml Ethanol wird mit 10 mg 10%igem Palladium auf Aktivkohle versetzt, und die Suspension wird über Nacht in einer Wasserstoffatmosphäre bei Normaldruck gerührt. Die Suspension wird über Kieselgur filtriert und mit Ethanol wird nachgewaschen. Das Filtrat wird eingeengt. Das Produkt wird über präparative HPLC gereinigt.
Ausbeute: 18 mg (15% d. Th.)
LC-MS (Methode 3): Rₜ = 1.59 min.
MS (ESI pos.): m/z = 311 (M+H)⁺.
¹H-NMR (DMSO-d₆, 300 MHz): δ = 6.18 (dd, 1H), 6.28 (s, 1H), 6.40-6.50 (m, 2H), 7.16 (t, 1H), 7.93 (s, 1H), 7.95 (d, 1H), 12.24 (s, 1H).

### Ausführungsbeispiele

### Beispiel 1

### N⁴-[3-Fluor-4-(1H-pyrrolo[2,3-b]pyridin-4-ylamino)phenyl]-6-(trifluormethyl)pyrimidin-2,4-diamin

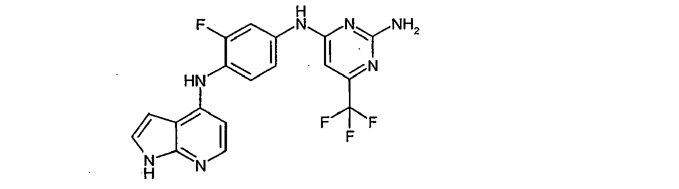

139 mg (0.5 mmol) 2-Fluor-*N*¹-1H-pyrrolo[2,3-b]pyridin-4-yl-1,4-diaminobenzol und 147 mg (0.75 mmol) 4-Chlor-6-(trifluormethyl)pyrimidin-2-amin werden in 10 ml Wasser suspendiert. Man setzt 0.75 ml 1M Salzsäure hinzu. Es wird über Nacht auf 100°C erhitzt. Die Suspension wird dann mit 1N Natronlauge auf pH10 gestellt, wobei Kristalle ausfallen. Es wird abgesaugt mit Wasser nachgewaschen. Das Rohprodukt wird durch Säulenchromatographie an Kieselgel gereinigt (Eluens: Dichlormethan/Methanol (7M Ammoniak) 100:1 bis 10:1).
Ausbeute: 62 mg (26% d. Th.)
LC-MS (Methode 2): Rₜ = 1.52 min.
MS (ESI pos.): m/z = 404 [M+H]⁺.
¹H-NMR (DMSO-d₆, 400 MHz): δ = 6.13 (d, 1H), 6.38 (s, 1H), 6.53 (m, 1H), 6.97 (s, 2H), 7.16 (s, 1H), 7.29-7.40 (m, 2H), 7.83 (d, 1H), 8.11 (d, 1H), 8.33 (s, 1H), 9.82 (s, 1H), 11.27 (s, 1H).

### Beispiel 2

### 6-Chlor-N⁴-[3-fluor-4-(1H-pyrrolo[2,3-b]pyridin-4-ylamino)phenyl]pyrimidin-2,4-diamin

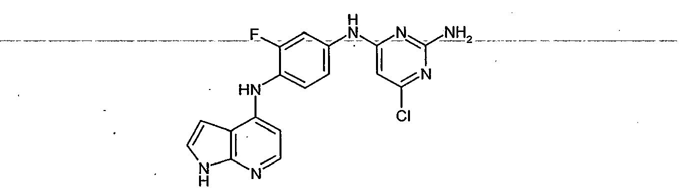

28 mg (0.12 mmol) 2-Fluor-*N*¹-1H-pyrrolo[2,3-b]pyridin-4-yl-1,4-diaminobenzol und 27 mg (0.16 mmol) 4,6-Dichlorpyrimidin-2-amin werden in 5 ml Wasser suspendiert. Man setzt 0.16 ml 1M Salzsäure hinzu, und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird durch Säulenchromatographie an Kieselgel gereinigt (Eluens: Dichlormethan/Methanol (7M Ammoniak) 100:1 bis 10:1).
Ausbeute: 33 mg (73% d. Th.)
LC-MS (Methode 3): Rₜ = 1.47 min.
MS (ESI pos.): m/z = 370 (M+H)⁺.
¹H-NMR (DMSO-d₆, 300 MHz): δ = 6.01 (s, 1H), 6.12 (d, 1H), 6.48-6.53 (m, 1H), 6.84 (s, 2H), 7.16 (d, 1H), 7.23-7.37 (m, 2H), 7.81 (d, 1H), 8.05 (d, 1H), 8.29 (s, 1H), 9.53 (s, 1H), 11.28 (s, 1H).

### Beispiel 3

### N⁴-[3-Fluor-4-(1H-pyrrolo[2,3-b]pyridin-4-ylamino)phenyl]pyrimidin-2,4-diamin

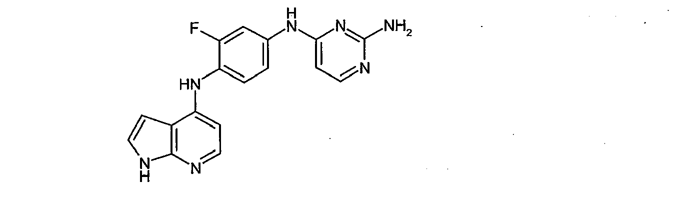

32 mg (0.10 mmol) 2-Fluor-*N*¹-1H-pyrrolo[2,3-b]pyridin-4-yl-1,4-diaminobenzol und 18 mg (0.14 mmol) 4-Chlorpyrimidin-2-amin werden in 5 ml Wasser suspendiert. Man setzt 0.14 ml 1M Salzsäure hinzu, und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird durch Säulenchromatographie an Kieselgel gereinigt (Eluens: Dichlormethan/Methanol (7M Ammoniak) 100:1 bis 10:1).
Ausbeute: 19 mg (55% d. Th.)
LC-MS (Methode 5): Rₜ = 2.30 min.
MS (ESI neg.): m/z = 334 (M-H)⁻.
¹H-NMR (DMSO-d₆, 300 MHz): δ = 6.02 (d, 1H), 6.10 (d, 1H), 6.30 (s, 2H), 6.48-6.53 (m, 1H), 7.16 (d, 1H), 7.20-7.29 (m, 1H), 7.31-7.87 (m, 1H), 7.72-7.89 (m, 2H), 8.11 (d, 1H), 8.27 (s, 1H), 9.38 (s, 1H), 11.26 (s, 1H).

### Beispiel 4

### 6-Chlor-N⁴-{4-[(3-chlor-1H-pyrrolo[2,3-b]pyridin-4-yl)amino]-3-fluorphenyl}pyrimidin-2,4-diamin

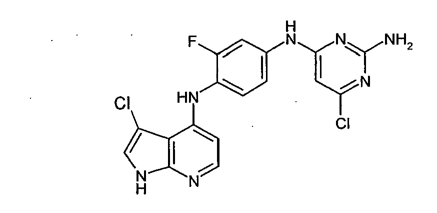

57 mg (210 µmol) *N*¹-(3-Chlor-1H-pyrrolo[2,3-b]pyridin-4-yl)-2-fluorbenzol-1,4-diamin und 47 mg (290 µmol) 4,6-Dichlorpyrimidin-2-amin werden in 5 ml Wasser suspendiert. Man setzt 290 µl (290 µmol) 1M Salzsäure hinzu, und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird durch Säulenchromatographie an Kieselgel (Eluens: Dichlormethan/Methanol (7M Ammoniak) 100:1 bis 10:1) und anschließender präparativer RP-HPLC gereinigt.
Ausbeute: 9 mg (11% d. Th.)
LC-MS (Methode 2): Rₜ = 1.67 min.
MS (ESI pos.): m/z = 404 (M+H)⁺.

### Beispiel 5

### 6-(4-Fluorphenyl)-N⁴-[3-fluor-4-(1H-pyrrolo[2,3-b]pyridin-4-ylamino)phenyl]pyrimidin-2,4-diamin

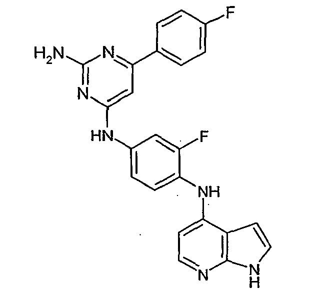

100 mg (0.27 mmol) 2-Fluor-*N*¹-(1-{[2-(trimethylsilyl)ethoxy]methyl}-1H-pyrrolo[2,3-b]pyridin-4-yl)benzol-1,4-diamin und 63 mg (0.28 mmol) 4-Chlor-6-(4-fluorphenyl)pyrimidin-2-amin werden in 3 ml Wasser suspendiert. Anschließend werden 39 µl (0.40 mmol) einer 37%igen Salzsäure-Lösung zugegeben und die Mischung über Nacht unter Rückfluss erhitzt. Nach Zugabe von weiteren 39 µl (0.40 mmol) einer 37%igen Salzsäure-Lösung wird erneut über Nacht unter Rückfluss umgesetzt. Die Suspension wird dann mit 1N Natronlauge auf pH 10 gebracht, wobei Kristalle ausfallen. Der Feststoff wird abfiltriert, mit etwas Wasser gewaschen, dann in DMSO gelöst und mittels präparativer RP-HPLC gereinigt.
Ausbeute: 32 mg (26% d. Th.)
LC-MS (Methode 9): Rₜ = 1.33 min.
MS (ESI neg.): m/z = 428 (M-H)⁻.
¹H-NMR (DMSO-d₆, 300 MHz): δ = 6.12 (d, 1H), 6.38-6.59 (m, 4H), 7,11-7.18 (m, 1H), 7.22-7.47 (m, 4H), 7.83 (d, 1H), 7.93-8.07 (m, 2H), 8,12 (d, 1H), 8.25 (s, 1H), 9.45 (s, 1H), 11.22 (s, 1H).

### Beispiel 6

### N⁴-[3-Fluor-4-(1H-pyrrolo[2,3-b]pyridin-4-ylamino)phenyl]-6-pyridin-4-yl-pyrimidin-2,4-diamin

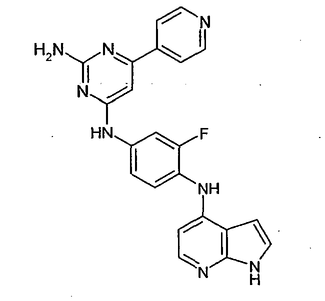

70 mg (0.19 mmol) 2-Fluor-*N*¹-(1-{[2-(trimethylsilyl)ethoxy]methyl}-1H-pyrrolo[2,3-b]pyridin-4-yl)benzol-1,4-diamin und 41 mg (0.20 mmol) 4-Chlor-6-pyridin-4-ylpyrimidin-2-amin werden in 2.5 ml Wasser suspendiert. Anschließend werden 27 µl (0.28 mmol) einer 37%igen Salzsäure-Lösung zugegeben und die Mischung über Nacht unter Rückfluss erhitzt. Die Suspension wird dann mit 1N Natronlauge auf pH 10 gebracht, wobei Kristalle ausfallen. Der Feststoff wird abfiltriert, mit etwas Wasser gewaschen, dann in DMSO gelöst und mittels präparativer RP-HPLC gereinigt.
Ausbeute: 14 mg (18% d. Th.)
LC-MS (Methode 7): Rₜ = 1.64 min.
MS (ESI pos.): m/z = 413 (M+H)⁺.
¹H-NMR (DMSO-d₆, 300 MHz): δ = 6.05-6.18 (m, 1H), 6.50-6.72 (m, 4H), 7.08-7.19 (m, 1H), 7.21-7.48 (m, 2H), 7.71-7.93 (m, 3H), 8.07-8.21 (m, 1H), 8.31 (s, 1H), 8.68 (s, 1H), 9.62 (s, 1H), 11.31 (s, 1H).

### Beispiel 7

### 6-Chlor-N⁴-[3-fluor-4-(1H-pyrrolo[2,3-b]pyridin-4-ylmethyl)phenyl]pyrimidin-2,4-diamin

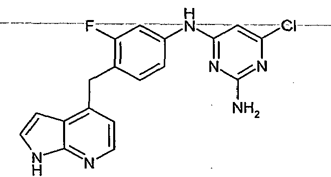

40 mg (0.15 mmol) 3-Fluor-4-(1H-pyrrolo[2,3-b]pyridin-4-ylmethyl)anilin und 29 mg (0.18 mmol) 4,6-Dichlorpyrimidin-2-amin werden in 2 ml Wasser und 0.11 ml 2N Salzsäure suspendiert. Man erhitzt die Reaktionsmischung 15 Stunden auf Rückfluss. Nach Abkühlen stellt man mit verdünnter Natronlauge alkalisch, setzt etwas DMF hinzu und extrahiert mit Essigsäureethylester. Man trocknet über Natriumsulfat und entfernt das Lösungsmittel im Vakuum. Der Rückstand wird mittel präparativer HPLC aufgereinigt.
Ausbeute: 27 mg (50% d. Th.)
LC-MS (Methode 2): Rₜ = 1.89 min.
MS (ESI pos.): m/z = 369 [M+H]⁺.
¹H-NMR (DMSO-d₆, 400 MHz): δ = 4.17 (s, 2H), 5.99 (s, 1H), 6.50-6.52 (m, 1H), 6.77-6.84 (m, 3H), 7.19-7.24 (m, 2H), 7.42 (dd, 1H), 7.83 (d, 1H), 8.11 (d, 1H), 9.47 (s, 1H), 11.62 (s, 1H).

### Beispiel 8

### N⁴-[3-Fluor-4-(1H-pyrrolo[2,3-b]pyridin-4-ylmethyl)phenyl]-6-(trifluormethyl)pyrimidin-2,4-diamin

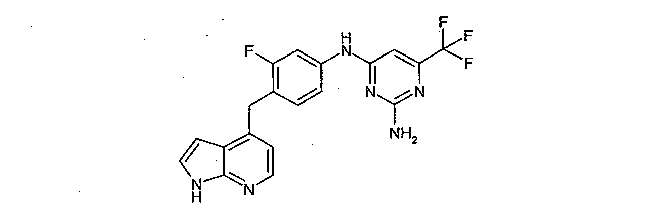

32 mg (0.11 mmol) 3-Fluor-4-(1H-pyrrolo[2,3-b]pyridin-4-ylmethyl)anilin und 26 mg (0.12 mmol) 4-Chlor-6-(trifluormethyl)pyrimidin-2-amin werden in 5 ml Wasser und 0.07 ml 2N Salzsäure suspendiert. Man erhitzt die Reaktionsmischung 20 Stunden auf Rückfluss. Nach Abkühlen stellt man mit verdünnter Natronlauge alkalisch, setzt etwas Methanol hinzu und extrahiert mit Essigsäureethylester. Man trocknet über Natriumsulfat und entfernt das Lösungsmittel im Vakuum. Der Rückstand wird mittel präparativer HPLC aufgereinigt.
Ausbeute: 24 mg (55% d. Th.)
LC-MS (Methode 2): Rₜ = 2.05 min.
MS (ESI pos.): m/z = 403 [M+H]⁺.
¹H-NMR (DMSO-d₆, 400 MHz): δ = 4.18 (s, 2H), 6.33 (s, 1H), 6.51 (dd, 1H), 6.82 (d, 1H), 6.93 (br. s, 2H), 7.21-7.29 (m, 2H), 7.41 (dd, 1H), 7.89 (dd, 1H), 8.11 (d, 1H), 9.72 (s, 1H), 11.60 (br. s, 1H).

### Beispiel 9

### {4-[(2-Amino-6-chlorpyrimidin-4-yl)amino]-2-fluorphenyl}(1H-pyrrolo[2,3-b]pyridin-4-yl)keton

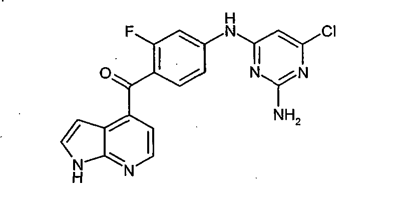

Das Produkt wurde als Nebenprodukt bei der Herstellung von 6-Chlor-*N*⁴-[3-fluor-4-(1H-pyrrolo[2,3-b]pyridin-4-ylmethyl)phenyl]pyrimidin-2,4-diamin nach Aufreinigung per HPLC erhalten.
LC-MS (Methode 1): Rₜ = 1.82 min.
HRMS: berechnet für C₁₈H₁₂N₆OFCl: 382.0745; gefunden: 382.0748.
¹H-NMR (DMSO-d₆, 400 MHz): δ = 6.11 (s, 1H), 6.47 (dd, 1H), 7.01 (br. s, 2H), 7.25 (d, 1H), 7.41 (dd, 1H), 7.58 (t, 1H), 7.66 (t, 1H), 8.15 (dd, 1H), 8.39 (d, 1H), 9.96 (s, 1H), 12.05 (br. s, 1H).

### Beispiel 10

### 6-Chlor-N⁴-{3-fluor-4-[(3-methyl-1H-pyrrolo[2,3-b]pyridin-4-yl)amino]phenyl}pyrimidin-2,4-diamin

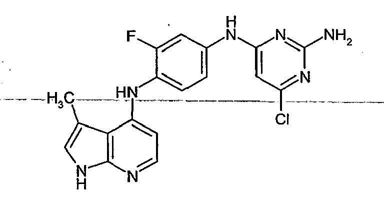

19 mg (70 µmol) 2-Fluor-*N*¹-(3-methyl-1H-pyrrolo[2,3-b]pyridin-4-yl)benzol-1,4-diamin und 17 mg (10 µmol) 4,6-Dichlorpyrimidin-2-amin werden in 3.5 ml Wasser suspendiert. Man setzt 100 µl 1M Salzsäure hinzu, und es wird über Nacht auf 100°C erhitzt. Die Suspension wird dann mit 1N Natronlauge auf pH 10 gestellt; wobei Kristalle ausfallen. Der Feststoff wird abfiltriert und mit Wasser gewaschen. Das Rohprodukt wird durch Säulenchromatographie an Kieselgel gereinigt (Eluens: Dichlormethan/Methanol (7M Ammoniak) 100:1 bis 10:1).
Ausbeute: 9 mg (32% d. Th.)
LC-MS (Methode 3): Rₜ = 1.60 min.
MS (ESI pos.): m/z = 384 (M+H)⁺.
¹H-NMR (DMSO-d₆, 300 MHz): δ = 2.45 (s, 3H), 5.98-6.08 (m, 2H), 6.83 (s, 2H), 6.95 (s, 1H), 7.23-7.36 (m, 2H), 7.40 (s, 1H), 7.78 (d, 1H), 8.00 (d, 1H), 9.51 (s, 1H), 10.99 (s, 1H).

### Beispiel 11

### N⁴-{3-Fluor-4-[(3-methyl-1H-pyrrolo[2,3-b]pyridin-4-yl)amino]phenyl}-6-(trifluormethyl)-pyrimidin-2,4-diamin

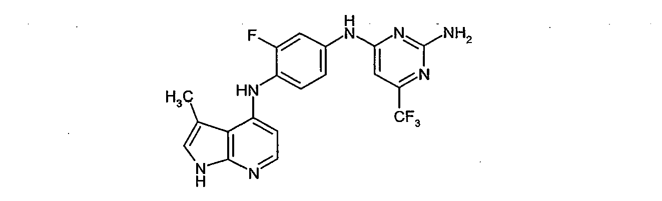

123 mg (480 µmol) 2-Fluor-*N*¹-(3-methyl-1H-pyrrolo[2,3-b]pyridin-4-yl)benzol-1,4-diamin und 123 mg (620 µmol) 4-Chlor-6-(trifluormethyl)pyrimidin-2-amin werden in 3.5 ml Wasser suspendiert. Man setzt 620 µl 1M Salzsäure hinzu, und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird durch Säulenchromatographie an Kieselgel gereinigt (Eluens: Dichlormethan/Methanol (7M Ammoniak) 100:1 bis 10:1).
Ausbeute: 154 mg (77% d. Th.)
LC-MS (Methode 3): Rₜ = 1.38 min.
MS (ESI pos.): m/z = 418 (M+H)⁺.
¹H-NMR (DMSO-d₆, 300 MHz): δ = 2.45 (s, 3H), 6.02-6.10 (m, 1H), 6.38 (s, 1H), 6.88-7.03 (m, 3H), 7.24-7.39 (m, 2H), 7.43 (s, 1H), 7.79 (d, 1H), 8.08 (d, 1H), 9.78 (s, 1H), 11.0 (s, 1H).

### Beispiel 12

### N-{3-Fluor-4-[(3-methyl-1H-pyrrolo[2,3-b]pyridin-4-yl)amino]phenyl}-2-pyridin-4-ylpyrimidin-4,6-diamin

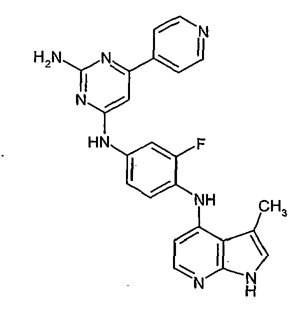

17 mg (40 µmol) 2-Fluor-*N*¹-(3-methyl-1-{[2-(trimethylsilyl)ethoxy]methyl}-1H-pyrrolo[2,3-b]pyridin-4-yl)benzol-1,4-diamin und 10 mg (50 µmol) 4-Chlor-6-pyridin-4-ylpyrimidin-2-amin werden in 1 ml Wasser suspendiert. Anschließend werden 7 µl (70 µmol) einer 37%igen Salzsäure-Lösung zugegeben und die Mischung über Nacht unter Rückfluss erhitzt. Die Suspension wird dann mit 1N Natronlauge auf pH 10 gebracht, wobei Kristalle ausfallen. Der Feststoff wird abfiltriert, mit etwas Wasser gewaschen und mittels Säulenchromatographie an Kieselgel gereinigt (Eluens: Dichlormethan/Methanol (7M Ammoniak) 100:1 bis 10:1).
Ausbeute: 3 mg (17% d. Th.)
LC-MS (Methode 7): Rₜ = 1.61 min.
MS (ESI neg.): m/z = 425 (M-H)⁻.
¹H-NMR (DMSO-d₆, 300 MHz): δ = 2.18 (s, 3H), 6.00-6.09 (m, 1H), 6.52-6.68 (m, 3H), 6.87 (s, 2H), 6.93-7.00 (m, 1H), 7.23-7.47 (m, 3H), 7.74-7.89 (m, 2H), 8.03-8.18 (m, 1H), 8.67-8.74 (m, 1H), 9.58 (s, 1H), 11.01 (s, 1H).

### Beispiel 13

### 6-Chlor-N⁴-[3,5-difluor-4-(1H-pyrrolo[2,3-b]pyridin-4-ylamino)phenyl]pyrimidin-2,4-diamin

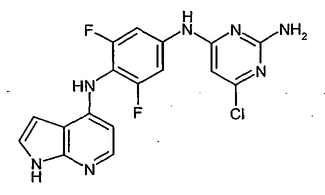

51 mg (200 µmol) 2,6-Difluor-*N*¹-1H-pyrrolo[2,3-b]pyridin-4-ylbenzol-1,4-diamin und 45 mg (270 µmol) 4,6-Dichloropyrimidin-2-amin werden in 5 ml Wasser suspendiert. Man setzt 270 µl 1M Salzsäure hinzu und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird durch Säulenchromatographie an Kieselgel gereinigt (Eluens: Dichlormethan/Methanol (7M Ammoniak) 100:1 bis 10:1).
Ausbeute: 53 mg (66% d. Th.)
LC-MS (Methode 3): Rₜ = 1.52 min.
MS (ESI pos.): m/z = 388 (M+H)⁺.
¹H-NMR (DMSO-d₆, 300 MHz): δ = 5.94 (d, 1H), 6.03 (s, 1H), 6.45-6.53 (m, 1H), 6.98 (s, 2H), 7.13-7.21 (m, 1H), 7.20-7.29 (m, 1H), 7.63 (s, 1H), 7.68 (s, 1H), 7.82 (d, 1H), 8.20 (s, 1H), 9.73 (s, 1H), 11.29 (s, 1H).

### Beispiel 14

### N⁴-{3-Fluor-4-[(1-methyl-1H-pyrrolo[2,3-b]pyridin-4-yl)amino]phenyl}-6-pyridin-4-ylpyrimidin-2,4-diamin

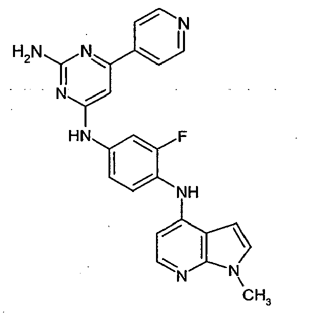

200 mg (0.78 mmol) 2-Fluor-*N*¹-(1-methyl-1H-pyrrolo[2,3-b]pyridin-4-yl)benzol-1,4-diamin und 169 mg (0.82 mmol) 4-Chlor-6-pyridin-4-ylpyrimidin-2-amin werden in 5 ml Wasser suspendiert. Anschließend werden 84 µl (1.01 mmol) einer 37%igen Salzsäure-Lösung zugegeben und die Mischung über Nacht unter Rückfluss erhitzt. Die Suspension wird dann mit 1N Natronlauge auf pH 10 gebracht, wobei Kristalle ausfallen. Der Feststoff wird abfiltriert, mit etwas Wasser gewaschen, dann in DMSO gelöst und mittels präparativer RP-HPLC gereinigt.
Ausbeute: 50 mg (15% d. Th.)
LC-MS (Methode 10): Rₜ = 2.24 min.
MS (ESI pos.): m/z = 427 (M+H)⁺.
¹H-NMR (DMSO-d₆, 200 MHz): δ = 3.74 (s, 3H), 6.16 (d, 1H), 6.48-6.80 (m, 4H), 7.17-7.51 (m, 3H), 7.78-8.02 (m, 3H), 8.18 (d, 1H), 8.48 (s, 1H), 8.72 (d, 2H), 9.68 (d, 1H).

### Beispiel 15

### 6-Chlor-N⁴-[4-(1H-pyrrolo[2,3-b]pyridin-4-ylamino)phenyl]pyrimidin-2,4-diamin

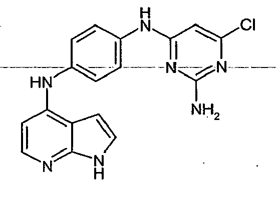

125 mg (0.56 mmol) *N*-1H-Pyrrolo[2,3-b]pyridin-4-ylbenzol-1,4-diamin und 118 mg (0.72 mmol) 4,6-Dichlorpyrimidin-2-amin werden in 5 ml Wasser suspendiert. Man setzt 0.72 ml 1M Salzsäure hinzu, und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird mittels Säulenchromatographie an Kieselgel (Eluens: Dichlormethan/Methanol (7M Ammoniak) 100:1 bis 10:1) gereinigt.
Ausbeute: 139 mg (71% d. Th.)
LC-MS (Methode 1): Rₜ = 1.54 min.
MS (ESI pos.): m/z = 352 (M+H)⁺.
¹H-NMR (DMSO-d₆, 400 MHz): δ = 5.98 (s, 1H), 6.56 (d, 1 H), 6.53-6.62 (m, 1H), 6.69 (s, 2H), 7.12-7.18 (m, 1H), 7.22 (d, 2H), 7.66 (d, 2H), 7.86 (d, 1H), 8.50 (s, 1H), 9.28 (s, 1H), 11.29 (s, 1H).

### Beispiel 16

### 6-Chlor-N⁴-{3-fluor-4-[methyl(1H-pyrrolo[2,3-b]pyridin-4-yl)amino]phenyl}pyrimidin-2,4-diamin

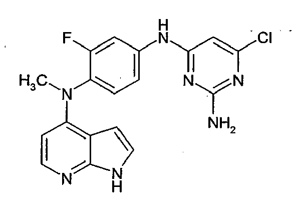

19 mg (0.08 mmol) *N*-Methyl-*N*-1H-pyrrolo[2,3-b]pyridin-4-ylbenzol-1,4-diamin und 17 mg (0.11 mmol) 4,6-Dichlorpyrimidin-2-amin werden in 4 ml Wasser suspendiert. Man setzt 0.11 ml 1M Salzsäure hinzu, und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird mittels Säulenchromatographie an Kieselgel (Eluens: Dichlormethan/Methanol (7M Ammoniak) 100:1 bis 10:1) gereinigt.
Ausbeute: 24 mg (83% d. Th.)
LC-MS (Methode 2): Rₜ = 1.50 min.
MS (ESI pos.): m/z = 384 (M+H)⁺.
¹H-NMR (DMSO-d₆, 400 MHz): δ = 3.34 (s, 3H), 5.02-5.10 (m, 1H), 6.05 (s, 1H), 6.39 (d, 1H), 6.89 (s, 2H), 6.90-6.98 (m, 1H), 7.22-7.38 (m, 2H), 7.95 (d, 1H), 8.07 (dd, 1H), 9.63 (s, 1H), 11.24 (s, 1H).

### Beispiel 17

### 6-Chlor-N⁴-[3-chlor-4-(1H-pyrrolo[2,3-b]pyridin-4-ylamino)phenyl]pyrimidin-2,4-diamin

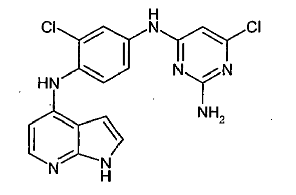

27 mg (0.10 mmol) 2-Chlor-*N*¹-1H-pyrrolo[2,3-b]pyridin-4-ylbenzol-1,4-diamin und 24 mg (0.15 mmol) 4,6-Dichlorpyrimidin-2-amin werden in 5 ml Wasser suspendiert. Man setzt 0.14 ml 1M Salzsäure hinzu, und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird durch präparative HPLC gereinigt.
Ausbeute: 24 mg (57% d. Th.)
LC-MS (Methode 2): Rₜ = 1.54 min.
MS (ESI pos.): m/z = 386 (M+H)⁺.
¹H-NMR (DMSO-d₆, 400 MHz): δ = 6.02 (s, 1H), 6.05 (d, 1 H), 6.41-6.49 (m, 1H), 6.83 (s, 2H), 7.09-7.18 (m, 1H), 7.31 (d, 1H), 7.64 (dd, 1H), 7.80 (d, 1H), 8.02 (d, 1H), 8.26 (s, 1H), 9.51 (s, 1H), 11.25 (s, 1H).

### Beispiel 18

### 6-Chlor-N⁴-{3,5-difluor-4-[(3-methyl-1H-pyrrolo[2,3-b]pyridin-4-yl)amino]phenyl}pyrimidin-2,4-diamin

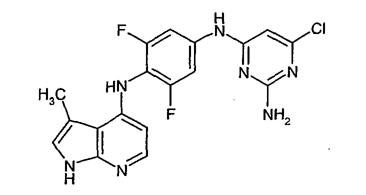

27 mg (0.10 mmol) 2,6-Difluor-*N*¹-(3-methyl-1H-pyrrolo[2,3-b]pyridin-4-yl)benzol-1,4-diamin und 23 mg (0.14 mmol) 4,6-Dichlorpyrimidin-2-amin werden in 5 ml Wasser suspendiert. Man setzt 0.14 ml 1M Salzsäure hinzu, und es wird über Nacht auf 100°C erhitzt. Die Suspension wird dann mit 1N Natronlauge auf pH 10 gestellt, wobei Kristalle ausfallen. Der Feststoff wird abfiltriert und mit Wasser gewaschen. Das Rohprodukt wird durch präparative HPLC gereinigt.
Ausbeute: 24 mg (61% d. Th.)
LC-MS (Methode 3): Rₜ = 1.29 min.
MS (ESI pos.): m/z = 402 (M+H)⁺.
¹H-NMR (DMSO-d₆, 400 MHz): δ = 2.48 (s, 3H), 5.78 (d, 1H), 6.04 (s, 1H), 6.94 (bs. 1H), 6.98 (bs., 2H), 7.19 (s, 1H), 7.59-7.68 (m, 2H), 7.74 (d, 1H), 9.73 (s, 1H), 11.01 (s, 1H).

### Beispiel 19

### N⁴-{4-[(3-Chlor-1H-pyrrolo[2,3-b]pyridin-4-yl)methyl]-3-fluorphenyl}-6-(trifluormethyl)-pyrimidin-2,4-diamin

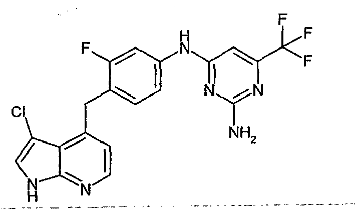

185 mg (0.60 mmol) 4-[(3-Chlor-1H-pyrrolo[2,3-b]pyridin-4-yl)methyl]-3-fluoranilin und 130 mg (0.66 mmol) 4-Chlor-6-(trifluormethyl)pyrimidin-2-amin werden in 4 ml Wasser und 0.30 ml 4N Salzsäure suspendiert. Man erhitzt die Reaktionsmischung 2 Stunden auf Rückfluss. Nach Abkühlen stellt man mit verdünnter Natronlauge alkalisch, setzt etwas Methanol hinzu und extrahiert mit Essigsäureethylester. Man trocknet über Natriumsulfat und entfernt das Lösungsmittel im Vakuum. Der Rückstand wird mittel präparativer HPLC aufgereinigt. Man erhält Kristalle.
Ausbeute: 123 mg (45% d. Th.)
LC-MS (Methode 1): Rₜ = 2.32 min.
MS (ESI pos.): m/z = 437 [M+H]⁺.
¹H-NMR (DMSO-d₆, 400 MHz): δ = 4.47 (s, 2H), 6.35 (s, 1H), 6.76 (d, 1H), 6.94 (br. s, 2H), 7.03 (t, 1H), 7.29 (dd, 1H), 7.64 (s, 1H), 7.93 (dd, 1H), 8.18 (d, 1H), 9.75 (s, 1H), 12.00 (s, 1H).

### Beispiel 20

### 4-{4-[(2-Aminopyrimidin-4-yl)amino]-2-fluorbenzyl}-1H-pyrrolo[2,3-b]pyridin-3-carbonitril

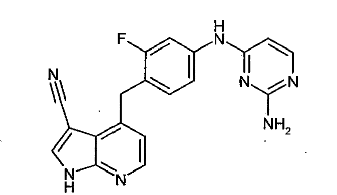

120 mg (0.45 mmol) 4-(4-Amino-2-fluorbenzyl)-1H-pyrrolo[2,3-b]pyridin-3-carbonitril und 64 mg (0.50 mmol) 4-Chlor-pyrimidin-2-amin werden in 4 ml Wasser, 2 ml Ethanol und 0.23 ml 4N Salzsäure suspendiert. Man erhitzt die Reaktionsmischung 2 Stunden auf Rückfluss. Nach Abkühlen stellt man mit verdünnter Natronlauge alkalisch, setzt etwas Methanol hinzu, extrahiert mit Essigsäureethylester und entfernt das Lösungsmittel im Vakuum. Der Rückstand wird mittels präparativer HPLC aufgereinigt. Man erhält Kristalle.
Ausbeute: 111 mg (65% d. Th.)
LC-MS (Methode 3): Rₜ = 1.53 min.
MS (ESI pos.): m/z = 360 [M+H]⁺.
¹H-NMR (DMSO-d₆, 400 MHz): δ = 4.39 (s, 2H), 5.99 (d, 1H), 6.29 (br. s, 2H), 6.90 (d, 1H), 7.09 (t, 1H), 7.31 (dd, 1H), 7.82 (d, 1H), 7.91 (dd, 1H), 8.29 (d, 1H), 8.45 (s, 1H), 9.29 (s, 1H), 12.84 (br. s, 1H).

### Beispiel 21

### 4-(4-{[2-Amino-6-(trifluormethyl)pyrimidin-4-yl]amino}-2-fluorbenzyl)-1H-pyrrolo[2,3-b]pyridin-3-carbonitril

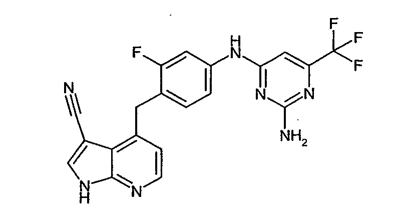

82 mg (0.31 mmol) 4-(4-Amino-2-fluorbenzyl)-1H-pyrrolo[2,3-b]pyridin-3-carbonitril und 67 mg (0.34 mmol) 4-Chlor-6-trifluormethylpyrimidin-2-amin werden in 3 ml Wasser, 1.5 ml Ethanol und 0.15 ml 4N Salzsäure suspendiert. Man erhitzt die Reaktionsmischung 2 Stunden auf Rückfluss. Nach Abkühlen stellt man mit verdünnter Natronlauge alkalisch, setzt etwas Methanol hinzu, extrahiert mit Essigsäureethylester und entfernt das Lösungsmittel im Vakuum. Der Rückstand wird mittels präparativer HPLC aufgereinigt. Man erhält Kristalle.
Ausbeute: 111 mg (65% d. Th.)
LC-MS (Methode 3): Rₜ = 2.34 min.
MS (ESI pos.): m/z = 428 [M+H]⁺.
¹H-NMR (DMSO-d₆, 300 MHz): δ = 4.41 (s, 2H), 6.35 (s, 1H), 6.90 (d, 1H), 6.96 (br. s, 2H), 7.14 (t, 1H), 7.32 (dd, 1H), 7.93 (dd, 1H), 8.29 (d, 1H), 8.46 (s, 1H), 9.77 (s, 1H), 12.86 (br. s, 1H).

### Beispiel 22

### 6-Chlor-N⁴-{3-fluor-4-[(3-fluor-1H-pyrrolo[2,3-b]pyridin-4-yl)amino]phenyl}pyrimidin-2,4-diamin

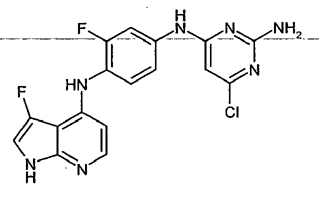

17.9 mg (0.07 mmol) 2-Fluor-N¹-(3-fluor-1H-pyrrolo[2,3-b]pyridin-4-yl)benzen-1,4-diamin und 12.4 mg (0.076 mmol) 4,6-Dichlorpyrimidin-2-amin werden in 2 ml Wasser suspendiert. Man setzt 83 µl 1M Salzsäure hinzu, und es wird 3 h auf 100°C erhitzt. Die Suspension wird dann mit 1N Natronlauge auf pH 10 gestellt. Es wird Dimethylsulfoxid zugegeben bis sich der Niederschlag löst, und die Lösung wird mittels präparativer HPLC gereinigt.
Ausbeute: 10.4 mg (39% d. Th.)
LC-MS (Methode 2): Rₜ = 1.55 min.
MS (ESI pos.): m/z = 388 (M+H)⁺.
1H-NMR (DMSO-d₆, 400 MHz): δ = 6.03 (s, 1H), 6.41 (d, 1H), 6.82 (s, 2H), 7.30 (dd, 1H), 7.41 (t, 1H), 7.77 (d, 1H), 7.92 (d, 1H), 8.01 (dd, 1H), 9.51 (s, 1H), 9.89 (s, 1H), 12.30 (br.s, 1H).

### Beispiel 23

### 6-Chlor-N⁴-(3-fluor-4-{[3-(trifluormethyl)-1H-pyrrolo[2,3-b]pyridin-4-yl]amino}phenyl)-pyrimidin-2,4-diamin

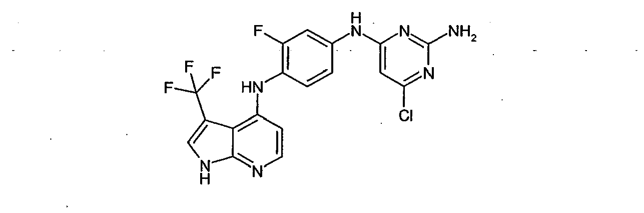

18 mg (0.058 mmol) 2-Fluor-N¹-[3-(trifluormethyl)-1H-pyrrolo[2,3-b]pyridin-4-yl]benzen-1,4-diamin und 10.4 mg (0.064 mmol) 4,6-Dichlorpyrimidin-2-amin werden in 2 ml Wasser suspendiert. Man setzt 70 µl 1M Salzsäure hinzu, und es wird 3 h auf 100°C erhitzt. Die Suspension wird dann mit 1N Natronlauge auf pH 10 gestellt. Es wird Dimethylsulfoxid zugegeben bis sich der Niederschlag löst, und die Lösung wird mittels präparativer HPLC gereinigt.
Ausbeute: 14 mg (55% d. Th.)
LC-MS (Methode 3): Rₜ = 2.01 min.
MS (ESI pos.): m/z = 438 (M+H)⁺.
¹H-NMR (DMSO-d₆, 400 MHz): δ = 6.02 (s, 1H), 6.45 (d, 1H), 6.82 (s, 2H), 7.30 (dd, 1H), 7.42 (t, 1H), 7.94 (d, 1H), 7.97 (s, 1H), 8.03 (dd, 1H), 9.56 (s, 1H), 9.93 (s, 1H), 12.33 (br.s, 1H).

### B. Bewertung der physiologischen Wirksamkeit

In einem in vitro-Assay mit rekombinanter Rho-Kinase-II wird die Hemmung des Enzyms untersucht. Die gefäßrelaxierende Wirkung wird an Phenylephrin-induzierten Kontraktionen isolierter Ringe der Kaninchen-Arteria-Saphena bestimmt. Die Eignung der erfindungsgemäßen Verbindungen zur Behandlung von kardiovaskulären Erkrankungen kann durch Untersuchung der blutdrucksenkenden Wirkung an narkotisierten Ratten gezeigt werden.

### Hemmung der rekombinanten Rho-Kinase II (ROKα)

Die Aktivität der Rho-Kinase wird durch den Einbau von ³³P₋Phosphat in ein Substratpeptid bestimmt. Dazu wird kommerziell erhältliche Rho-Ihinase II (Upstate Biotechnology) in Gegenwart des S6 Phosphate-Acceptor-Peptides mit den Testsubstanzen oder einer Lösungsmittelkontrolle 10 min bei 37°C vorinkubiert. Anschließend wird die Kinase-Reaktion durch Zugabe von ³³P-markiertem ATP gestartet. Nach 20 min bei 37°C wird die Reaktion durch Zugabe von H₃PO₄ gestoppt. Aliquots werden auf Filter pipettiert, die Filter gewaschen und anschließend mit Scintillator beschichtet. Die Radioaktivität der am Filter gebundenen ³³P-markierten Peptide wird in einem Micro-Beta-Counter gemessen. Der IC₅₀-Wert entspricht der Konzentration einer Testsubstanz bei welcher der Rho-Kinase katalysierte Einbau von ³³P in das Peptid im Vergleich zu einer Lösungsmittelkontrolle um 50% gehemmt ist. Die experimentellen Daten sind in der folgenden Tabelle A zusammengestellt.

**Tabelle A:**

| **Beispiel-Nr.** | **IC₅₀ (nM)** |
|---|---|
| **8** | 7 |
| **10** | 3 |
| **11** | 4 |

### Gefäßrelaxierende Wirkung in vitro

3 mm breite Ringe der isolierten Kaninchen-Arteria-Saphena werden einzeln in 5 ml-Organbäder mit 37°C warmer, carbogenbegaster Krebs-Henseleit-Lösung gebracht. Der Gefäßtonus wird isometrisch erfasst und registriert. Kontraktionen werden durch Zugabe von 3x10⁻⁸ g/ml Phenylephrin induziert und nach 4 min wieder ausgewaschen. Nach mehreren Kontrollzyklen wird die zu untersuchende Substanz mit jedem weiteren Durchgang in steigender Dosierung vorinkubiert und die darauffolgende Kontraktion mit der Höhe der letzten Kontrollkontraktion verglichen. Es wird die Konzentration errechnet, die erforderlich ist, um die Höhe des Kontrollwertes um 50% zu reduzieren (IC₅₀). Die experimentellen Daten sind in der folgenden Tabelle B zusammengestellt.

**Tabelle B:**

| **Beispiel-Nr.** | **IC₅₀ (nM)** |
|---|---|
| **8** | 86 |
| **10** | 8 |
| **11** | 3 |

### Blutdruckmessung an narkotisierten Ratten

Männliche Wistar-Ratten mit einem Körpergewicht von 300 - 350 g werden mit Thiopental (100 mg/kg i.p.) anästhesiert. Nach der Tracheotomie wird in die Femoralarterie ein Katheter zur Blutdruckmessung eingeführt. Die zu prüfenden Substanzen werden als Lösungen entweder oral mittels Schlundsonde oder über die Femoralvene intravenös verabreicht.

### C. Ausführunesbeispiele für pharmazeutische Zusammensetzungen

Die erfindungsgemäßen Verbindungen können folgendermaßen in pharmazeutische Zubereitungen überführt werden:

### Tablette:

### Zusammensetzung:

100 mg der Verbindung von Beispiel 1, 50 mg Lactose (Monohydrat), 50 mg Maisstärke (nativ), 10 mg Polyvinylpyrrolidon (PVP 25) (Fa. BASF, Ludwigshafen, Deutschland) und 2 mg Magnesiumstearat.

Tablettengewicht 212 mg. Durchmesser 8 mm, Wölbungsradius 12 mm.

### Herstellung:

Die Mischung aus erfindungsgemäßer Verbindung, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

### Zusammensetzung:

1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

### Herstellung:

Das Rhodigel wird in Ethanol suspendiert, die erfindungsgemäßer Verbindung wird der Suspension zugefügt. Unter Rühren erfolgt die Zugabe des Wassers. Bis zum Abschluss der Quellung des Rhodigels wird ca. 6 Stunden gerührt.

## Patentansprüche

1. Verbindung der Formel (I) 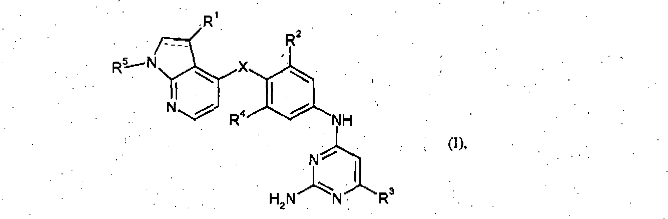 worin
----- eine Einfach- oder eine Doppelbindung bedeutet,
X -NR⁶-, -CR⁷R⁸- oder -C(=O)- bedeutet,
worin
R⁶ für Wasserstoff oder (C₁-C₃)-Alkyl steht,
R⁷ und R⁸ unabhängig voneinander für Wasserstoff oder Methyl stehen,
R¹ Wasserstoff, Halogen, Cyano, (C₁-C₆)-Alkyl, (C₃-C₆)-Cycloalkyl, (C₁-C₆)-Alkoxycarbonyl oder (C₁-C₆)-Alkylaminocarbonyl bedeutet,
wobei Alkyl, Alkoxycarbonyl und Alkylaminocarbonyl ihrerseits durch Hydroxy, Halogen, Hydroxycarbonyl, (C₁-C₆)-Alkoxy, (C₁-C₆)-Alkoxycarbonyl, Amino, Aminocarbonyl, (C₁-C₆)-Alkylamino oder (C₁-C₆)-Alkylaminocarbonyl substituiert sein können,
R² Fluor oder Chlor bedeutet,
R³ einen Rest bedeutet, der ausgewählt ist aus der Gruppe von:
• Wasserstoff, Hydroxy, Halogen, Trifluormethyl, Pentafluorethyl,
• (C₁-C₆)-Alkyl, (C₁-C₆)-Alkoxy, (C₃-C₈)-Cycloalkyl,
wobei Alkyl, Alkoxy und Cycloalkyl ihrerseits durch Hydroxy, Hydroxycarbenyl, (C₁-C₆)-Alkoxy, (C₁-C₆)-Alkoxycarbonyl, (C₆-C₁₀)-Aryl, -NR⁹R¹⁰ oder -C(=O)NR⁹R¹⁰ substituiert sein können,
worin
R⁹ und R¹⁰ unabhängig voneinander für Wasserstoff, (C₁-C₈)-Alkyl, gegebenenfalls mit (C₁-C₆)-Alkyl substituiertes (C₃-C₆)-Cycloalkyl, gegebenenfalls mit Halogen substituiertes (C₆-C₁₀)-Aryl oder 5- bis 10-gliedriges Heteroaryl stehen
oder
R⁹ und R¹⁰ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,
• (C₆-C₁₀)-Aryl, (C₆-C₁₀)-Aryloxy, 5- bis 10-gliedriges Heteroaryl, 5- bis 10-gliedriges Heteroaryloxy, über ein Kohlenstoffatom gebundenes 5- bis 10-gliedriges Heterocyclyl,
wobei Aryl, Aryloxy, Heteroaryl, Heteroaryloxy und Heterocyclyl ihrerseits durch Halogen, Cyano, Nitro, Hydroxycarbonyl, Amino, Trifluormethyl, gegebenenfalls mit Hydroxy substituiertes (C₁-C₆)-Alkyl, (C₁-C₆)-Alkoxy, (C₁-C₆)-Alkylamino, (C₁-C₆)-Alkanoyl, (C₁-C₆)-Alkoxy, carbonyl, (C₁-C₆)-Alkanoylamino, (C₁-C₆)-Alkoxycarbonylamino oder 5- oder 6-gliedriges Heterocyclyl substituiert sein können,
• -NR¹¹R¹²,
worin
R¹¹ und R¹² unabhängig voneinander für-Wasserstoff, (C₁-C₆)-Alkyl, -(C₃-C₈) Cycloalkyl, (C₆-C₁₀)-Aryl oder 5-bis 10-gliidriges Heteroaryl stehen,
wobei Alkyl und Cycloalkyl ihrerseits durch Hydroxy, (C₁-C₆)-Alkoxy, (C₆-C₁₀)-Aryl, 5- bis 10-gliedriges Heteroaryl oder -NR¹³R¹⁴ substituiert sein können,
worin
R¹³ und R¹⁴ unabhängig voneinander für Wasserstoff, (C₁-C₆)-Alkyl (C₃- C₆)-Cycloalkyl, (C₆-C₁₀)-Aryl oder 5- oder 6-gliedriges Heteroaryl stehen
oder
R¹³ und R¹⁴ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,
und
wobei Aryl und Heteroaryl ihrerseits durch Halogen, Hydroxy, Amino, Cyano, Trifluormethyl, (C₁-C₆)-Alkyl, (C₁-C₆)-Alkoxy, (C₁-C₆)-Alkylamino oder (C₁-C₆)-Alkanoylamino substituiert sein können,
oder
R¹¹ und R¹² gemeinsam mit dem Stickstoffatom, an das sie gebunden sind, einen 4- bis 6-gliedrigen Heterocyclus bilden, der ein weiteres Heteroatom O oder N im Ring enthalten kann und der durch Fluor, Hydroxy, Hydroxycarbonyl, 5- bis 7-gliedriges Heterocyclyl, das ein oder zwei weitere Heteroatome N und/oder O im Ring enthalten kann, das seinerseits durch (C₁-C₄)-Alkyl oder (C₁-C₄)-Alkoxycarbonyl substituiert sein kann, (C₁-C₄)-Alkoxy, (C₁-C₄)-Alkyl, (C₁-C₄)-Alkanoyl, (C₁-C₄)-Alkoxycarbonyl oder -NR¹⁵R¹⁶ substituiert sein kann,
wobei Alkyl seinerseits durch Hydroxy, (C₁-C₄)-Alkoxy oder -NR¹⁷R¹⁸ substituiert sein kann,
worin
R¹⁷ und R¹⁸ unabhängig voneinander für Wasserstoff, gegebenen falls mit Hydroxy substituiertes (C₁-C₆)-Alkyl, (C₃-C₆)-Cycloalkyl, (C₆-C₁₀)-Aryl oder 5- oder 6-gliedriges Heteroaryl stehen
oder
R¹⁷ und R¹⁸ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,
worin
R¹⁵ und R¹⁶ unabhängig voneinander Wasserstoff, (C₁-C₆)-Alkyl, (C₁-C₄)- Alkoxycarbonyl, (C₃-C₈)-Cycloalkyl oder (C₁-C₄)-Alkanoyl bedeuten
oder
R¹⁵ und R¹⁶ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,
oder
R¹¹ und R¹² gemeinsam mit dem Stickstoffatom, an das sie gebunden sind, einen 7- bis 12-gliedrigen bicyclischen oder tricyclischen Heterocyclus bilden, der anneliert oder spirocyclisch ist und ein oder zwei weitere Heteroatome aus der Reihe N und/oder O im Ring aufweisen kann und der durch Fluor, (C₁-C₄)-Alkyl, (C₁-C₄)-Alkoxycarbonyl, (C₁-C₄)-Alkanoyl oder Benzyl substituiert sein kann,
• und -C(=O)R¹⁹,
worin
R¹⁹ für (C₁-C₆)-Alkoxy, (C₁-C₆)-Alkylamino oder einen über ein Stickstoffatom gebundenen 5- bis 10-gliedrigen mono- oder bicyclischen Heterocyclus, der anneliert oder spiroeyclisch ist und ein oder zwei weitere Heteroatome aus der Reihe N und/oder O im Ring aufweisen kann, steht,
wobei Alkylamino seinerseits mit einem 5- oder 6-gliedrigen Heterocyclyl substituiert sein kann,
R⁴ Wasserstoff, Fluor oder Chlor bedeutet,
R⁵ Wasserstoff oder (C₁-C₆)-Alkyl bedeutet
oder eines ihrer Salze, ihrer Solvate oder der Solvate ihrer Salze.

2. Verbindung der Formel (I) nach Anspruch 1,
worin
------ eine Doppelbindung bedeutet,
X -NR⁶-, -CH₂- oder -C(=O)- bedeutet,
worin
R⁶ für Wasserstoff oder Methyl steht,
R¹ Wasserstoff, Fluor, Chlor, Cyano, Trifluormethyl, Difluormethyl, Methyl, Ethyl, hydroxyethyl, Methoxyethyl, Cyclopropyl, (C₁-C₆)-Alkoxycarbonyl oder (C₁-C₆)Alkylaminocarbonyl bedeutet,
wobei Alkoxycarbonyl und Alkylaminocarbonyl ihrerseits durch Hydroxy, Hydroxycarbonyl, (C₁-C₆)-Alkoxy, (C₁-C₆)-Alkoxycarbonyl, Amino, Aminocarbonyl, (C₁-C₆)-Alkylamino oder (C₁-C₆)-Alkylaminocarbonyl substituiert sein können,
R² Fluor oder Chlor bedeutet,
R³ einen Rest bedeutet, der ausgewählt ist aus der Gruppe von.
• Wasserstoff, Hydroxy, Halogen, Trifluomethyl, Pentafluorethyl,
• (C₁-C₆)-Alkyl, (C₁-C₆)-Alkoxy, (C₃-C₈)-Cycloalkyl,
wobei Alkyl, Alkoxy und Cycloalkyl ihrerseits durch Hydroxy, Hydroxycarbonyl, (C₁-C₆)-Alkoxy, (C₁-C₆)-Alkoxycarbonyl, (C₆-C₁₀)-Aryl, -NR⁹R¹⁰ oder -C(=O)NR⁹R¹⁰ substituiert sein können,
worin
R⁹ und R¹⁰ unabhängig voneinander für Wasserstoff oder (C₁-C₈)-Alkyl stehen
oder
R⁹ und R¹⁰ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₆)-Alkyl, (C₁-C₆)-Alkanoyl oder (C₁-C₆)-Alkoxycarbonyl substituiert sein kann,
• Phenyl, 5- oder 6-gliedriges Heteroaryl, über ein Kohlenstoffatom gebundenes 5- bis 10-gliedriges Heterocyclyl,
wobei Phenyl, Heteroaryl und Heterocyclyl ihrerseits durch Halogen, Cyano, Nitro, Hydroxycarbonyl, Amino, Trifluormethyl, gegebenenfalls mit Hydroxy substituiertes (C₁-C₆)-Alkyl, (C₁-C₆)-Alkoxy, (C₁-C₆)-Alkylamino, (C₁-C₆)-Alkanoyl, (C₁-C₆)-Alkoxycarbonyl, (C₁-C₆)-Alkanoylamino oder (C₁-C₆)-Alkoxycarbonylamino substituiert sein können,
• -NR¹¹R¹²,
worin
R¹¹ und R¹² unabhängig voneinander für Wasserstoff, (C₁-C₆)-Alkyl, (C₃-C₈)-Cycloalkyl, (C₆-C₁₀)-Aryl oder 5- bis 10-gliedriges Heteroaryl stehen,
wobei Alkyl und Cycloalkyl ihrerseits durch Hydroxy, (C₁-C₆)-Alkoxy, (C₆-C₁₀)-Aryl oder 5- bis 10-gliedriges Heteroaryl substituiert sein können,
und
wobei Aryl und Heteroaryl ihrerseits durch Halogen, Hydroxy, Amino, Cyano, Trifluormethyl, (C₁-C₆)-Alkyl, (C₁-C₆)-Alkoxy, (C₁-C₆)-Alkylamino oder (C₁-C₆)-Alkanoylamino substituiert sein können,
oder
R¹¹ und R¹² gemeinsam mit dem Stickstoffatom, an das sie gebunden sind, einen 4-bis 6-gliedrigen Heterocyclus bilden, der ein weiteres Heteroatom O oder N im Ring enthalten kann und der durch Fluor, Hydroxy, Hydroxycarbonyl, (C₁-C₄)-Alkoxy, (C₁-C₄)-Alkyl, (C₁-C₄)-Alkanoyl oder (C₁-C₄)-Alkoxycarbonyl substituiert sein kann,
• und -C(=O)R¹⁹,
worin
R¹⁹ für (C₁-C₆)-Alkoxy, (C₁-C₆)-Alkylamino oder einen über ein Stickstoffatom gebundenen 5- bis 10-gliedrigcn mono- oder bicyclischen Heterocyclus, der anneliert oder spirocyclisch ist und ein oder zwei weitere Heteroatome aus der Reihe N und/oder O im Ring aufweisen kann, steht,
wobei Alkylamino seinerseits mit einem 5- oder 6-gliedrigen Heterocyclyl substituiert sein kann,
R⁴ Wasserstoff, Fluor oder Chlor bedeutet,
R⁵ Wasserstoff oder Methyl bedeutet
oder eines ihrer Salze, ihrer Solvate oder der Solvate ihrer Salze.

3. Verbindung der Formel (1) nach Anspruch 1 oder 2,
worin
----- eine Doppelbindung bedeutet,
X X -NR⁶-, -CH₂- oder -C(=O)- bedeutet,
worin
R⁶ für Wasserstoff oder Methyl steht,
R¹ Wasserstoff, Fluor, Chlor, Cyano, Trifluormethyl. Ditluormethyl, Methyl, Ethyl, Hydroxyethyl, Methoxyethyl oder Cyclopropyl bedeutet,
R² Fluor oder Chlor bedeutet,
R³ einen Rest bedeutet, der ausgewählt ist aus der Gruppe von:
• Wasserstoff, Hydroxy, Halogen, Trifluormethyl, Pentafluorethyl,
• (C₁-C₆)-Alkyl, (C₁-C₆)-Alkoxy, (C₃-C₈)-Cycloalkyl,
wobei Alkyl, Alkoxy und Cycloalkyl ihrerseits durch Hydroxy, (C₁-C₆)-Alkoxy oder -NR⁹R¹⁰ substituiert sein können,
worin
R⁹ und R¹⁰ unabhängig voneinander für Wasserstoff oder (C₁-C₈)-Alkyl stehen
oder
R⁹ und R¹⁰ gemeinsam mit dem Stickstoffatom, an das sie gebunden sind, einen 5- oder 6-gliedrigen Heterocyclus bilden, der ein weiteres Heteroatom O oder N im Ring enthalten kann und der durch (C₁-C₃)-Alkyl oder(C₁-C₃)-Alkanoyl substituiert sein kann,
• Phenyl und 5- oder 6-gliedriges Heteroaryl,
wobei Phenyl und Heteroaryl ihrerseits durch Halogen, Amino oder Trifluormethyl substituiert sein können,
R⁴ Wasserstoff, Fluor oder Chlor bedeutet,
R⁵ Wasserstoff oder Methyl bedeutet
oder eines ihrer Salze, ihrer Solvate oder der Solvate ihrer Salze.

4. Verbindung der Formel (1) nach einem der Ansprüche 1 bis 3,
worin
eine Doppelbindung bedeutet,
X -NH- oder -CH₂- bedeutet,
R¹ Wasserstoff, Chlor oder Methyl bedeutet,
R² Fluorbedeutet,
R³ einen Rest bedeutet, der ausgewählt ist aus der Gruppe von Wasserstoff, Halogen, Trifluormethyl, gegebenenfalls durch Halogen substituiertes Phenyl und Pyridin,
R⁴ Wasserstoff oder Fluor bedeutet,
R⁵ Wasserstoff bedeutet
oder eines ihrer Satze, ihrer Solvate oder der Solvate ihrer Salze.

5. Verfahren zur Herstellung einer Verbindung der Formel (1) wie in Anspruch 1 definiert, **dadurch gekennzeichnet, dass** man
Verbindungen der Formel (II) 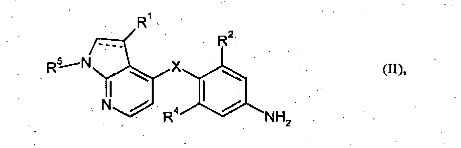 worin
-----, X, R', R², R⁴ und R⁵ die in Anspruch 1 angegebene Bedeutung aufweisen,
mit Verbindungen der Formel (III) 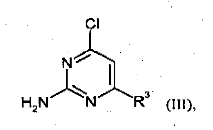 worin
R³ die in Anspruch 1 angegebene Bedeutung aufweist,
umsetzt,
wobei sich für den Fall, dass X für eine CH₂-Gruppe steht, eine Oxidation zu der entsprechenden Ketogruppe -C(=O)- oder eine Methylierung zu der entsprechenden Mono- bzw. Dimethylverbindung [-CH(CH₃)- bzw. -C(CH3)₂-] anschließen kann.

6. Verbindung der Formel (I), wie in einem der Ansprüche 1 bis 4 definiert, zur Behandlung und/oder Prophylaxe von Erkrankungen.

7. Verwendung einer Verbindung der Formel (I), wie in einem der Ansprüche 1 bis 4 definiert, zur Herstellung von Arzneimitteln zur Behandlung und/oder Prophylaxe von kardiovaskulären Erkrankungen.

8. Verwendung einer Verbindung der Formel (I), wie in einem der Ansprüche 1 bis 4 definiert, zur Herstellung von Arzneimitteln zur Behandlung und/oder Prophylaxe von erektiler Dysfunktion.

9. Arzneimittel enthaltend eine Verbindung der Formel (I), wie in einem der Ansprüche 1 bis 4 definiert, in Kombination mit einem weiteren Wirkstoff.

10. Arzneimittel enthaltend eine Verbindung der Formel (I), wie in einem der Ansprüche 1 bis 4 definiert, in Kombination mit einem inerten nichttoxischen, pharmazeutisch geeigneten Hilfsstoff.

11. Arzneimittel nach Anspruch 9 oder 10 zur Behandlung und/oder Prophylaxe von kardiovaskulären Erkrankungen.

12. Arzneimittel nach Anspruch 9 oder 10 zur Behandlung und/oder Prophylaxe von erektiler Dysfunktion.

## Claims

1. Compound of the formula (I) 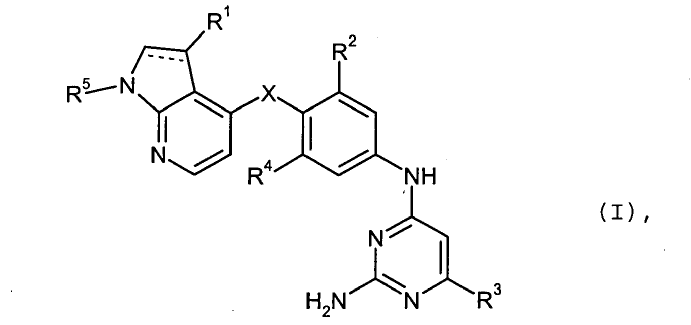 in which
----- represents a single bond or a double bond,
X represents -NR⁶-, -CR⁷R⁸- or -C(=O)-,
in which
R⁶ represents hydrogen or (C₁-C₃)-alkyl,
R⁷ and R⁸ independently of one another represent hydrogen or methyl,
R¹ represents hydrogen, halogen, cyano, (C₁-C₆)-alkyl, (C₃-C₆)-cycloalkyl, (C₁-C₆)-alkoxycarbonyl or (C₁-C₆)-alkylaminocarbonyl,
where alkyl, alkoxycarbonyl and alkylaminocarbonyl for their part may be substituted by hydroxyl, halogen, hydroxycarbonyl, (C₁-C₆)-alkoxy, (C₁-C₆)-alkoxycarbonyl, amino, aminocarbonyl, (C₁-C₆) -alkylamino or (C₁-C₆)-alkylaminocarbonyl,
R² represents fluorine or chlorine,
R³ represents a radical selected from the group consisting of:
• hydrogen, hydroxyl, halogen, trifluoromethyl, pentafluoroethyl,
• (C₁-C₆)-alkyl, (C₁-C₆)-alkoxy, (C₃-C₈)-cycloalkyl,
• where alkyl, alkoxy and cycloalkyl for their part may be substituted by hydroxyl, hydroxycarbonyl, (C₁-C₆)-alkoxy, (C₁-C₆)-alkoxycarbonyl, (C₆-C₁₀)-aryl, -NR⁹R¹⁰ or -C(=O)NR⁹R¹⁰,
in which
R⁹ and R¹⁰ independently of one another represent hydrogen, (C₁-C₈)-alkyl, optionally (C₁-C₆)-alkyl-substituted (C₃-C₆)-cycloalkyl, optionally halogen-substituted (C₆-C₁₀)-aryl or 5- to 10- membered heteroaryl
or
R⁹ and R¹⁰ together with the nitrogen atom to which they are attached form a 5- or 6-membered heterocycle which may contain a further heteroatom O or N in the ring and which may be substituted by (C₁-C₆)-alkyl, (C₁-C₆)-alkanoyl or (C₁-C₆)-alkoxycarbonyl,
• (C₆-C₁₀)-aryl, (C₆-C₁₀)-aryloxy, 5- to 10- membered heteroaryl, 5- to 10-membered heteroaryloxy, 5- to 10-membered heterocyclyl which is attached via a carbon atom,
where aryl, aryloxy, heteroaryl, heteroaryloxy and heterocyclyl for their part may be substituted by halogen, cyano, nitro, hydroxycarbonyl, amino, trifluoromethyl, optionally hydroxyl-substituted (C₁-C₆)-alkyl, (C₁-C₆)-alkoxy, (C₁-C₆) -alkylamino, (C₁-C₆) -alkanoyl, (C₁-C₆)-alkoxycarbonyl, (C₁-C₆)-alkanoylamino, (C₁-C₆)-alkoxycarbonylamino or 5- or 6-membered heterocyclyl,
• -NR¹¹R¹²,
in which
R¹¹ and R¹² independently of one another represent hydrogen, (C₁-C₆)-alkyl, (C₃-C₈)-cycloalkyl, (C₆-C₁₀)-aryl or 5- to 10- membered heteroaryl,
where alkyl and cycloalkyl for their part may be substituted by hydroxyl, (C₁-C₆)-alkoxy, (C₆-C₁₀)-aryl, 5- to 10- membered heteroaryl or -NR¹³R¹⁴,
in which
R¹³ and R¹⁴ independently of one another represent hydrogen, (C₁-C₆)-alkyl, (C₃-C₆)-cycloalkyl, (C₆-C₁₀)-aryl or 5- or 6-membered heteroaryl
or
R¹³ and R¹⁴ together with the nitrogen atom to which they are attached form a 5- or 6-membered heterocycle which may contain a further heteroatom O or N in the ring and which may be substituted by (C₁-C₆)-alkyl, (C₁-C₆)-alkanoyl or (C₁-C₆)-alkoxycarbonyl,
and
where aryl and heteroaryl for their part may be substituted by halogen, hydroxyl, amino, cyano, trifluoromethyl, (C₁-C₆)-alkyl, (C₁-C₆)-alkoxy, (C₁-C₆)-alkylamino or (C₁-C₆)-alkanoylamino,
or
R¹¹ and R¹² together with the nitrogen atom to which they are attached form a 4- to 6- membered heterocycle which may contain a further heteroatom O or N in the ring and which may be substituted by fluorine, hydroxyl, hydroxycarbonyl, 5- to 7-membered heterocyclyl which may contain one or two further heteroatoms N and/or O in the ring and which for its part may be substituted by (C₁-C₄)-alkyl or (C₁-C₄)-alkoxycarbonyl, (C₁-C₄)-alkoxy, (C₁-C₄)-alkyl, (C₁-C₄) -alkanoyl, (C₁-C₄)-alkoxycarbonyl or -NR¹⁵R¹⁶,
where alkyl for its part may be substituted by hydroxyl, (C₁-C₄)-alkoxy or -NR¹⁷R¹⁸,
where
R¹⁷ and R¹⁸ independently of one another represent hydrogen, optionally hydroxyl-substituted (C₁-C₆)-alkyl, (C₃-C₆)-cycloalkyl, (C₆-C₁₀)-aryl or 5- to 6-membered heteroaryl
or
R¹⁷ and R¹⁸ together with the nitrogen atom to which they are attached form a 5- or 6-membered heterocycle which may contain a further heteroatom O or N in the ring and which may be substituted by (C₁-C₆)-alkyl, (C₁-C₆)-alkanoyl or (C₁-C₆)-alkoxycarbonyl,
in which
R¹⁵ and R¹⁶ independently of one another represent hydrogen, (C₁-C₆)-alkyl, (C₁-C₄)-alkoxycarbonyl, (C₃-C₈)-cycloalkyl or (C₁-C₄)-alkanoyl
or
R¹⁵ and R¹⁶ together with the nitrogen atom to which they are attached form a 5- or 6- membered heterocycle which may contain a further heteroatom O or N in the ring and which may be substituted by (C₁-C₆)-alkyl, (C₁-C₆)-alkanoyl or (C₁-C₆)-alkoxycarbonyl,
or
R¹¹ and R¹² together with the nitrogen atom to which they are attached form a 7- to 12-membered bicyclic or tricyclic heterocycle which is fused or spirocyclic and which may have one or two further heteroatoms from the group consisting of N and O in the ring and which may be substituted by fluorine, (C₁-C₄)-alkyl, (C₁-C₄)-alkoxycarbonyl, (C₁-C₄)-alkanoyl or benzyl,
• and -C(=O)R¹⁹,
in which
R¹⁹ represents (C₁-C₆) -alkoxy, (C₁-C₆) -alkylamino or a 5- to 10-membered mono- or bicyclic heterocycle which is attached via a nitrogen atom, which is fused or spirocyclic and which may have one or two further heteroatoms from the group consisting of N and O in the ring,
where alkylamino for its part may be substituted by a 5- or 6-membered heterocycle,
R⁴ represents hydrogen, fluorine or chlorine,
R⁵ represents hydrogen or (C₁-C₆)-alkyl
or one of its salts, its solvates or the solvates of its salts.

2. Compound of the formula (I) according to Claim 1,
in which
----- represents a double bond,
X represents -NR⁶-, -CH₂- or -C(=O)-,
in which
R⁶ represents hydrogen or methyl,
R¹ represents hydrogen, fluorine, chlorine, cyano, trifluoromethyl, difluoromethyl, methyl, ethyl, hydroxyethyl, methoxyethyl, cyclopropyl, (C₁-C₆)-alkoxycarbonyl or (C₁-C₆)-alkylaminocarbonyl,
where alkoxycarbonyl and alkylaminocarbonyl for their part may be substituted by hydroxyl, hydroxycarbonyl, (C₁-C₆)-alkoxy, (C₁-C₆)-alkoxycarbonyl, amino, aminocarbonyl, (C₁-C₆)-alkylamino or (C₁-C₆)-alkylaminocarbonyl,
R² represents fluorine or chlorine,
R³ represents a radical selected from the group consisting of:
• hydrogen, hydroxyl, halogen, trifluoromethyl, pentafluoroethyl,
• (C₁-C₆)-alkyl, (C₁-C₆)-alkoxy, (C₃-C₈)-cycloalkyl, where alkyl, alkoxy and cycloalkyl for their part may be substituted by hydroxyl, hydroxycarbonyl, (C₁-C₆)-alkoxy, (C₁-C₆)-alkoxycarbonyl, (C₆-C₁₀)-aryl, -NR⁹R¹⁰ or -C(=O)NR⁹R¹⁰,
in which
R⁹ and R¹⁰ independently of one another represent hydrogen or (C₁-C₈)-alkyl
or
R⁹ and R¹⁰ together with the nitrogen atom to which they are attached form a 5- or 6-membered heterocycle which may contain a further heteroatom O or N in the ring and which may be substituted by (C₁-C₆)-alkyl, (C₁-C₆)-alkanoyl or (C₁-C₆)-alkoxycarbonyl,
• phenyl, 5- or 6-membered heteroaryl, 5- to 10- membered heterocyclyl which is attached via a carbon atom,
where phenyl, heteroaryl and heterocyclyl for their part may be substituted by halogen, cyano, nitro, hydroxycarbonyl, amino, trifluoromethyl, optionally hydroxyl-substituted (C₁-C₆)-alkyl, (C₁-C₆)-alkoxy, (C₁-C₆)-alkylamino, (C₁-C₆)-alkanoyl, (C₁-C₆)-alkoxycarbonyl, (C₁-C₆)-alkanoylamino or (C₁-C₆)-alkoxycarbonylamino,
• -NR¹¹R¹²,
in which
R¹¹ and R¹² independently of one another represent hydrogen, (C₁-C₆)-alkyl, (C₃-C₈)-cycloalkyl, (C₆-C₁₀)-aryl or 5- to 10-membered heteroaryl,
where alkyl and cycloalkyl for their part may be substituted by hydroxyl, (C₁-C₆)-alkoxy, (C₆-C₁₀)-aryl or 5- to 10-membered heteroaryl,
and
where aryl and heteroaryl for their part may be substituted by halogen, hydroxyl, amino, cyano, trifluoromethyl, (C₁-C₆)-alkyl, (C₁-C₆)-alkoxy, (C₁-C₆)-alkylamino or (C₁-C₆)-alkanoylamino,
or
R¹¹ and R¹² together with the nitrogen atom to which they are attached form a 4- to 6-membered heterocycle which may contain a further heteroatom O or N in the ring and which may be substituted by fluorine, hydroxyl, hydroxycarbonyl, (C₁-C₄)-alkoxy, (C₁-C₄)-alkyl, (C₁-C₄)-alkanoyl or (C₁-C₄)-alkoxycarbonyl,
• and -C(=O)R¹⁹,
in which
R¹⁹ represents (C₁-C₆)-alkoxy, (C₁-C₆)-alkylamino or a 5- to 10-membered mono- or bicyclic heterocycle which is attached via a nitrogen atom, which is fused or spirocyclic and which may have one or two further heteroatoms from the group consisting of N and O in the ring, where alkylamino for its part may be substituted by a 5- or 6-membered heterocycle,
R⁴ represents hydrogen, fluorine or chlorine,
R⁵ represents hydrogen or methyl,
or one of its salts, its solvates or the solvates of its salts.

3. Compound of the formula (I) according to Claim 1 or 2,
in which
----- represents a double bond,
X represents -NR⁶-, -CH₂- or -C(=O)-,
in which
R⁶ represents hydrogen or methyl,
R¹ represents hydrogen, fluorine, chlorine, cyano, trifluoromethyl, difluoromethyl, methyl, ethyl, hydroxyethyl, methoxyethyl or cyclopropyl,
R² represents fluorine or chlorine,
R³ represents a radical selected from the group consisting of:
• hydrogen, hydroxyl, halogen, trifluoromethyl, pentafluoroethyl,
• (C₁-C₆)-alkyl, (C₁-C₆)-alkoxy, (C₃-C₈)-cycloalkyl,
where alkyl, alkoxy and cycloalkyl for their part may be substituted by hydroxyl, (C₁-C₆)-alkoxy or -NR⁹R¹⁰,
in which
R⁹ and R¹⁰ independently of one another represent hydrogen or (C₁-C₈)-alkyl
or
R⁹ and R¹⁰ together with the nitrogen atom to which they are attached form a 5- or 6- membered heterocycle which may contain a further heteroatom O or N in the ring and which may be substituted by (C₁-C₃)-alkyl or (C₁-C₃)-alkanoyl,
• phenyl and 5- or 6-membered heteroaryl,
where phenyl and heteroaryl for their part may be substituted by halogen, amino or trifluoromethyl,
R⁴ represents hydrogen, fluorine or chlorine,
R⁵ represents hydrogen or methyl,
or one of its salts, its solvates or the solvates of its salts.

4. Compound of the formula (I) according to any of Claims 1 to 3
in which
----- represents a double bond,
X represents -NH- or -CH₂-,
R¹ represents hydrogen, chlorine or methyl,
R² represents fluorine,
R³ represents a radical selected from the group consisting of hydrogen, halogen, trifluoromethyl, optionally halogen-substituted phenyl and pyridine,
R⁴ represents hydrogen or fluorine,
R⁵ represents hydrogen,
or one of its salts, its solvates or the solvates of its salts.

5. Process for preparing a compound of the formula (I) as defined in Claim 1, **characterized in that**
compounds of the formula (II) 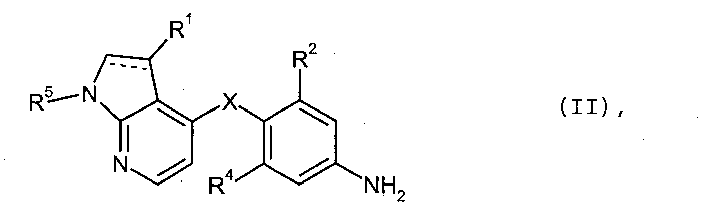 in which
-----, X, R¹, R², R⁴ and R⁵ are as defined in Claim 1
are reacted with compounds of the formula (III) 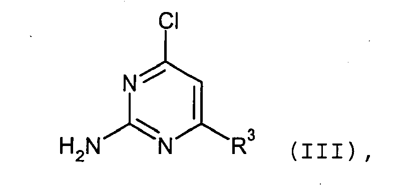 in which
R³ is as defined in Claim 1,
which, if X represents a CH₂ group, may be followed by an oxidation to give the corresponding keto group - C(=O)- or a methylation to give the corresponding mono- or dimethyl compound [ -CH(CH₃)- or -C(CH₃)₂- ].

6. Compound of the formula (I) as defined in any of Claims 1 to 4 for the treatment and/or prophylaxis of disorders.

7. Use of a compound of the formula (I) as defined in any of Claims 1 to 4 for preparing medicaments for the treatment and/or prophylaxis of cardiovascular disorders.

8. Use of a compound of the formula (I) as defined in any of Claims 1 to 4 for preparing medicaments for the treatment and/or prophylaxis of erectile dysfunction.

9. Medicament, comprising a compound of the formula (I) as defined in any of Claims 1 to 4 in combination with a further active compound.

10. Medicament, comprising a compound of the formula (I) as defined in any of Claims 1 to 4 in combination with an inert nontoxic pharmaceutically acceptable auxiliary.

11. Medicament according to Claim 9 or 10 for the treatment and/or prophylaxis of cardiovascular disorders.

12. Medicament according to Claim 9 or 10 for the treatment and/or prophylaxis of erectile dysfunction.

## Revendications

1. Composé de formule (I) 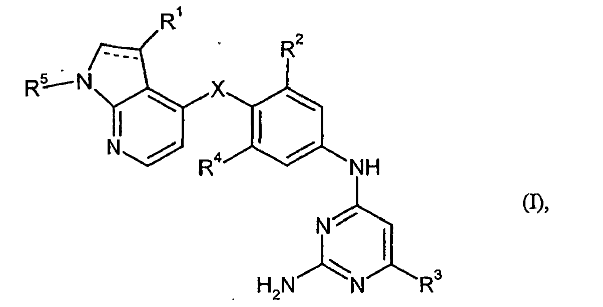 dans laquelle
----- désigne une liaison simple ou une double liaison,
X représente un groupe -NR⁶-, -CR⁷R⁸- ou -C(=O),
où
R⁶ représente l'hydrogène ou un reste alkyle en C₁ à C₃, R⁷ et R⁸ représentent, indépendamment l'un de l'autre, l'hydrogène ou un reste méthyle,
R¹ représente l'hydrogène, un halogène, un groupe cyano, un reste alkyle en C₁ à C₆, cycloalkyle en C₃ à C₆, (alkoxy en C₁ à C₆)carbonyle ou (alkylamino en C₁ à C₆) carbonyle,
les restes alkyle, alkoxycarbonyle et alkylaminocarbonyle pouvant eux-mêmes être substitués par un radical hydroxy, halogéno, hydroxycarbonyle, alkoxy en
C₁ à C₆, (alkoxy en C₁ à C₆)carbonyle, amino, aminocarbonyle, alkylamino en C₁ à C₆ ou (alkylamino en C₁ à C₆)carbonyle,
R² représente le fluor ou le chlore,
R³ représente un reste qui est choisi dans le groupe suivant :
• hydrogène, hydroxy, halogéno, trifluorométhyle, pentafluoréthyle,
• alkyle en C₁ à C₆, alkoxy en C₁ à C₆, cycloalkyle en C₃ à C₈, les restes alkyle, alkoxy et cycloalkyle pouvant eux-mêmes être substitués par un radical hydroxy, hydroxycarbonyle, alkoxy en C₁ à C₆, (alkoxy en C₁ à C₆)carbonyle, aryle en C₆ à C₁₀, -NR⁹R¹⁰ ou -C(=O)NR⁹R¹⁰,
où
R⁹ et R¹⁰ représentent, indépendamment l'un de l'autre, l'hydrogène, un reste alkyle en C₁ à C₈, cycloalkyle en C₃ à C₆ éventuellement substitué avec un radical alkyle en C₁ à C₆, un reste aryle en C₆ à C₁₀ éventuellement substitué par un halogène, ou bien un reste hétéroaryle de 5 à 10 chaînons,
ou bien
R⁹ et R¹⁰ forment, conjointement avec l'atome d'azote auquel ils sont liés, un hétérocycle penta- ou hexagonal qui peut contenir un autre hétéroatome O ou N dans le noyau et qui peut être substitué par un radical alkyle en C₁ à C₆, alcanoyle en C₁ à C₆ ou (alkoxy en C₁ à C₆)carbonyle,
• un reste aryle en C₆ à C₁₀, aryloxy en C₆ à C₁₀, hétéroaryle de 5 à 10 chaînons, hétéroaryloxy de 5 à 10 chaînons, hétérocyclyle de 5 à 10 chaînons lié par l'intermédiaire de l'atome de carbone, les restes aryle, aryloxy, hétéroaryle, hétéroaryloxy et hétérocyclyle pouvant eux-mêmes être substitués par un radical halogéno, cyano, nitro, hydroxycarbonyle, amino, trifluorométhyle, alkyle en C₁ à C₆ portant éventuellement un substituant hydroxy, par un radical alkoxy en C₁ à C₆, alkylamino en C₁ à C₆, alcanoyle en C₁ à C₆, (alkoxy en C₁ à C₆)carbonyle, alcanoylamino en C₁ à C₆, (alkoxy en C₁ à C₆)carbonylamino ou hétérocyclyle penta- ou hexagonal,
• un groupe -NR¹¹R¹²
dans lequel
R¹¹ et R¹² représentent, indépendamment l'un de l'autre, l'hydrogène, un reste alkyle en C₁ à C₆, cycloalkyle en C₃ à C₈, aryle en C₆ à C₁₀ ou hétéroaryle de 5 à 10 chaînons, les restes alkyle et cycloalkyle pouvant eux-mêmes être substitués par un radical hydroxy, alkoxy en C₁ à C₆, aryle en C₆ à C₁₀, hétéroaryle de 5 à 10 chaînons ou -NR¹³R¹⁴,
où
R¹³ et R¹⁴ représentent, indépendamment l'un de l'autre, l'hydrogène, un reste alkyle en C₁ à C₆, cycloalkyle en C₃ à C₈, aryle en C₆ à C₁₀ ou hétéroaryle penta- ou hexagonal,
ou bien
R¹³ et R¹⁴ forment, conjointement avec l'atome d'azote auquel ils sont liés, un hétérocycle penta- ou hexagonal qui peut contenir un autre hétéroatome O ou N dans le noyau et qui peut être substitué par un radical alkyle en C₁ à C₆, alcanoyle en C₁ à C₆ ou (alkoxy en C₁ à C₆)carbonyle,
et
les restes aryle et hétéroaryle pouvant eux-mêmes être substitués par un radical halogéno, hydroxy, amino, cyano, trifluorométhyle, alkyle en C₁ à C₆, alkoxy en C₁ à C₆, (alkyle en C₁ à C₆)amino ou alcanoylamino en C₁ à C₆, ou bien
R¹¹ et R¹² forment, conjointement avec l'atome d'azote auquel ils sont liés, un hétérocycle tétra- à hexagonal, qui peut contenir dans le noyau un autre hétéroatome O ou N et qui peut être substitué par un radical fluoro, hydroxy, hydroxycarbonyle, hétérocyclyle penta- à heptagonal qui peut contenir dans le noyau un ou deux autres hétéroatomes N et/ou O et qui peut lui-même porter un substituant alkyle en C₁ à C₄ ou (alkoxy en C₁ à C₄)-carbonyle, par un radical alkoxy en C₁ à C₄, alkyle en C₁ à C₄, alcanoyle en C₁ à C₄, (alkoxy en C₁ à C₄)carbonyle ou -NR¹⁵R¹⁶, le radical alkyle pouvant lui-même porter un substituant hydroxy, alkoxy en C₁ à C₄ ou -NR¹⁷R¹⁸,
où
R¹⁷ et R¹⁸ représentent, indépendamment l'un de l'autre, l'hydrogène, un reste alkyle en C₁ à C₆ portant éventuellement un substituant hydroxy, un reste cycloalkyle en C₃ ou C₄, aryle en C₆ à C₁₀ ou hétéroaryle penta- ou hexagonal,
ou bien
R¹⁷ et R¹⁸ forment, conjointement avec l'atome d'azote auquel ils sont liés, un hétérocycle penta- ou hexagonal, qui peut contenir dans le noyau un autre hétéroatome O ou N et qui peut être substitué par un radical alkyle en C₁ à C₆, alcanoyle en C₁ à C₆ ou (alkoxy en C₁ à C₆)carbonyle,
où
R¹⁵ et R¹⁶ représentent, indépendamment l'un de l'autre, l'hydrogène, un reste alkyle en C₁ à C₆, (alkoxy en C₁ à C₄)carbonyle, cycloalkyle en C₃ à C₈ ou alcanoyle en C₁ à C₄,
ou bien
R¹⁵ et R¹⁶ forment, conjointement avec l'atome d'azote auquel ils sont liés, un hétérocycle penta- ou hexagonal qui peut contenir dans le noyau un autre hétéroatome O ou N et qui peut être substitué par un radical alkyle en C₁ à C₆, alcanoyle en C₁ à C₆ ou (alkoxy en C₁ à C₆)carbonyle,
ou bien
R¹¹ et R¹² forment, conjointement avec l'atome d'azote auquel ils sont liés, un hétérocycle bicyclique ou tricyclique de 7 à 12 chaînons, qui est condensé ou spirocyclique, et qui peut présenter dans le noyau un ou deux autres hétéroatomes de la série N et/ou O et qui peut être substitué par un radical fluoro, alkyle en C₁ à C₄, (alkoxy en C₁ à C₄)carbonyle, alcanoyle en C₁ à C₄ ou benzyle,
• et -C(=O)R¹⁹
où
R¹⁹ représente un reste alkoxy en C₁ à C₆, alkylamino en C₁ à C₆ ou un hétérocycle monocyclique ou bicyclique de 5 à 10 chaînons lié par l'intermédiaire d'un atome d'azote, qui est condensé ou spirocyclique et qui peut présenter dans le noyau un ou deux autres hétéroatomes de la série N et/ou O, le reste alkylamino pouvant lui-même porter un substituant hétérocyclyle penta- ou hexagonal,
R⁴ représente l'hydrogène, le fluor ou le chlore,
R⁵ représente l'hydrogène ou un reste alkyle en C₁ à C₆, ou l'un de ses sels, de ses produits de solvatation ou des produits de solvatation de ses sels.

2. Composé de formule (I) suivant la revendication 1, formule dans laquelle
----- représente une double liaison,
X représente un groupe -NR⁶-, -CH₂- ou -C(=O),
où
R⁶ représente l'hydrogène ou un reste méthyle,
R¹ représente l'hydrogène, le fluor, le chlore, un groupe cyano, un reste trifluorométhyle, difluorométhyle, méthyle, éthyle, hydroxyéthyle, méthoxyéthyle, cyclopropyle, (alkoxy en C₁ à C₆)carbonyle ou (alkylamino en C₁ à C₆)carbonyle,
les restes alkoxycarbonyle et alkylaminocarbonyle pouvant eux-mêmes être substitués par un radical hydroxy, hydroxycarbonyle, alkoxy en C₁ à C₆, (alkoxy en C₁ à
C₆)carbonyle, amino, aminocarbonyle, alkylamino en C₁ à C₆ ou (alkylamino en C₁ à C₆)carbonyle,
R² représente le fluor ou le chlore,
R³ représente un reste qui est choisi dans le groupe suivant :
• hydrogène, hydroxy, halogéno, trifluorométhyle, pentafluoréthyle,
• alkyle en C₁ à C₆, alkoxy en C₁ à C₆, cycloalkyle en C₃ à C₈, les restes alkyle, alkoxy et cycloalkyle pouvant eux-mêmes être substitués par un radical hydroxy, hydroxycarbonyle, alkoxy en C₁ à C₆, (alkoxy en C₁ à C₆)carbonyle, aryle en C₆ à C₁₀, -NR⁹R¹⁰ ou -C(=O)NR⁹R¹⁰,
où
R⁹ et R¹⁰ représentent, indépendamment l'un de l'autre, l'hydrogène ou un reste alkyle en C₁ à C₈,
ou
bien
R⁹ et R¹⁰ forment, conjointement avec l'atome d'azote auquel ils sont liés, un hétérocycle penta- ou hexagonal qui peut contenir dans le noyau un autre hétéroatome O ou N et qui peut être substitué par un radical alkyle en C₁ à C₆, alcanoyle en C₁ à C₆ ou (alkoxy en C₁ à C₆)carbonyle,
• phényle, hétéroaryle penta- ou hexagonal, hétérocyclyle de 5 à 10 chaînons, lié par l'intermédiaire de l'atome de carbone, les restes phényle, hétéroaryle, et hétérocyclyle pouvant eux-mêmes être substitués par un radical halogéno, cyano, nitro, hydroxycarbonyle, amino, trifluorométhyle, alkyle en C₁ à C₆ portant éventuellement un substituant hydroxy, par un radical alkoxy en C₁ à C₆, alkylamino en C₁ à C₆, alcanoyle en C₁ à C₆, (alkoxy en C₁ à C₆)carbonyle, alcanoylamino en C₁ à C₆ ou (alkoxy en C₁ à C₆)carbonylamino,
• un groupe -NR¹¹R¹²
où
R¹¹ et R¹² représentent, indépendamment l'un de l'autre, l'hydrogène, un reste alkyle en C₁ à C₆, cycloalkyle en C₃ à C₈, aryle en C₆ à C₁₀ ou hétéroaryle de 5 à 10 chaînons, les restes alkyle et cycloalkyle pouvant eux-mêmes être substitués par un radical hydroxy, alkoxy en C₁ à C₆, aryle en C₆ à C₁₀, ou hétéroaryle de 5 à 10 chaînons,
et
les restes aryle et hétéroaryle pouvant eux-mêmes être substitués par un radical halogéno, hydroxy, amino, cyano, trifluorométhyle, alkyle en C₁ à C₆, alkoxy en C₁ à C₆, alkylamino en C₁ à C₆ ou alcanoylamino en C₁ à C₆, ou bien
R¹¹ et R¹² forment, conjointement avec l'atome d'azote auquel ils sont liés, un hétérocycle tétra- à hexagonal, qui peut contenir dans le noyau un autre hétéroatome O ou N et qui peut être substitué par un radical fluoro, hydroxy, hydroxycarbonyle, alkoxy en C₁ à C₄, alkyle en C₁ à C₄, alcanoyle en C₁ à C₄ ou (alkoxy en C₁ à C₄)carbonyle,
• et -C(=O)R¹⁹
où
R¹⁹ représente un reste alkoxy en C₁ à C₆, alkylamino en C₁ à C₆ ou un hétérocycle monocyclique ou bicyclique de 5 à 10 chaînons lié par l'intermédiaire d'un atome d'azote, qui est condensé ou spirocyclique et qui peut présenter dans le noyau un ou deux autres hétéroatomes de la série N et/ou O, le reste alkylamino pouvant lui-même être substitué avec un radical hétérocyclyle penta- ou hexagonal,
R⁴ représente l'hydrogène, le fluor ou le chlore,
R⁵ représente l'hydrogène ou un groupe méthyle, ou l'un de ses sels, de ses produits de solvatation ou des produits de solvatation de ses sels.

3. Composé de formule (I) suivant la revendication 1
ou 2,
formule dans laquelle
----- désigne une double liaison,
X représente un groupe -NR⁶-, CH₂- ou -C(=O),
où
R⁶ représente l'hydrogène ou un reste méthyle,
R¹ représente l'hydrogène, le fluor, le chlore, un groupe cyano, un reste trifluorométhyle, difluorométhyle, méthyle, éthyle, hydroxyéthyle, méthoxyéthyle ou cyclopropyle,
R² représente le fluor ou le chlore,
R³ représente un reste qui est choisi dans le groupe suivant :
• hydrogène, hydroxy, halogéno, trifluorométhyle, pentafluoréthyle,
• alkyle en C₁ à C₆, alkoxy en C₁ à C₆, cycloalkyle en C₃ à C₈, les restes alkyle, alkoxy et cycloalkyle pouvant eux-mêmes être substitués par un radical hydroxy, alkoxy en C₁ à C₆ ou -NR⁹R¹⁰,
où
R⁹ et R¹⁰ représentent, indépendamment l'un de l'autre, l'hydrogène ou un reste alkyle en C₁ à C₈,
ou bien
R⁹ et R¹⁰ forment, conjointement avec l'atome d'azote auquel ils sont liés, un hétérocycle penta- ou hexagonal qui peut contenir dans le noyau un autre hétéroatome O ou N et qui peut être substitué par un radical alkyle en C₁ à C₃ ou alcanoyle en C₁ à C₃,
• phényle ou hétéroaryle de penta- ou hexagonal, les restes phényle et hétéroaryle pouvant eux-mêmes être substitués par un radical halogéno, amino ou trifluorométhyle,
R⁴ représente l'hydrogène, le fluor ou le chlore,
R⁵ représente l'hydrogène ou un groupe méthyle, ou l'un de ses sels, de ses produits de solvatation ou des produits de solvatation de ses sels.

4. Composé de formule (I) suivant l'une des revendications 1 à 3,
formule dans laquelle
----- désigne une double liaison,
X représente un groupe -NH- ou -CH₂-,
R¹ représente l'hydrogène, le chlore, ou un reste méthyle,
R² représente le fluor,
R³ représente un reste qui est choisi dans le groupe des restes hydrogène, halogéno, trifluorométhyle, phényle éventuellement substitué par un halogène, et pyridine,
R⁴ représente l'hydrogène ou le fluor,
R⁵ représente l'hydrogène,
ou l'un de ses sels, de ses produits de solvatation ou des produits de solvatation de ses sels.

5. Procédé de production d'un composé de formule (I), telle que définie dans la revendication 1, **caractérisé en ce qu'**on fait réagir des composés de formule (II) 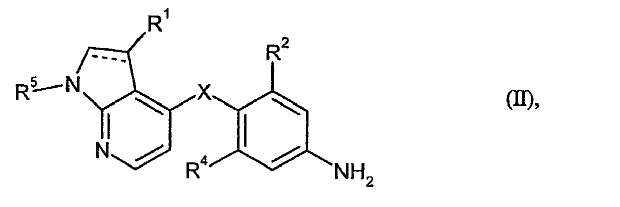 dans laquelle
-----, X, R¹, R², R⁴ et R⁵ ont la définition indiquée
dans la revendication 1,
avec des composés de formule (III) 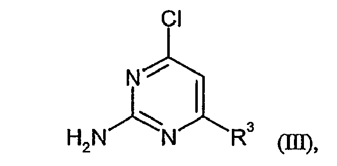 dans laquelle
R³ a la définition indiquée dans la revendication 1 la réaction pouvant être suivie, au cas où X représente un groupe CH₂, d'une oxydation en le groupe céto -C(=O)-correspondant ou d'une méthylation en le composé mono- ou diméthylé [CH(CH₃)- et respectivement -C(CH₃)₂-] correspondant.

6. Composé de formule (I) telle que définie dans l'une des revendications 1 à 4, destiné au traitement et/ou à la prophylaxie de maladies.

7. Utilisation d'un composé de formule (I), telle que définie dans l'une des revendications 1 à 4 pour la préparation de médicaments destinés au traitement et/ou à la prophylaxie de maladies cardiovasculaires.

8. Utilisation d'un composé de formule (I), telle que définie dans l'une des revendications 1 à 4 pour la préparation de médicaments destinés au traitement et/ou à la prophylaxie de dysfonctionnements érectiles.

9. Médicament contenant un composé de formule (I), telle que définie dans l'une des revendications 1 à 4, en combinaison avec une autre substance active.

10. Médicament contenant un composé de formule (I), telle que définie dans l'une des revendications 1 à 4, en combinaison avec une substance auxiliaire non toxique, pharmaceutiquement acceptable.

11. Médicament suivant la revendication 9 ou 10, destiné au traitement et/ou à la prophylaxie de maladies cardiovasculaires.

12. Médicament suivant la revendication 9 ou 10, destiné au traitement et/ou à la prophylaxie d'un dysfonctionnement érectile.
